(19) Europäisches Patentamt
European Patent Office
Office européen des brevets

(11) EP 4 570 271 A1

(12) **EUROPEAN PATENT APPLICATION**
published in accordance with Art. 153(4) EPC

(43) Date of publication:
**18.06.2025 Bulletin 2025/25**

(21) Application number: **23851728.8**

(22) Date of filing: **04.08.2023**

(51) International Patent Classification (IPC):
***A61K 47/54** (2017.01)* ***A61K 9/127** (2025.01)*
***A61K 31/12** (2006.01)* ***A61P 35/00** (2006.01)*
***A61P 3/10** (2006.01)* ***A61P 31/12** (2006.01)*
***A61P 37/00** (2006.01)* ***A61P 25/28** (2006.01)*

(52) Cooperative Patent Classification (CPC):
**A61K 9/127; A61K 31/12; A61K 47/54; A61P 3/10; A61P 25/28; A61P 31/12; A61P 35/00; A61P 37/00**

(86) International application number:
**PCT/CN2023/111279**

(87) International publication number:
**WO 2024/032507 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.08.2022 CN 202210951941**
**09.08.2022 CN 202210950392**
**09.08.2022 CN 202210950391**

(71) Applicants:
- **Hunan Lonstar Biotech Co., Ltd.**
  **Changsha, Hunan 410000 (CN)**
- **Hunan Fanaplos Biotechnology LLC.**
  **Changsha, Hunan 410000 (CN)**

(72) Inventors:
- **WANG, Shan**
  **Changsha, Hunan 410000 (CN)**
- **HU, Dun**
  **Changsha, Hunan 410000 (CN)**
- **SUN, Yiyi**
  **Changsha, Hunan 410000 (CN)**

(74) Representative: **Stork Bamberger Patentanwälte PartmbB**
**Meiendorfer Strasse 89**
**22145 Hamburg (DE)**

Remarks:
The complete document including Reference Table(s) and the Sequence Listing(s) can be downloaded from the EPO website

(54) **METAL-PHOSPHOLIPID COMPLEX, METAL-PHOSPHOLIPID COMPLEX PARTICLE, DRUG-LIPID PARTICLE, METHOD FOR PREPARING SAME, AND USE THEREOF**

(57) The present disclosure provides a metal-chelated phospholipid complex, a metal-chelated phospholipid complex nanoparticle and a drug-lipid particle, and preparation methods and uses thereof, which relates to the field of biotechnology. The metal-chelated phospholipid complex is formed by reaction of a phospholipid molecular moiety, a linker molecular moiety, and a metal ion moiety. The metal-chelated phospholipid complex nanoparticle comprises a metal-chelated phospholipid complex, a particle aggregation-inhibiting conjugated lipid, and a non-cationic lipid or a non-ionizable lipid. The drug-lipid particle comprises a drug and the metal-chelated phospholipid complex nanoparticle. The metal-chelated phospholipid complex has a function in adsorbing drugs with negative charges and self-assembling with other lipids into metal-chelated phospholipid complex nanoparticles (MPP), so as to avoid the use of cationic lipid or ionizable lipid under the condition of ensuring the effectiveness which is not less than that of LNP, so that the toxicity of the drug-lipid particle is greatly reduced compared with LNP, the biological safety is remarkably improved, and the delivery of drugs with negative charges in organisms is facilitated.

EP 4 570 271 A1

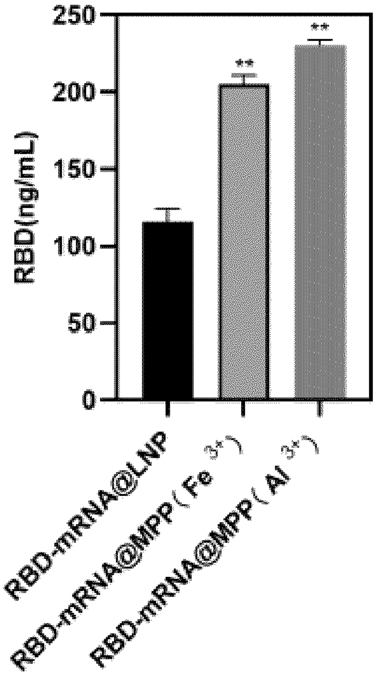
250
200
150
100
50
0
RBD(ng/mL)
**
**
RBD-mRNA@LNP
RBD-mRNA@MPP（Fe 3+）
RBD-mRNA@MPP（Al 3+）

Fig. 2-10

## Description

### Cross Reference to Related Applications

The present disclosure claims priority to and the benefit of Chinses Patent Application No. 202210951941.8, "Drug-Lipid Particle, Preparation Method for The Same, And Use Thereof", filed on August 09, 2022, Chinse Patent Application No. 202210950392.2, "Metal-Chelated Phospholipid complex Nanoparticles, Preparation method for The Same, And Use Thereof", filed on August 09, 2022, and Chinses Patent Application No. 202210950391.8, "Metal-chelated phospholipid complex, Preparation Method for The Same, And Use Thereof", filed on August 09, 2022, in the Chinese Intellectual Property Office, the entire content of which is incorporated herein by reference.

### Technical Field

The disclosure relates to the field of biotechnology, and in particular relates to a metal-chelated phospholipid complex, a metal-chelated phospholipid complex nanoparticle, a drug-lipid particle, and preparation methods and uses thereof.

### Background

With the continuous development of molecular biology technology, the association between genes and diseases is more and more deeply known. Nucleic acid drugs refer to artificially synthesized DNA or RNA fragments with disease treatment functions, which are of great interest because they show great application potential in disease diagnosis and therapeutic process. Such drugs can act directly on pathogenic target genes or mRNA, having a therapeutic effect at the gene level. Compared with the traditional small-molecule drugs and antibody drugs, the nucleic acid drug is not limited by the druggability of the target protein, can treat a broader range of diseases, and can regulate the expression of pathogenic genes radically. The nucleic acid drug also has the outstanding advantages of high efficiency, low toxicity, high specificity and the like, and is expected to become the third largest type of drugs after small-molecule drugs and antibody drugs.

However, nucleic acid drugs are susceptible to degradation by nucleases in vivo, and due to their large molecular weight and their negatively charged nature, they have difficulty in crossing cell membranes to function. Therefore, the search for a safe and effective nucleic acid drug delivery system is a bottleneck problem to be solved urgently in the development of nucleic acid drugs. Currently, vectors capable of delivering nucleic acid drugs can be mainly classified into viral vectors and non-viral vectors. Viral vectors are less frequently used because they cause immune responses after entering a human body. The more commonly used non-viral vectors are mainly nanoparticles and small-molecule conjugates. Compared with small-molecule conjugates directly conjugated with nucleic acid drugs, the nanoparticles can more effectively encapsulate the nucleic acid drugs and prevent the nucleic acid drugs from being rapidly degraded by nuclease, so that the body circulation time of the nucleic acid drugs is prolonged. The mechanism by which nanoparticles encapsulate nucleic acids is the adsorption of negatively charged nucleic acids by positively charged cationic lipid. However, cationic lipids are highly cytotoxic and their toxic mechanisms of action are: (1) cell atrophy, reduction in mitotic numbers and vacuolization of cytoplasm; (2) interaction with biological proteins such as protein kinase C and the like to destroy their activity; (3) triggering the secretion of a variety of pro-inflammatory cytokines and chemokines by activation of p38 mitogen-activated protein kinase and nuclear factor κB transcription factor. Furthermore, ionizable lipid is a lipid containing a positively charged ionizable amine group, which is uncharged under physiological conditions (pH = 7.4), but can be protonated and positively charged at a lower pH environment. Thus, ionizable lipid can be used to partially or completely replace cationic lipid as the main component of the nanoparticles for the adsorption of nucleic acids. When nanoparticles containing ionizable lipid enter lysosomes of biological cells, the ionizable lipid become positively charged lipids in a low pH (pH = 4.0-6.5) environment within the lysosome. Although ionizable lipid reduce the cytotoxic and highly inflammatory effects of some permanently positively charged cationic lipid, their cytotoxicity and immunogenicity remain high. Lipid Nanoparticles (LNPs) based on cationic lipid and/or ionizable lipid, which are the main component of LNPs for the adsorption of nucleic acids, are currently used as a Nanoparticle nucleic acid drug delivery system for clinical use. At the same time, the cytotoxicity and immunogenicity mediated by cationic lipid and/or ionizable lipid are still one of the major causes for the high toxicity of LNPs.

When a delivery system is used to deliver a negatively charged drug (e.g., a nucleic acid drug, a protein drug, a polypeptide drug, a small-molecule drug, and the like.), none of the nanoparticle delivery systems based on cationic lipid and/or ionizable lipid can fundamentally solve the toxicity problem of the nanoparticle delivery system. A liposome delivery system having low toxicity, which does not use cationic lipid and/or ionizable lipid, is urgently needed.

Disclosure of Invention

One or more aspects of embodiments of the present disclosure are directed to a metal-chelated phospholipid complex and a preparation method for the same to improve at least one of the technical problems of the prior art.

In order to achieve the purpose, the following embodiments are provided.

An embodiment of the present disclosure provides a metal-chelated phospholipid complex forming by reaction of a phospholipid molecular moiety, a linker molecular moiety, and a metal ion moiety, wherein the phospholipid molecular moiety is linked to the linker molecular moiety, the linker molecular moiety is linked to the metal ion moiety through a coordination bond, and the metal-chelated phospholipid complex is not a cationic lipid or an ionizable lipid.

Further, the phospholipid molecular moiety may be selected from the group consisting of lecithin (PC), phosphatidyl ethanolamine (PE), phosphatidyl serine (PS), phosphatidic acid (PA), phosphatidyl glycerol (PG), ceramide-1-phosphate (SP), phosphatidyl inositol (PI), phosphatidyl threonine (PT), sphingomyelin (SM), lysolecithin (LPC), lysophosphatidyl ethanolamine (LPE), lysophosphatidylserine (LPS), lysophosphatidic acid (LPA), lysophosphatidyl glycerol (LPG), lysophosphatidylinositol (LPI), lysophosphatidylthreonine (LPT), lysosphingomyelin (LSM), sphingosine 1-phosphate (S1P), derivatives thereof, and combinations thereof. Wherein the term "thereof" in "derivatives thereof" refers to "lecithin (PC), phosphatidyl ethanolamine (PE), phosphatidyl serine (PS), phosphatidic acid (PA), phosphatidyl glycerol (PG), ceramide-1-phosphate (SP), phosphatidyl inositol (PI), phosphatidyl threonine (PT), sphingomyelin (SM), lysolecithin (LPC), lysophosphatidyl ethanolamine (LPE), lysophosphatidylserine (LPS), lysophosphatidic acid (LPA), lysophosphatidyl glycerol (LPG), lysophosphatidylinositol (LPI), lysophosphatidylthreonine (LPT), lysosphingomyelin (LSM), sphingosine 1-phosphate (S1P)". In the present disclosure, " derivatives thereof " may be interpreted similarly. The phospholipid molecular moiety may be, for example, but not limited to, lecithin (PC), lecithin (PC) derivatives, phosphatidyl ethanolamine (PE), phosphatidyl ethanolamine (PE) derivatives, phosphatidyl glycerol (PG), phosphatidyl glycerol (PG) derivatives, phosphatidyl glycerol (PG) and lecithin (PC), lecithin (PC) and lecithin (PC) derivatives, and the like.

Further, the phospholipid molecular moiety may be selected from the group consisting of

lecithin (PC)

(Formula 1) 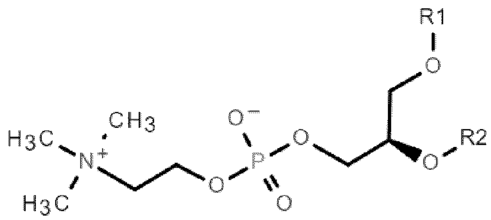 ,

phosphatidyl ethanolamine (PE)

(Formula 2) 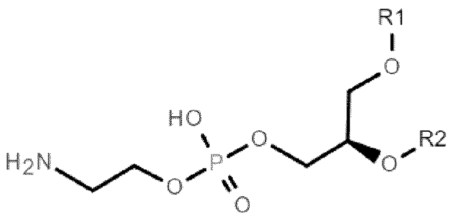 ,

phosphatidyl serine (PS)

(Formula 3) 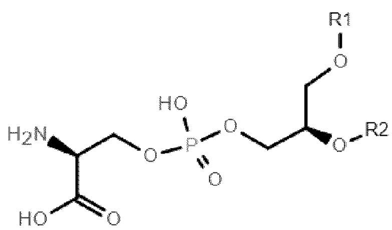 ,

phosphatidic acid (PA)

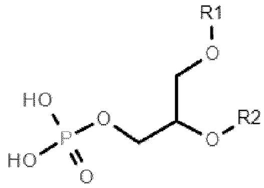

(Formula 4) ,

phosphatidyl glycerol (PG)

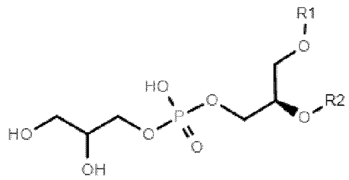

(Formula 5) ,

ceramide-1-phosphate (CP)

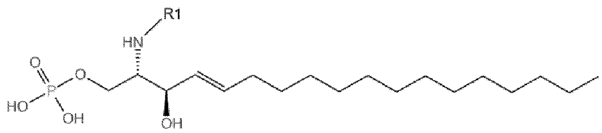

(Formula 6) ,

phosphatidyl inositol (PI)

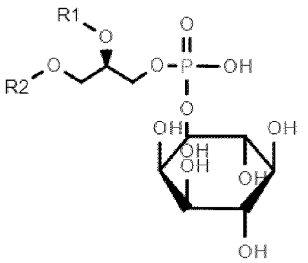

(Formula 7) ,

phosphatidyl threonine (PT)

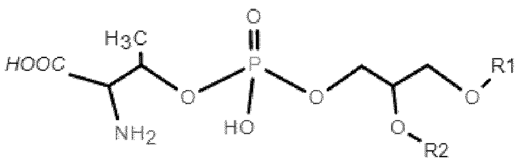

(Formula 8) ,

sphingomyelin (SM)

(Formula 9) 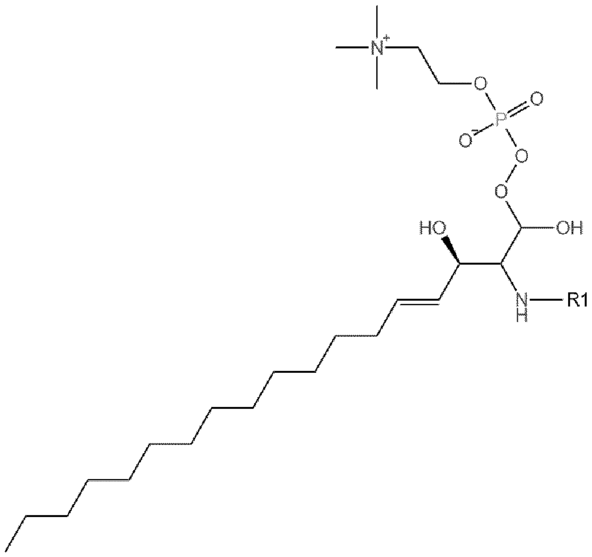 ,

lysolecithin (LPC)

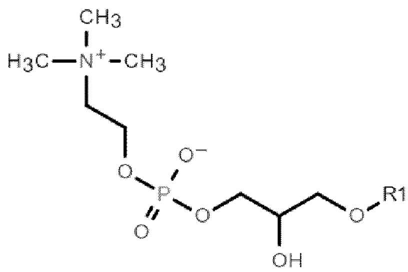

(Formula 10) ,

lysophosphatidyl ethanolamine (LPE)

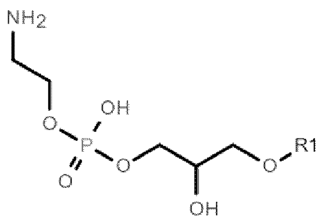

(Formula 11) ,

lysophosphatidylserine (LPS)

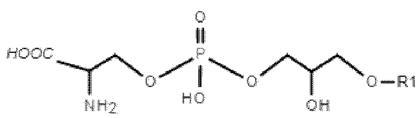

(Formula 12) ,

lysophosphatidic acid (LPA)

(Formula 13) 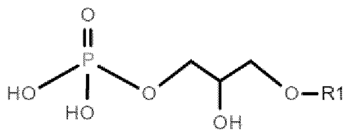 ,

lysophosphatidyl glycerol (LPG)

(Formula 14) 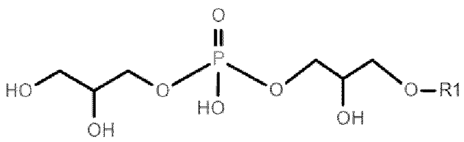 ,

lysophosphatidylinositol (LPI)

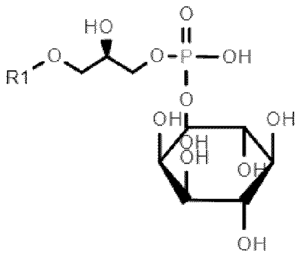

(Formula 15) ,

lysophosphatidylthreonine (LPT)

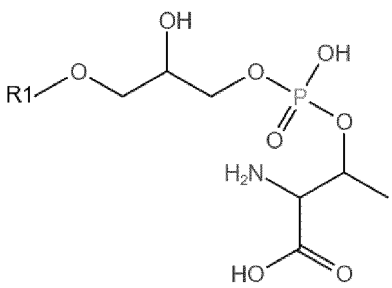

(Formula 16) ,

lysosphingomyelin (LSM)

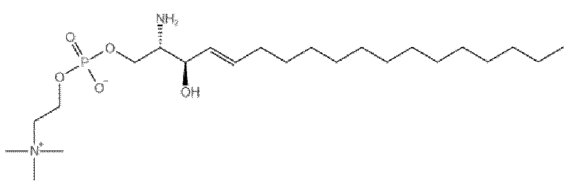

(Formula 17) ,

1-Sphingosine phosphate (S1P)

(Formula 18) 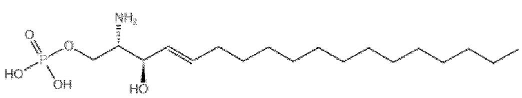 ,

derivatives thereof, and combinations thereof;

wherein, R1, R2 are each independently:

decanoyl

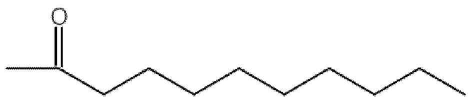

,

lauroyl

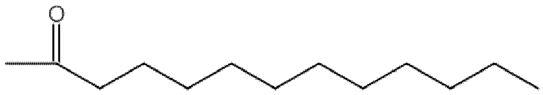

,

myristoyl

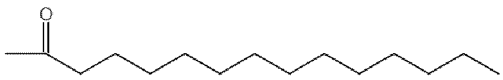

,

palmitoyl

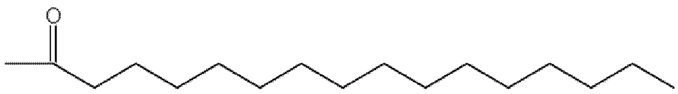

,

stearyl

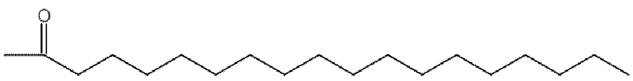

,

oleoyl

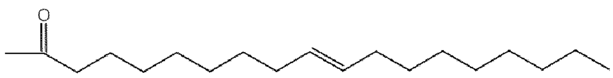

,

linoleoyl

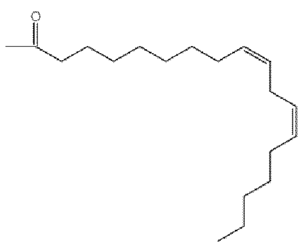

,

erucoyl

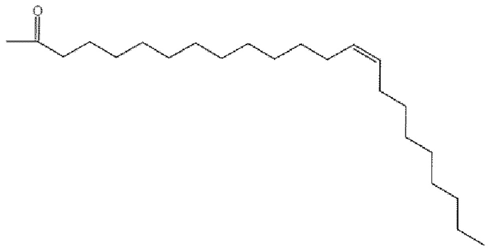

,

arachidoyl

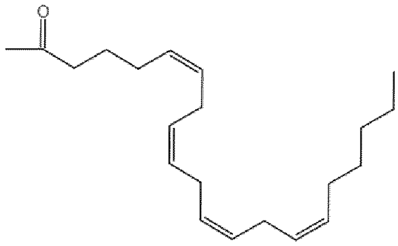

or

phytanoyl

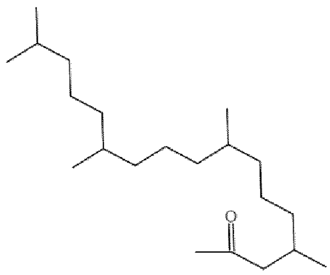

.

Further, the phospholipid molecular moiety may be selected from the group consisting of lecithin (PC) (Formula 1), Phosphatidyl ethanolamine (PE) (Formula 2), Phosphatidic Acid (PA) (Formula 4), Phosphatidyl glycerol (PG) (Formula 5), derivatives thereof, and combinations thereof.

Further, the phospholipid molecular moiety may be selected from distearoylphosphatidylcholine (DSPC), distearoylphosphatidyl ethanolamine (DSPE), distearoylphosphatidic acid (DSPA), distearoylphosphatidyl glycerol (D SPG), derivatives thereof, and combinations thereof.

Further, the phospholipid molecule moiety may be selected from the group consisting of

distearoylphosphatidylcholine (DSPC) (Formula 46)

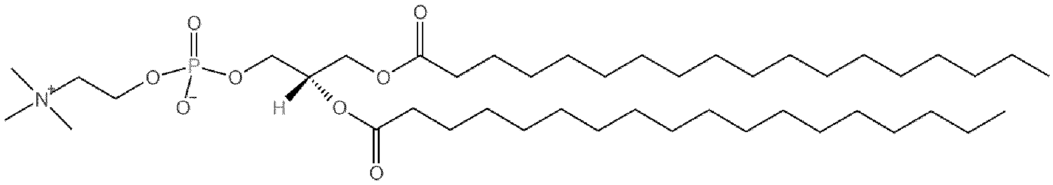

,

distearoylphosphatidyl ethanolamine (DSPE) (Formula 47)

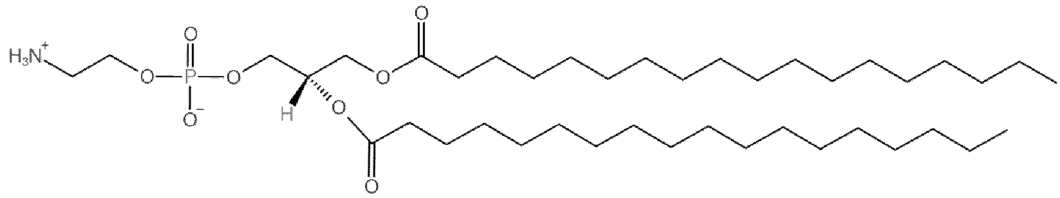

,

distearoylphosphatidic acid (DSPA) (Formula 48)

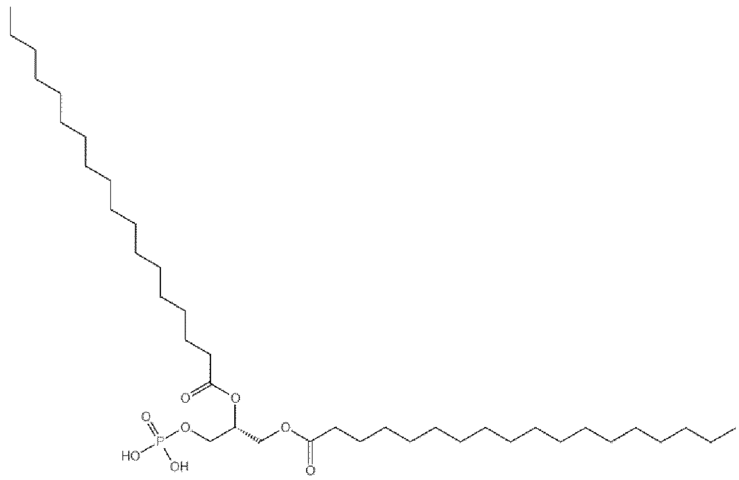

,

distearoylphosphatidyl glycerol (DSPG) (Formula 49)

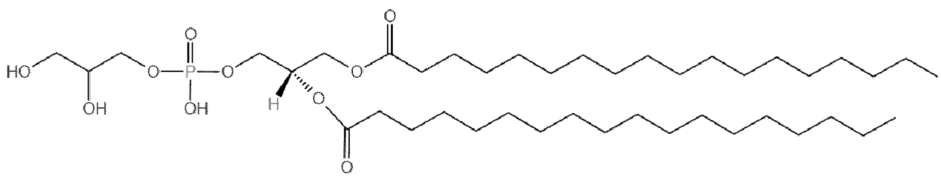

,

derivatives thereof, and combinations thereof.

Further, the phospholipid molecular moiety may be selected from DSPC (Formula 46), DSPE (Formula 47), DSPA (Formula 48) and DSPG (Formula 49).

Further, the linker molecular moiety may be selected from the group consisting of curcumin, chlorogenic acid, anthocyanins, quercetin, dihydromyricetin, hesperetin, naringenin, apigenin, catechin, tea polyphenol, epigallocatechin gallate, ellagic acid, morin, epicatechin gallate, catechin gallate, gallocatechin gallate, Pingpeimine C, derivatives thereof, and combinations thereof.

Further, the linker molecular moiety may be selected from the group consisting of

curcumin

(Formula 19) 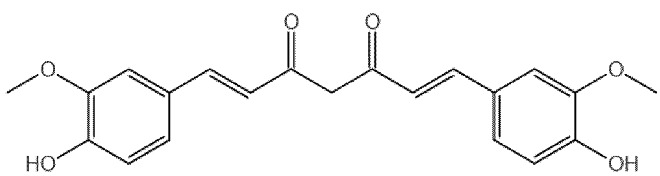 ;

chlorogenic acid

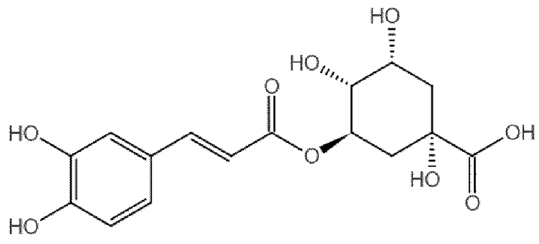

(Formula 20) ;

anthocyanidin

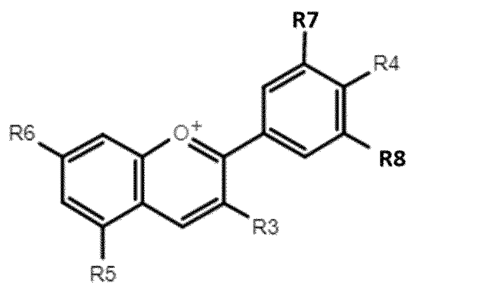

(Formula 21) ,

wherein R7 and R8 may be H, OH or $OH_3$, R3 may be H or glycosyl, R4, R5 and R6 may be OH or glycosyl;

quercetin

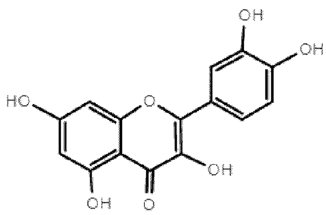

(Formula 22) ;

dihydromyricetin

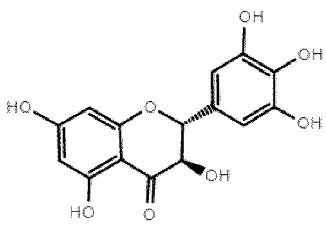

(Formula 23) ;

hesperetin

(Formula 24) 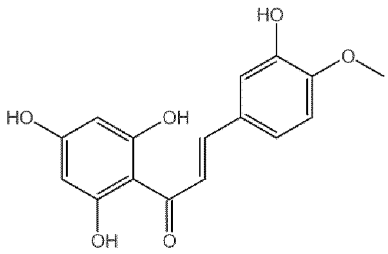 ;

naringenin

(Formula 25) 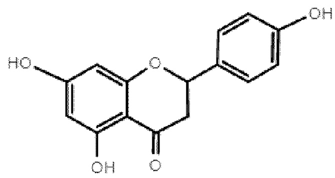 ;

apigenin

(Formula 26) 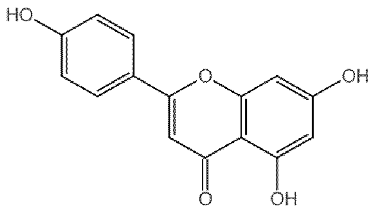 ;

catechin

(Formula 27) 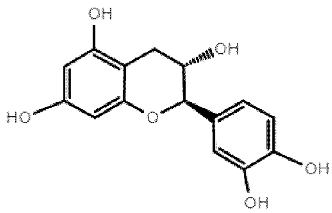 ;

tea polyphenols

(Formula 28) 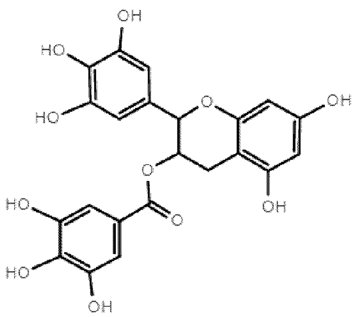 ;

epigallocatechin gallate

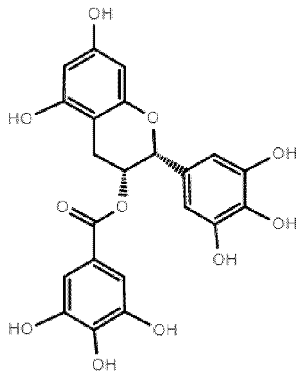

(Formula 29) ;

ellagic acid

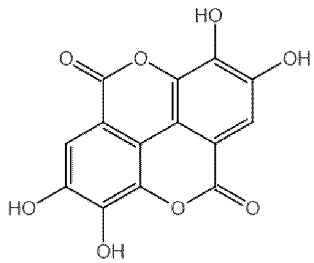

(Formula 30) ;

morin

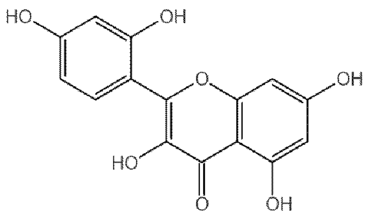

(Formula 31) ;

epicatechin gallate

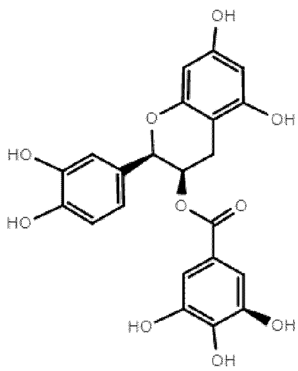

(Formula 32) ;

catechin gallate

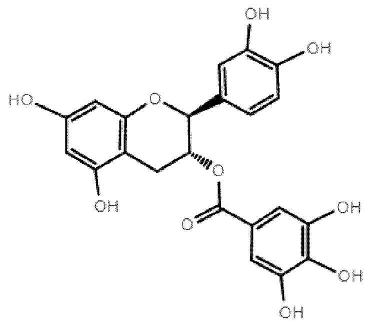

(Formula 33) ;

gallocatechin gallate

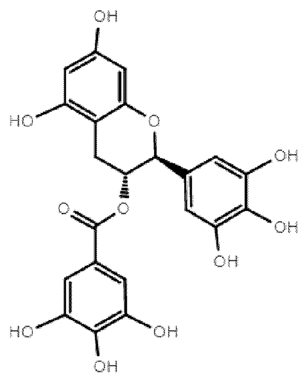

(Formula 34) ;

Pingpeimine C

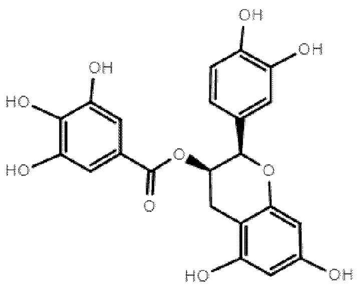

(Formula 35) ,

derivatives thereof, and combinations thereof.

Further, the linker molecular moiety may be selected from the group consisting of curcumin (Formula 19),

dihydrocurcumin

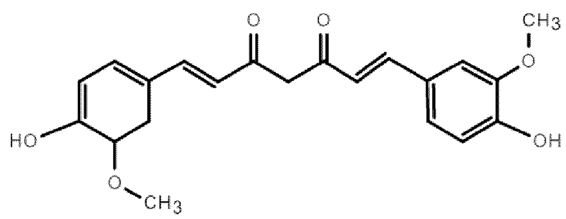

(Formula 36) ,

hexahydrocurcumin

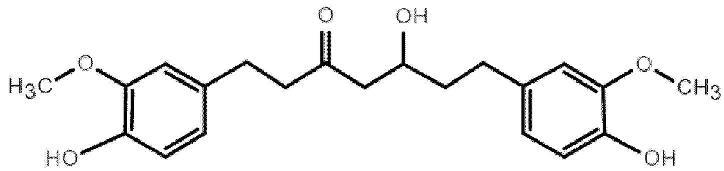

(Formula 37) ,

curcumin sulfate

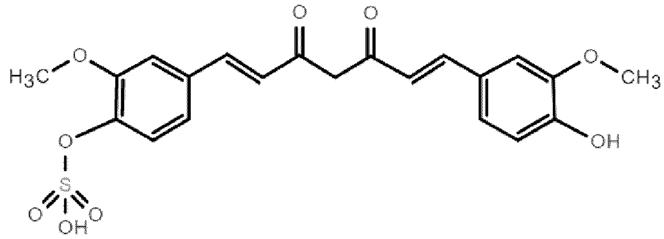

(Formula 38) ,

bisdemethoxycurcumin

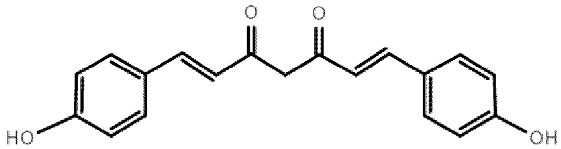

(Formula 39) ,

and combinations thereof.

Further, the linker molecular moiety may be selected from the group consisting of curcumin (Formula 19), hesperetin (Formula 24), tea polyphenol (Formula 28), derivatives thereof, and combinations thereof.

Further, the linker molecular moiety may be selected from curcumin (Formula 19), hesperetin (Formula 24) and tea polyphenol (Formula 28).

Further, the metal ion moiety may be selected from the group consisting of $Fe^{3+}$, $Ag^{+}$, $Ba^{2+}$, $Ca^{2+}$, $Cd^{2+}$, $Cu^{2+}$, $Fe^{2+}$, $Mn^{2+}$, $Mg^{2+}$, $Mo^{2+}$, $Zn^{2+}$, $Pt^{2+}$, $Au^{2+}$, $Al^{3+}$, $Ce^{3+}$, $Co^{3+}$, $Cr^{3+}$, $Eu^{3+}$, $Gd^{3+}$, $Ni^{3+}$, $W^{3+}$, $V^{3+}$, $Zr^{3+}$, and combinations thereof.

Further, the metal ion moiety may be selected from the group consisting of $Fe^{3+}$, $Ca^{2+}$, $Al^{3+}$, and combinations thereof.

Further, the metal ion moiety may be selected from $Fe^{3+}$, $Ca^{2+}$and $Al^{3+}$.

Further, the metal-chelated phospholipid complex may be made from a phospholipid molecular moiety selected from DSPC, DSPE and DSPA, a linker molecular moiety selected from curcumin, hesperetin and tea polyphenol, and a metal ion moiety selected from $Fe^{3+}$, $Ca^{2+}$ and $Al^{3+}$.

Further, the metal-chelated phospholipid complex may be made from a phospholipid molecular moiety selected from DSPC (Formula 46), DSPE (Formula 47) and DSPA (Formula 48), a linker molecular moiety selected from curcumin (Formula 19), hesperetin (Formula 24) and tea polyphenol (Formula 28), and a metal ion moiety selected from $Fe^{3+}$, $Ca^{2+}$ and $Al^{3+}$.

Further, the molar ratio among the phospholipid molecular moiety, the linker molecular moiety, and the metal ion moiety may be 1: 1: (0.5-2).

Further, the phospholipid molecular moiety may be DSPC (Formula 46), the linker molecular moiety may be curcumin (Formula 19), and the metal ion moiety may be $Fe^{3+}$.

Further, the molar ratio among the phospholipid molecular moiety, the linker molecular moiety, and the metal ion moiety may be 1:1: 1.

Further, the mole ratio among DSPC, curcumin and $Fe^{3+}/Al^{3+}$ may be 1:1: 1.

Furthermore, the mol ratio among DSPC, hesperetin and $Fe^{3+}/Al^{3+}$ may be 1:1: 1.

Furthermore, the mol ratio among DSPC, tea polyphenol and $Fe^{3+}/Al^{3+}$ may be 1:1: 2.

Further, $Fe^{3+}$ may be selected from $FeCl_3$, and $Al^{3+}$ may be selected from $Al(NO_3)_3 \cdot 9H_2O$.

An embodiment of the present disclosure provides a preparation method for a metal-chelated phospholipid

complex, comprising the steps of:

(1)forming a phospholipid complex by reacting and connecting a phospholipid molecule and a linker molecule; and

(2)forming a metal-chelated phospholipid complex by reacting the prepared phospholipid complex and a metal ion through a coordination bond.

Further, in the step of forming the phospholipid complex, the phospholipid molecule and the linker molecule are dissolved in ethanol to react, n-hexane is added and the resultant is precipitated, so as to obtain the phospholipid complex. Further, the reaction conditions include a reaction temperature of 65 °C and a duration of 2 hours.

Further, the molar ratio of the phospholipid molecule to the linker molecule is 1: 1.

Further, in the step of forming a metal-chelated phospholipid complex, the phospholipid complex and the metal ion are dissolved in ethanol to react, so as to obtain the metal-chelated phospholipid complex. Further, the reaction conditions include a reaction temperature of 60 °C and a duration of 2 hours.

Further, the molar ratio of the phospholipid complex to the metal ion is 1: (1-2).

An embodiment of the present disclosure provides a metal-chelated phospholipid complex nanoparticle (MPP) comprising (i) the above metal-chelated phospholipid complex, (ii) a particle aggregation-inhibiting conjugated lipid, wherein the particle aggregation-inhibiting conjugated lipid is not a cationic lipid or an ionizable lipid; and (iii) a non-cationic lipid or a non-ionizable lipid other than the metal-chelated phospholipid complex and the particle aggregation-inhibiting conjugated lipid. Wherein "(iii) a non-cationic lipid or a non-ionizable lipid other than the metal-chelated phospholipid complex and the particle aggregation-inhibiting conjugated lipid" may be simply referred to as "non-cationic lipid or non-ionizable lipid".

Further, the particle aggregation-inhibiting conjugated lipid may include polyethylene glycol (PEG) -lipid conjugate and/or PEG-Dialkoxypropyl (DAA).

Further, the PEG-lipid conjugate may be selected from

phosphatidyl ethanolamine-polyethylene glycol 2000 (PE-PEG 2000) (Formula 42)

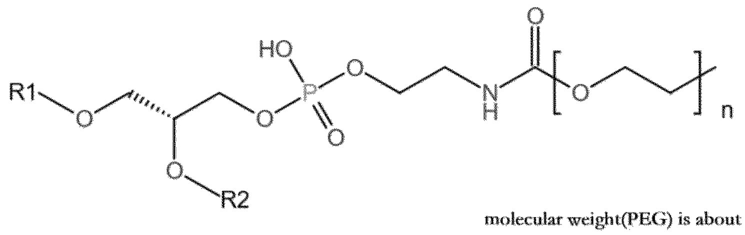

molecular weight(PEG) is about 2000

,

phosphatidyl ethanolamine-polyethylene glycol 700 (PE-PEG 700) (Formula 43)

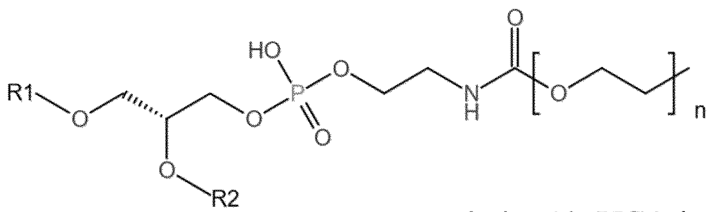

molecular weight (PEG) is about 700

,

phosphatidyl ethanolamine-polyethylene glycol 1000 (PE-PEG 1000) (Formula 44)

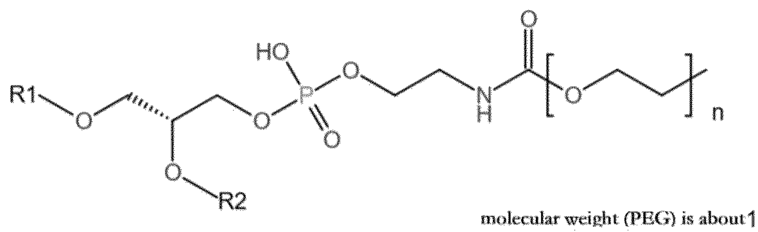

molecular weight (PEG) is about 1000

,

Phosphatidyl ethanolamine-polyethylene glycol 5000 (PE-PEG 5000) (Formula 45)

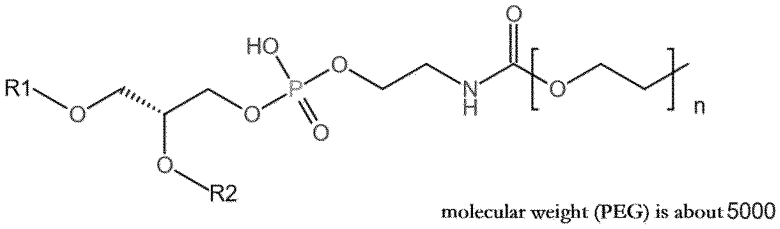


,

derivatives thereof, and combinations thereof;

R1, R2 may each independently be decanoyl, lauroyl, myristoyl, palmitoyl, stearoyl, oleoyl, linoleoyl, erucyl, arachidoyl or phytanoyl.

Further, the PEG-lipid conjugate may be selected from one or more of DSPE-PEG2000, DSPE-PEG700, DSPE-PEG1000, and DSPE-PEG 5000.

Further, the PEG-lipid conjugate may be selected from the group consisting of

DSPE-PEG2000 (Formula 53)

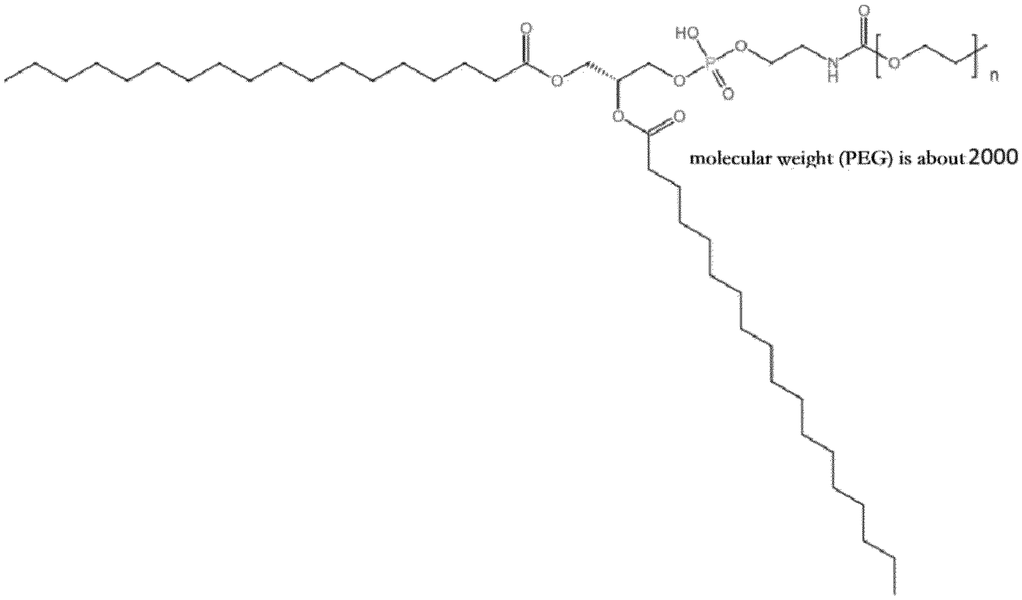


,

DSPE-PEG700 (Formula 50)

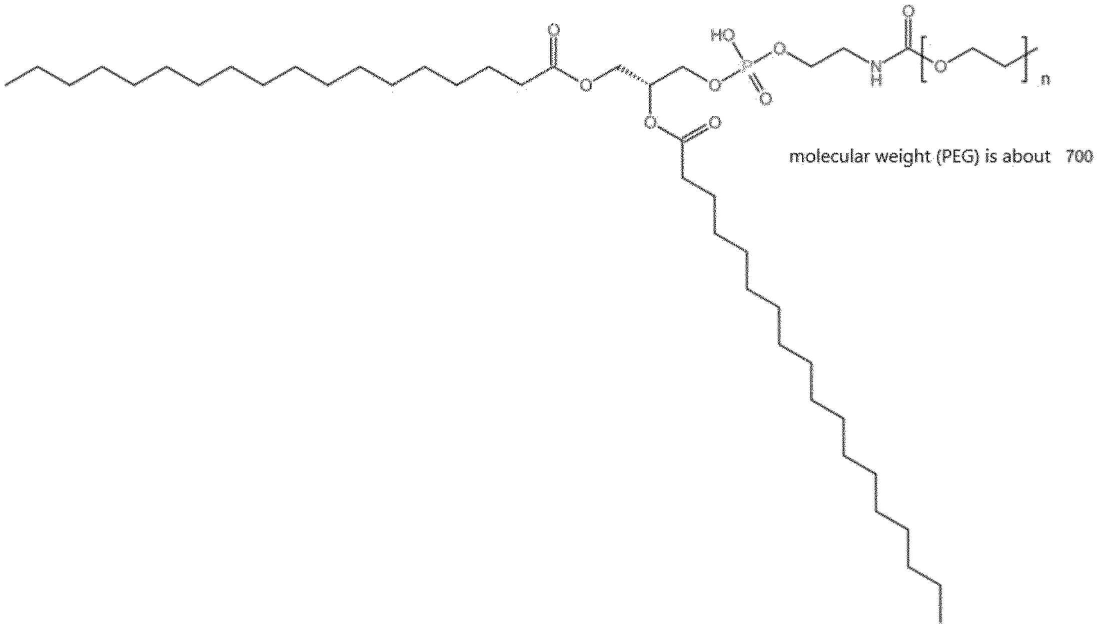


,

DSPE-PEG1000 (Formula 51)

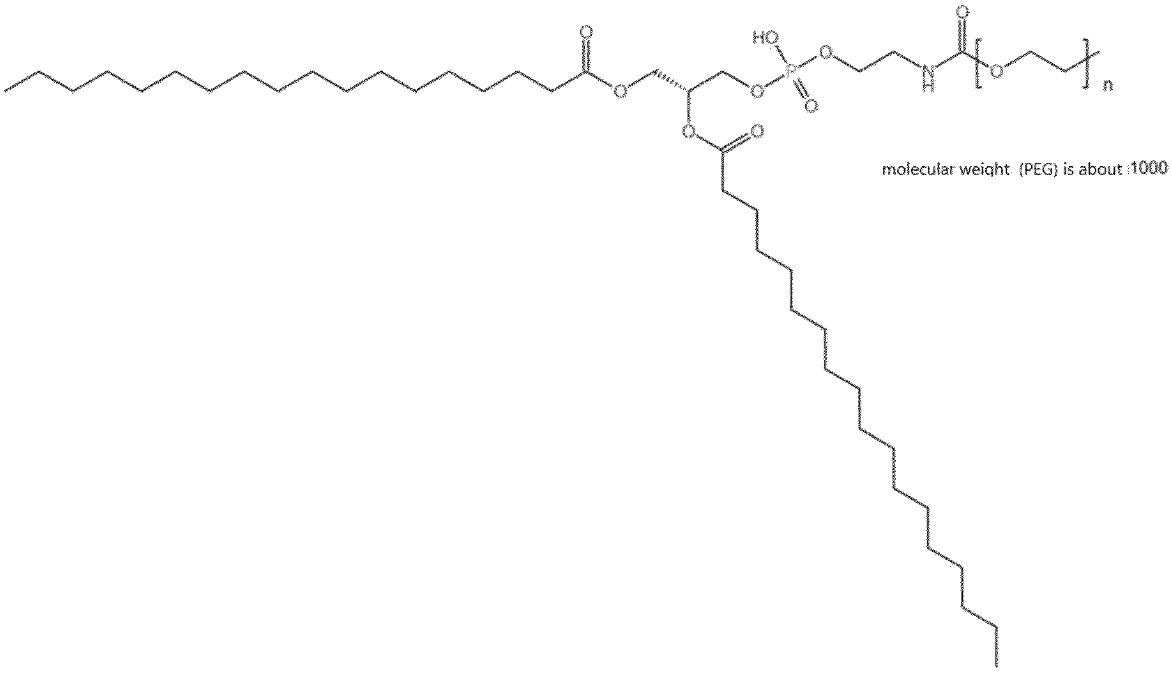


,

and DSPE-PEG5000 (Formula 52)

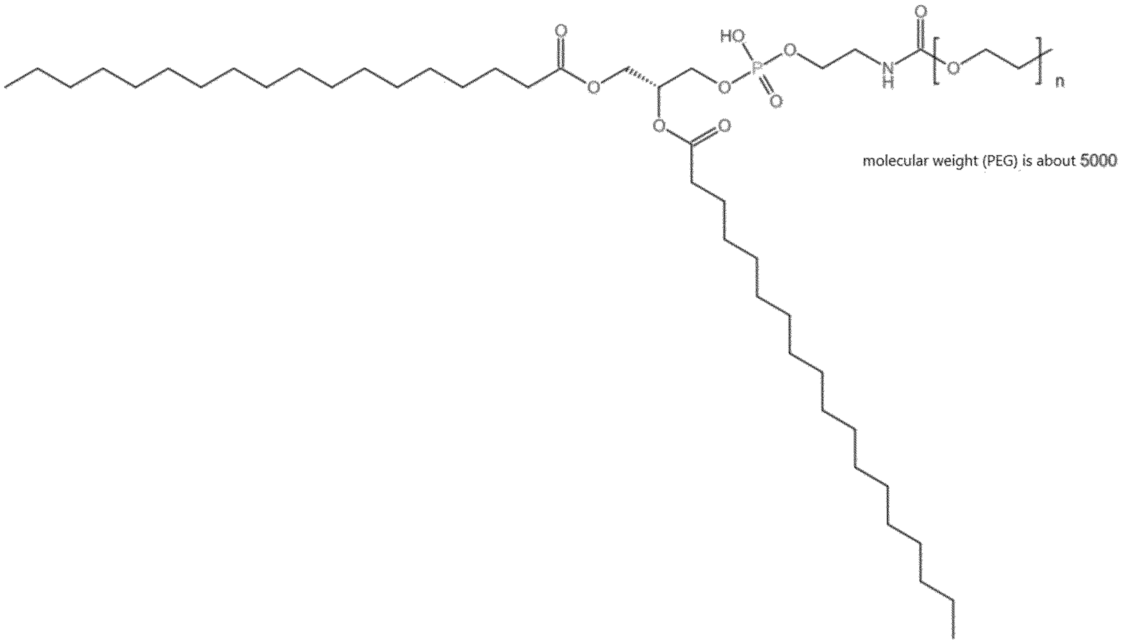


,

and combinations thereof.

Further, the PEG-lipid conjugate may be selected from DSPE-PEG2000 (Formula 53), DSPE-PEG700 (Formula 50), DSPE-PEG1000 (Formula 51) and DSPE-PEG5000 (Formula 52).

Further, the non-cationic lipid or the non-ionizable lipid other than the metal-chelated phospholipid complex and the particle aggregation-inhibiting conjugated lipid in (iii) may be selected from cholesterol, its derivatives, and combinations thereof.

Further, the non-cationic lipid or the non-ionizable lipid other than the metal-chelated phospholipid complex and the particle aggregation-inhibiting conjugated lipid in (iii) may be selected from cholesterol

(Formula 40) 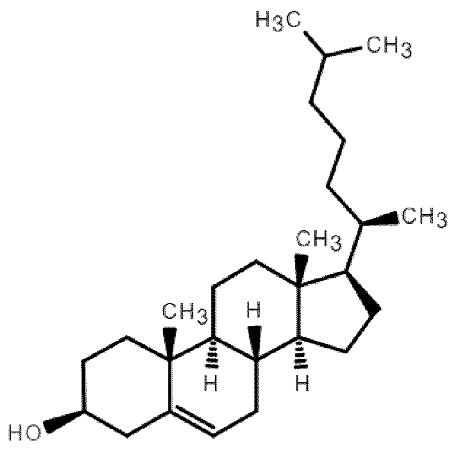 ,

its derivatives, and combinations thereof.

Further, the non-cationic lipid or the non-ionizable lipid in (iii) may be cholesterol (Formula 40).

Further, the non-cationic lipid or the non-ionizable lipid other than the metal-chelated phospholipid complex and the particle aggregation-inhibiting conjugated lipid of (iii) may further comprise, in addition to cholesterol, one or more selected from the group consisting of lecithin PC, phosphatidyl ethanolamine PE, phosphatidyl serine PS, phosphatidic acid PA, phosphatidyl glycerol PG, ceramide-1-phosphate CP, phosphatidyl inositol PI, phosphatidyl threonine PT, sphingomyelin SM, lysolecithin LPC, lysophosphatidyl ethanolamine LPE, lysophosphatidylserine LPS, lysophosphatidic acid LPA, lysophosphatidyl glycerol LPG, lysophosphatidylinositol LPI, lysophosphatidylthreonine LPT, lysosphingomyelin LSM, sphingosine 1-phosphate S1P, cholesterol sulfate, and derivatives thereof.

Further, the non-cationic lipid or the non-ionizable lipid other than the metal-chelated phospholipid complex and

the particle aggregation-inhibiting conjugated lipid in (iii) may further comprise, in addition to cholesterol, one or more selected from the group consisting of

lecithin (PC)

(Formula 1) 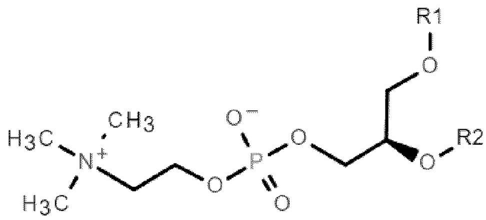 ,

phosphatidyl ethanolamine (PE)

(Formula 2) 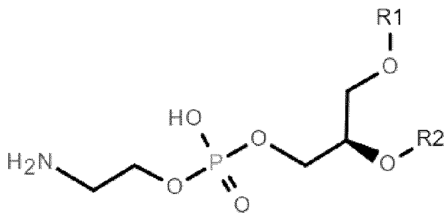 ,

phosphatidylserine (PS)

(Formula 3) 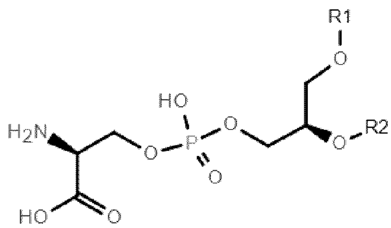 ,

phosphatidic Acid (PA)

(Formula 4) 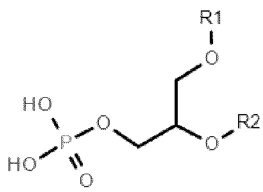 ,

phosphatidyl glycerol (PG)

(Formula 5) 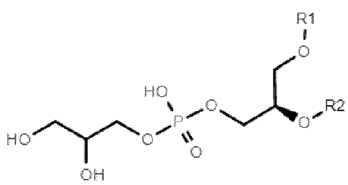 ,

ceramide-1-phosphate (CP)

(Formula 6) 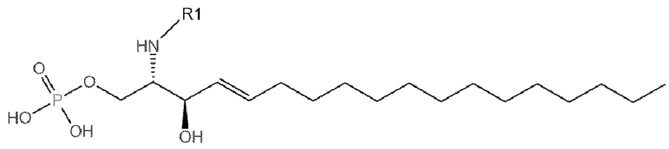 ,

phosphatidyl inositol (PI)

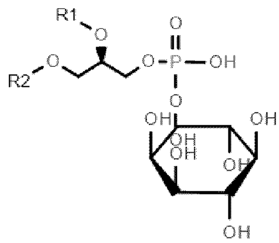

(Formula 7) ,

phosphatidyl threonine (PT)

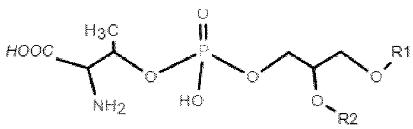

(Formula 8) ,

sphingomyelin (SM)

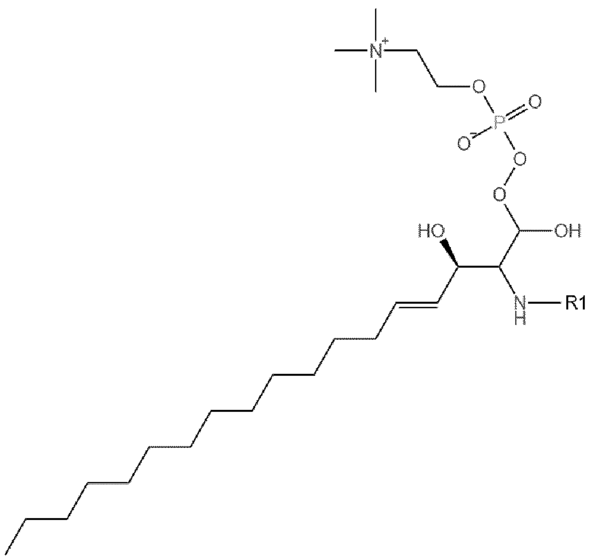

(Formula 9) ,

lysolecithin (LPC)

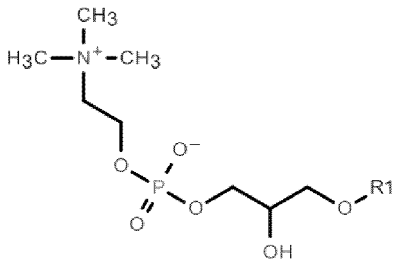

(Formula 10) ,

lysophosphatidyl ethanolamine (LPE)

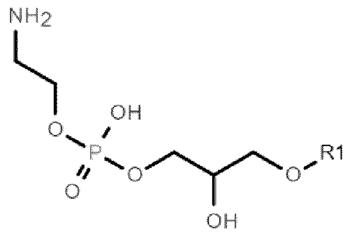

(Formula 11) ,

lysophosphatidylserine (LPS)

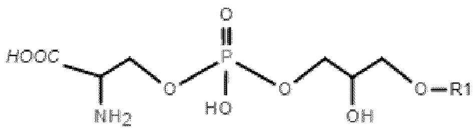

(Formula 12) ,

lysophosphatidic acid (LPA)

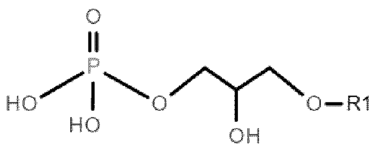

(Formula 13) ,

lysophosphatidyl glycerol (LPG)

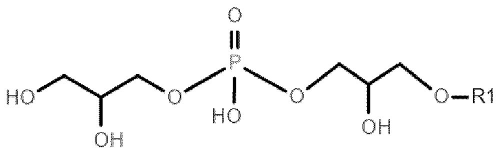

(Formula 14) ,

lysophosphatidylinositol (LPI)

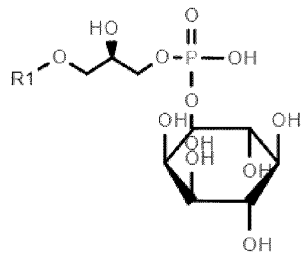

(Formula 15) ,

lysophosphatidylthreonine (LPT)

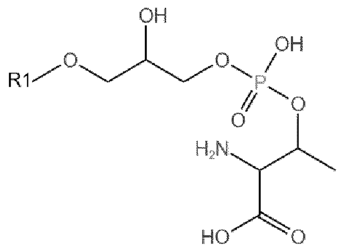

(Formula 16) ,

lysosphingomyelin (LSM)

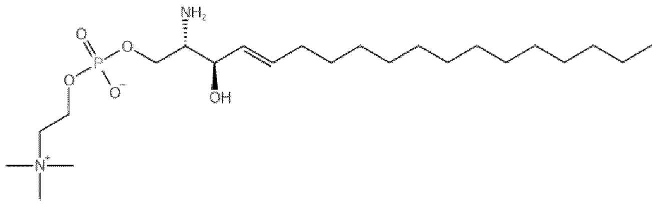

(Formula 17) ,

sphingosine-1-phosphate (S1P)

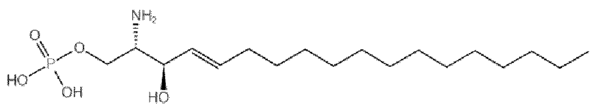

(Formula 18) ,

cholesterol sulfate

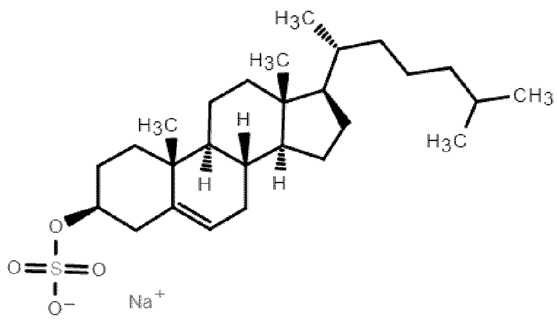

(Formula 41) ,

and derivatives thereof;
wherein, R1 and R2 may be decanoyl, lauroyl, myristoyl, palmitoyl, stearoyl, oleoyl, linoleoyl, erucyl, arachidoyl or phytanoyl.

Further, the non-cationic lipid or the non-ionizable lipid of (iii) other than the metal-chelated phospholipid complex

and the particle aggregation-inhibiting conjugated lipid may comprise cholesterol, and one or more selected from DSPC, DSPE, DSPA and DSPG.

Further, the non-cationic lipid or the non-ionizable lipid of (iii) other than the metal-chelated phospholipid complex and the particle aggregation-inhibiting conjugated lipid may comprise cholesterol (Formula 40), and one or more selected from DSPC (Formula 46), DSPE (Formula 47), DSPA (Formula 48), and DSPG (Formula 49).

Further, the non-cationic lipid or non-ionizable lipid described in (iii) may comprise cholesterol (Formula 40) and DSPC (Formula 46).

Further, the metal-chelated phospholipid complex nanoparticle may made from (i) a metal-chelated phospholipid complex, (ii) a particle aggregation-inhibiting conjugated lipid, and (iii) a non-cationic lipid or a non-ionizable lipid other than the metal-chelated phospholipid complex and the particle aggregation-inhibiting conjugated lipid, wherein the molar proportion of metal-chelated phospholipid complex in starting material is 10% to 40%, the molar proportion of the particle aggregation-inhibiting conjugated lipid in starting material is 2% to 10%, the molar proportion of the cholesterol in starting material is 35% to 75%, and the molar proportion of the non-cationic lipid or the non-ionizable lipid other than cholesterol in the starting material is 0% to 40%.

Further, the metal-chelated phospholipid complex nanoparticle may made from (i) a metal-chelated phospholipid complex, (ii) a particle aggregation-inhibiting conjugated lipid, and (iii) a non-cationic lipid or a non-ionizable lipid, wherein the molar proportion of metal-chelated phospholipid complex in starting material may be 5% to less than 10%, the molar proportion of the particle aggregation-inhibiting conjugated lipid in starting material may be 2% to 10%, the molar proportion of the cholesterol in starting material may be 15% to less than 35%, 35% to 75% or more than 75% to 80%, and the molar proportion of the non-cationic lipid or the non-ionizable lipid other than cholesterol in the starting material may be 0% to 40% or more than 40% to 51%; or

the molar proportion of metal-chelated phospholipid complex in starting material may be 40% to 50%, the molar proportion of the particle aggregation-inhibiting conjugated lipid in starting material may be 2% to 10%, the molar proportion of the cholesterol in starting material may be 15% to less than 35%, 35% to 75%, or more than 75% to 80%, and the molar proportion of the non-cationic lipid or the non-ionizable lipid other than cholesterol in the starting material may be 0% to 40% or more than 40% to 51%; or

the molar proportion of metal-chelated phospholipid complex in starting material may be 10% to 40%, the molar proportion of the particle aggregation-inhibiting conjugated lipid in starting material may be 2% to 10%, the molar proportion of the cholesterol in starting material may be 15% to less than 35%, or more than 75% to 80%, and the molar proportion of the non-cationic lipid or the non-ionizable lipid other than cholesterol in the starting material may be 0% to 40% or more than 40% to 51%.

Further, the molar proportion of the metal-chelated phospholipid complex in starting material may be 7% to less than 10%, 10% to 30%, or 20% to 30%, preferably 25%.

Further, the molar proportion of the particle aggregation-inhibiting conjugated lipid in starting material may be 3% to 10% or 5% to 10%, preferably 10%.

Further, the molar proportion of the cholesterol in starting material may be 15% to less than 35%, 35% to 56%, or 35% to 55%, preferably 40%.

Further, the molar proportion of the non-cationic lipid or the non-ionizable lipid other than cholesterol in starting material may be 5% to 30%, 25% to 40%, greater than 40% to 45%, or 20% to 25%.

Further, the molar proportion of metal-chelated phospholipid complex in starting material may be 15% to 25%, the molar proportion of the particle aggregation-inhibiting conjugated lipid in starting material may be 4% to 10%, the molar proportion of the cholesterol in starting material may be 40% to 46%, and the molar proportion of DSPC in the starting material may be 25% to 35%, and the metal ion moiety in the metal-chelated phospholipid complex may be $Fe^{3+}$.

Further, the molar proportion of metal-chelated phospholipid complex (the metal ion moiety is $Fe^{3+}$) in starting material may be 15%, the molar proportion of the particle aggregation-inhibiting conjugated lipid in starting material may be 4%, the molar proportion of the cholesterol in starting material may be 46%, and the molar proportion of DSPC in the starting material may be 35%; or wherein the molar proportion of metal-chelated phospholipid complex (the metal ion moiety is $Fe^{3+}$) in starting material may be 25%, the molar proportion of the particle aggregation-inhibiting conjugated lipid in starting material may be 10%, the molar proportion of the cholesterol in starting material may be 40%, and the molar proportion of DSPC in the starting material may be 25%.

Further, the molar proportion of metal-chelated phospholipid complex in starting material may be 10% to 30%, the molar proportion of the particle aggregation-inhibiting conjugated lipid in starting material may be 3% to 10%, the molar proportion of the cholesterol in starting material may be 35% to 56%, and the molar proportion of DSPC in the starting material may be 34% to 40%, and the metal ion moiety in the metal-chelated phospholipid complex may be $Al^{3+}$.

Further, the molar proportion of metal-chelated phospholipid complex in starting material may be 10% to 30%, the molar proportion of the particle aggregation-inhibiting conjugated lipid in starting material may be 3% to 10%, the molar

proportion of the cholesterol in starting material may be 35% to 56%, the molar proportion of DSPC in the starting material may be 40% to 45%, and the metal ion moiety in the metal-chelated phospholipid complex may be $Al^{3+}$; or
The molar proportion of metal-chelated phospholipid complex in starting material may be 10% to 30%, the molar proportion of the particle aggregation-inhibiting conjugated lipid in starting material may be 3% to 10%, the molar proportion of the cholesterol in starting material may be 15% to 35%, the molar proportion of DSPC in the starting material may be 34% to 40% or more than 40% to 45%, and the metal ion moiety in the metal-chelated phospholipid complex may be $Al^{3+}$; or
The molar proportion of metal-chelated phospholipid complex in starting material may be 7% to less than 10%, the molar proportion of the particle aggregation-inhibiting conjugated lipid in starting material may be 3% to 10%, the molar proportion of the cholesterol in starting material may be 15% to less than 35% or 35% to 56%, the molar proportion of DSPC in the starting material may be 34% to 40% or more than 40% to 45%, and the metal ion moiety in the metal-chelated phospholipid complex may be $Al^{3+}$.

Further, the molar proportion of the metal-chelated phospholipid complex (metal ion moiety is $Al^{3+}$) may be 7% in the starting material, the molar proportion of the particle aggregation-inhibiting conjugated lipid in starting material may be 3%, the molar proportion of the cholesterol in starting material may be 56%, and the molar proportion of DSPC in the starting material may be 34%.

Further, the metal-chelated phospholipid complex may be prepared from DSPC, curcumin and $Fe^{3+}$at a molar ratio of 1:1:1, the particle aggregation-inhibiting conjugated lipid may be DSPE-PEG2000, the non-cationic lipid or non-ionizable lipid may be cholesterol and DSPC, the molar proportion of metal-chelated phospholipid complex in starting material may be 15%, the molar proportion of DSPE-PEG200 in starting material may be 4%, the molar proportion of the cholesterol in starting material may be 46%, the molar proportion of DSPC in the starting material may be 35%.

Further, the metal-chelated phospholipid complex may be prepared from DSPC, curcumin and $Fe^{3+}$ at a molar ratio of 1:1:1, the particle aggregation-inhibiting conjugated lipid may be DSPE-PEG2000, the non-cationic lipid or non-ionizable lipid may be cholesterol and DSPC, the molar proportion of metal-chelated phospholipid complex in starting material may be 25%, the molar proportion of DSPE-PEG200 in starting material may be 10%, the molar proportion of the cholesterol in starting material may be 40%, the molar proportion of DSPC in the starting material may be 25%.

Further, the metal-chelated phospholipid complex may be prepared from DSPC, curcumin and $Al^{3+}$ at a molar ratio of 1:1:1, the particle aggregation-inhibiting conjugated lipid may be DSPE-PEG2000, the non-cationic lipid or the non-ionizable lipid may be cholesterol and DSPC, the molar proportion of metal-chelated phospholipid complex in starting material may be 7%, the molar proportion of DSPE-PEG200 in starting material may be 3%, the molar proportion of the cholesterol in starting material may be 56%, the molar proportion of DSPC in the starting material may be 34%.

An embodiment of the present disclosure provides a preparation method for the above-described metal-chelated phospholipid complex nanoparticle, comprising mixing (i) a metal-chelated phospholipid complex, (ii) a particle aggregation-inhibiting conjugated lipid, and (iii) a non-cationic lipid or a non-ionizable lipid other than the metal-chelated phospholipid complex and the particle aggregation-inhibiting conjugated lipid to obtain the metal-chelated phospholipid complex nanoparticle.

An embodiment of the present disclosure provides a drug-lipid particle comprising:

(a) a drug being a negatively charged molecule; and
(b) a metal-chelated phospholipid complex nanoparticle (MPP) of the present disclosure.

Further, the drug may be encapsulated in the metal-chelated phospholipid complex nanoparticles.

Further, the drug may be selected from the group consisting of nucleic acid, protein, polypeptide, small molecule, nucleic acid analog, protein analog, polypeptide analog, and combinations thereof.

Further, the nucleic acid may be selected from the group consisting of mRNA, siRNA, sgRNA, ASO, circRNA, microRNA, DNA, ecDNA, artificial nucleic acid, and combinations thereof.

Further, the nucleic acid may be an mRNA sequence shown in SEQ ID No.1 for encoding eGFP, an mRNA sequence shown in SEQ ID No.2 for encoding a receptor binding domain RBD of a novel coronavirus S1 subunit, an mRNA sequence shown in SEQ ID No.3 for encoding NY-ESO-1 (New York esophageal squamous cell carcinoma 1), an siRNA sequence of a Bcl-2 gene having an antisense strand shown in SEQ ID No.4 and a sense strand shown in SEQ ID No.21, an siRNA sequence of a PLK1 gene having an antisense strand shown in SEQ ID No.6 and a sense strand showed in SEQ ID No.23, an siRNA sequence of a Gal-1 gene shown in SEQ ID No.8, an ASO sequence of a STAT-3 gene shown in SEQ ID No.10, an ASO sequence of an alpha-syn gene shown in SEQ ID No.12, an ASO sequence of a Bcl-2 gene shown in SEQ ID No.14, an mRNA sequence shown in SEQ ID No.16 for encoding a wild-type novel coronavirus S protein, a double-stranded DNA sequence having an antisense strand shown in SEQ ID No.17 and a sense strand shown in SEQ ID No.25, a single-stranded DNA shown in SEQ ID No.18, or a siRNA sequence of a B7-H4 gene having a sense strand shown in SEQ ID No.19 and an antisense strand shown in SEQ ID No. 26.

An embodiment of the present disclosure provides a preparation method of the above drug-lipid particle, wherein a drug is encapsulated in the metal-chelated phospholipid complex nanoparticles to obtain the drug-lipid particle.

Further, the metal-chelated phospholipid complex, the particle aggregation-inhibiting conjugated lipid, and the non-cationic lipid or the non-ionizable lipid may be dissolved in an organic compound to form an organic phase, the drug may be dissolved in a buffer to form an aqueous phase, the organic phase may be mixed with the aqueous phase to obtain the drug-lipid particle.

Further, the organic compound may be ethanol.

Further, the buffer may be an enzyme-free PBS buffer.

Further, mixing means of the organic phase and the aqueous phase may include micro-fluidic chip or ultrasound.

Further, (a) a drug, (i) a metal-chelated phospholipid complex, (ii) a particle aggregation-inhibiting conjugated lipid, and (iii) a non-cationic lipid or a non-ionizable lipid may be mixed to obtain the drug-lipid particle.

An embodiment of the present disclosure provides a use of the above metal-chelated phospholipid complex in a nucleic acid delivery system. Further, the nucleic acid delivery system is used for introducing a nucleic acid into a cell. Further, the nucleic acid may be used for silencing expression of a target sequence in a mammalian subject, for delivering a drug in a mammal body, for delivering a drug into mammalian cells from the body, or for treating a disease or disorder in a mammal.

An embodiment of the present disclosure provides a use of the above metal-chelated phospholipid complex nanoparticle or the above drug-lipid particle in a composition for the delivery of a drug. Further, the composition may be used for introducing a drug into a cell. The composition may be a medicament. Further, the medicament may be used for silencing expression of a target sequence in a mammalian subject, for delivering a drug in a mammal body, for delivering a drug into mammalian cells from the body, or for treating a disease or disorder in a mammal.

An embodiment of the present disclosure provides a use of the above metal-chelated phospholipid complex or metal-chelated phospholipid complex nanoparticle or drug-lipid particle or medicament in prevention/treatment of a disease or disorder in a mammal.

In the above technical solutions, the mammal may be a human. Further, the disease or disorder is associated with expression of a gene comprising a target sequence for a drug. Further, the disease or disorder may include cancer, viral infection, autoimmune disease, diabetes, and Alzheimer's disease. Further, the viral infection may include hepatitis A, hepatitis B, hepatitis C, SARS-Cov-2 (2019 novel coronavirus), HIV (AIDS virus), HPV (human papilloma virus), influenza, smallpox and syphilis. Further, the cancer may include liver cancer, glioma, melanoma, lung cancer, pancreatic cancer, and breast cancer.

Further, the medicament may be a vaccine. Further, the route of administration of the medicament may include intrathecal injection, intramuscular injection, intracranial injection, intravenous injection, and intratumoral injection.

An embodiment of the present disclosure provides a medicament comprising a metal-chelated phospholipid complex or a metal-chelated phospholipid complex nanoparticle or a drug-lipid particle of the present disclosure.

Further, the medicament may be a vaccine.

Further, the vaccine may be a novel coronavirus vaccine.

Compared with the prior art, the present disclosure has the following technical effects.

The metal-chelated phospholipid complex provided by the disclosure mainly has a function in adsorbing drugs with negative charges and self-assembling with other lipids into metal-chelated phospholipid complex nanoparticles (MPP), so as to avoid the use of cationic lipid or ionizable lipid under the condition of ensuring the effectiveness which is not less than that of LNP, so that the toxicity of the drug-lipid particle is greatly reduced compared with LNP, the biological safety is remarkably improved, and the delivery of drugs with negative charges in organisms is facilitated.

## Drawings

In order to more clearly illustrate the detailed description of the present disclosure or the technical solutions in the prior art, the drawings used in the detailed description or the prior art will be briefly described below. It is appreciated that the drawings in the following description illustrate some embodiments of the present disclosure, and other drawings can be obtained by those of skill in the art without creative efforts.

FIG.1-1 shows the percentage of eGFP-positive cells resulting from 293T transfection of eGFP-mRNA@MPP ($Fe^{3+}$) provided in example 3.5.1 of the present disclosure;

FIG.1-2 shows the expression level of RBD resulting from 293T transfection of RBD-mRNA@MPP ($Fe^{3+}$) provided in example 3.5.1 of the present disclosure;

FIG.1-3 shows the humoral immunity-inducing capability of RBD-mRNA@MPP ($Fe^{3+}$) provided in example 3.5.1 of the present disclosure;

FIG.1-4 shows the humoral immunity-inducing capability of NY-ESO-1-mRNA@MPP ($Fe^{3+}$) provided in example 3.5.1 of the present disclosure;

FIG.1-5 shows the cellular immunity-inducing capability of RBD-mRNA@MPP ($Fe^{3+}$) provided in example 3.5.1 of the present disclosure;

FIG.1-6 shows the cellular immunity-inducing capability of NY-ESO-1-mRNA@MPP ($Fe^{3+}$) provided in example 3.5.1 of the present disclosure;

FIG.1-7 shows the percentage of eGFP-positive cells resulting from 293T transfection of eGFP-mRNA@MPP ($Al^{3+}$) provided in example 3.5.2 of the present disclosure;

FIG.1-8 shows the expression level of RBD resulting from 293T transfection of RBD-mRNA@MPP ($Al^{3+}$) provided in example 3.5.2 of the present disclosure;

FIG.1-9 shows the humoral immunity-inducing capability of RBD-mRNA@MPP ($Al^{3+}$) provided in example 3.5.2 of the present disclosure;

FIG.1-10 shows the humoral immunity-inducing capability of NY-ESO-1-mRNA@MPP ($Al^{3+}$) provided in example 3.5.2 of the present disclosure;

FIG.1-11 shows the cellular immunity-inducing capability of RBD-mRNA@MPP ($Al^{3+}$) provided in example 3.5.2 of the present disclosure;

FIG.1-12 shows the cellular immunity-inducing capability of NY-ESO-1-mRNA@MPP ($Al^{3+}$) provided in example 3.5.2 of the present disclosure;

FIG.1-13 shows the target gene-silencing capability of Bcl-2-siRNA@MPP ($Fe^{3+}$) provided in example 3.6.1 of the present disclosure;

FIG.1-14 shows the target gene-silencing capability of PLK1-siRNA@MPP ($Fe^{3+}$) provided in example 3.6.1 of the present disclosure;

FIG.1-15 shows the target gene-silencing capability of Gal-1-siRNA@MPP ($Fe^{3+}$) provided in example 3.6.1 of the present disclosure;

FIG.1-16 shows the target gene-silencing capability of Bcl-2-siRNA@MPP ($Al^{3+}$) provided in example 3.6.2 of the present disclosure;

FIG.1-17 shows the target gene-silencing capability of PLK1-siRNA@MPP ($Al^{3+}$) provided in example 3.6.2 of the present disclosure;

FIG.1-18 shows the target gene-silencing capability of Gal-1-siRNA@MPP ($Al^{3+}$) provided in example 3.6.2 of the present disclosure;

FIG.1-19 shows the capability of of STAT3-ASO@MPP ($Fe^{3+}$) to silence a target gene of a cell as provided in example 3.7.1 of the present disclosure;

FIG.1-20 shows the capability of $\alpha$ -syn-ASO@MPP ($Fe^{3+}$) to silence a target gene of a cell provided in example 3.7.1 of the present disclosure;

FIG.1-21 shows the capability of Bcl-2-ASO@MPP ($Fe^{3+}$) to silence a target gene of a cell provided by example 3.7.1 of the present disclosure;

FIG.1-22 shows the capability of STAT3-ASO@MPP ($Al^{3+}$) to silence a target gene of a cell provided in example 3.7.2 of the present disclosure;

FIG.1-23 shows the capability of $\alpha$ -syn-ASO@MPP ($Al^{3+}$) to silence a target gene of a cell provided in example 3.7.2 of the present disclosure;

FIG.1-24 shows the capability of Bcl-2-ASO@MPP ($Al^{3+}$) to silence a target gene of a cell provided in example 3.7.2 of the present disclosure;

FIG.1-25 shows the expression levels of S protein resulting from 293T transfection of S-mRNA@MPP ($Fe^{3+}$) provided in example 3.8.1 of the present disclosure;

FIG. 1-26 shows the functions of drug (dsDNA and ssDNA) -lipid particles ($Fe^{3+}$) provided in example 3.8.1 of the present disclosure;

FIG.1-27 shows the expression level of S protein resulting from 293T transfection of S-mRNA@MPP ($Al^{3+}$) provided in example 3.8.2 of the present disclosure;

FIG.1-28 shows the functions of drug (dsDNA and ssDNA) -lipid particles ($Al^{3+}$) provided in example 3.8.2 of the present disclosure;

FIG.2-1 is a graph of differential scanning calorimetry of phospholipid complex provided in example 4.1 of the present disclosure;

FIG.2-2 is a graph of the UV absorption of the metal-chelated phospholipid complex ($Fe^{3+}$) provided in example 4.1 of the present disclosure;

FIG.2-3 is a graph of the UV absorption of metal-chelated phospholipid complex ($Al^{3+}$) provided in example 4.2 of the present disclosure;

FIG. 2-4 shows the illustration of the $Fe^{3+}$ detached from metal-chelated phospholipid complex under low pH conditions (pH = 5.0) provided by example 5 of the present disclosure;

FIG. 2-5 shows the elemental analyses of drug-metal-chelated phospholipid complex nanoparticle ($Fe^{3+}$) provided in example 6.1 of the present disclosure;

FIG. 2-6 shows the electron microscopy analyses of MPP in the drug-metal-chelated phospholipid complex nanoparticle ($Al^{3+}$) provided in example 6.2 of the present disclosure;

FIG.2-7 shows the efficiency of drug-lipid particles encapsulating nucleic acids (mRNA and siRNA) in example 7 of the present disclosure;

FIG.2-8 shows escape capability of nucleic acid of siRNA/mRNA@MPP and siRNA/mRNA @ LNP provided in example 8 from lysosome of the present disclosure;

FIG.2-9 shows eGFP positive cell rates of MPP and LNP provided in example 9 of the present disclosure;

FIG.2-10 shows a comparison of mRNA expression promoting capabilities of MPP and LNP provided in example 10 of the present disclosure;

FIG.2-11 shows a comparison of the humoral immunity-inducing capabilities of MPP and LNP provided by example 10 of the present disclosure;

FIG.2-12 shows a comparison of the cellular immunity-inducing capabilities of MPP and LNP provided in example 10 of the present disclosure;

FIG. 3-1 shows liver cancer treatment results of the drug-metal-chelated phospholipid complex nanoparticles via intratumoral injection provided in example 13 of the present disclosure.

## Detailed Description

### Definition

For the purpose of illustration, specific terms in the specification, embodiments, and attached claims are set forth collectively. Unless otherwise defined, the scientific and technical terms used herein have the same meaning as commonly understood and used by those of skill in the art. In addition, unless otherwise expressly specified, it should be understood that singular terms shall include the same plural forms, and plural terms shall include the singular forms. In particular, unless indicated to the contrary, the terms "at least one" and "one or more" as used herein and in the appended claims include one, two, three or more.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The term "lipids" refers to a group of organic compounds including, but not limited to, esters of fatty acids. They are generally divided into three categories: simple lipids, compound lipids, and derived lipids. Simple lipids include lipids, oils and waxes. Compound lipids include phospholipids and glycolipids. Examples of Derived lipids may be steroids.

The term "lipid vesicles" refers to lipid compositions that are capable of delivering a compound including, but not limited to, liposomes, wherein an aqueous volume is encapsulated by an amphiphilic lipid bilayer; or wherein the lipids coat an interior comprising a large molecular component, such as comprising mRNA, with a reduced aqueous interior; or lipid aggregates or micelles, wherein the encapsulated ingredient is contained within a relatively disordered lipid mixture. Herein, metal-chelated phospholipid complex nanoparticles (MMP) are "lipid vesicles". A drug such as a nucleic acid mRNA, is encapsulated in MMP as an encapsulated ingredient, which may be fully encapsulated and/or partially encapsulated.

The term "phospholipid" refers to a lipid containing a phosphate group, and belongs to a complex lipid, also called phospholipids. Phospholipid is the main component constituting biological membranes and is divided into two major classes, glycerophospholipid and sphingomyelin, which are composed of glycerol and sphingosine, respectively. Phospholipid is an amphiphilic molecule with a hydrophilic head containing nitrogen or phosphorus at one end and a hydrophobic (oleophilic) head having long hydrocarbyl chain at the other end. For this reason, the phospholipid molecules have their hydrophilic ends close to each other and their hydrophobic ends close to each other, and often form a phospholipid bilayer, i.e., a cell membrane structure, together with other molecules such as protein, glycolipid, and cholesterol.

The phrase "phospholipid molecular moiety" as used herein refers to the structure originated from a phospholipid molecule that has been reacted with other substances.

The phrase "linker molecular moiety" as used herein refers to a structure originated from a linker molecule that has reacted with other substances.

The phrase "metal ion moiety" as used herein refers to a structure originated from a metal ion that has reacted with other substances.

The phrase "phospholipid complex" as used herein refers to a complex formed by the reaction of the above-mentioned phospholipid molecular moiety with phosphate group and the above-mentioned linker molecular moiety.

The phrase "metal-chelated phospholipid complex" as used herein refers to a complex formed by the reaction of the above-mentioned phospholipid molecular moiety with phosphate group, the above-mentioned linker molecular moiety, and the above-mentioned metal ion moiety, wherein the phospholipid molecular moiety is linked to the linker molecular moiety, the linker molecular moiety is linked to the metal ion moiety through a coordination bond, and the metal-chelated phospholipid complex is not a cationic lipid or an ionizable lipid.

The term "ionizable lipid" refers to lipids that contain a positively charged ionizable amine group that can be protonated with a positive charge at lower pH and uncharged at physiological pH.

The term "neutral lipids" refers to any of a number of lipid species that exist either in an uncharged or neutral zwitterionic form at a selected pH. At physiological pH, such lipids include, for example, diacyl phosphatidylcholine, diacyl phosphatidylethanolamine, ceramide, sphingomyelin, cephalin, cholesterol, cerebrosides, and diacylglycerols.

The term "anionic lipids" refers to any lipid that is negatively charged at physiological pH. These lipids include, but are not limited to, phosphatidyl glycerol, cardiolipin, diacyl phosphatidylserine, diacyl phosphatidic acid, N-lauroyl phosphatidylethanolamine, N-succinyl phosphatidylethanolamine, N-glutaryl phosphatidylethanolamine, lysyl phosphatidyl glycerol, palmit oyloleoyl phosphatidyl glycerol (POPG), and other anionic groups attached to neutral lipids.

The term "cationic lipids" refers to any of a number of lipid species that carry a net positive charge at a selected pH such as physiological pH. These lipids include, but are not limited to, N, N-dioleyl-N, N-dimethylammonium chloride (DODAC); N- (2,3-dioleyloxy) propyl) -N, N,N-trimethylammonium chloride (DOTMA); N,N-distearyl-N, N-dimethylammonium bromide(DDAB); N-(2,3-dioleoyloxy)propyl)-N,N,N-trimethylammonium chloride (DOTAP); 3- (N- (N ', N' -dimethylaminoethane) carbamoyl) cholesterol (DC-Chol) and N-(1,2dimyristoyloxyprop-3-yl)-N,N-dimethyl-N-hydroxyethyl ammonium bromide (DMRIE). The following lipids are cationic and have a positive charge at a pH below physiological pH: DODAP, DODMA, DMDMA, and the like.

The term "hydrophobic lipids" refers to compounds having apolar groups that include, but are not limited to, long chain saturated and unsaturated aliphatic hydrocarbon groups and such groups are optionally substituted by one or more aromatic, alicyclic or heterocyclic group(s). Suitable examples include, but are not limited to, diacylglycerol, dialkylglycerol, N-N-dialkylamino, 1,2-diacyloxy-3-aminopropane and 1, 2-dialkyl-3-aminopropane.

The term "non-cationic lipids or non-ionizable lipids" refers to lipids that are neither cationic lipids nor ionizable lipids, and may be, for example, anionic lipids or neutral lipids.

The "non-cationic lipid or non-ionizable lipid other than the metal-chelated phospholipid complex and the particle aggregation-inhibiting conjugated lipid" in (iii) among the components of the metal-chelated phospholipid complex nanoparticles means that the non-cationic lipid or non-ionizable lipid in (iii) is the lipids remaining in the metal-chelated phospholipid complex nanoparticles when the metal-chelated phospholipid complex and the particle aggregation-inhibiting conjugated lipid are both excluded.

The term "fusogenic" refers to the capability of a liposome, a drug-lipid particle, or other drug delivery system to fuse with membranes of a cell. The membranes can be either a plasma membrane or membranes surrounding organelles such as endosome, nucleus, etc.

In the metal-chelated phospholipid complex nanoparticles, non-cationic lipid or non-ionizable lipid other than particle aggregation-inhibiting conjugated lipid is mainly present as vesicle-forming lipid. The term "vesicle-forming lipid" is intended to include amphiphilic lipid having a hydrophobic portion and a polar head group and which itself can spontaneously form a bilayer vesicle in water, such as most of phospholipids.

In metal-chelated phospholipid complex nanoparticles, the particle aggregation-inhibiting conjugated lipid is present primarily as vesicle- adopting lipid. The term " vesicle-adopting lipid" is intended to include amphipathic lipid that that is stably incorporated into the lipid bilayer, and other amphipathic lipids, with its hydrophobic portion in contact with the interior, hydrophobic region of the bilayer membrane, and its polar head group moiety oriented toward to the exterior, polar surface of the membrane. The vesicle-adopting lipids include lipids that on their own tend to adopt a non-lamellar phase, yet which are capable of assuming a bilayer structure in the presence of a bilayer stabilizing component. The particle aggregation-inhibiting conjugated lipid include, but is not limited to, polyamide oligomers (e.g., ATTA-lipid derivatives), peptides, proteins, detergents, lipid derivatives, PEG-lipid derivatives such as dialkoxypropyl-coupled PEG, diacylglycerol-coupled PEG, phosphatidylethanolamine-coupled PEG, and ceramide-coupled PEG (see U.S. Patent No. 5,885,613 which is incorporated herein by reference).

The term "amphiphilic lipid" refers to any suitable material in which the hydrophobic portion of the lipid material orients into a hydrophobic phase, while the hydrophilic portion orients toward the hydrophilic phase. Amphiphilic lipids are generally the major component of lipid vesicles. Hydrophilic characteristics derive from the presence of polar or charged group(s) such as carbohydrate, phosphate, carboxyl, sulfato, amino, mercapto, nitro, hydroxyl and other like groups. Hydrophobicity may be conferred by the inclusion of apolar groups, which include, but not limited to, long chain saturated

and unsaturated aliphatic hydrocarbon groups and such groups substituted with one or more aromatic, alicyclic, or heterocyclic group(s). Examples of amphiphilic compounds include, but are not limited to, phospholipids, aminolipids, and sphingolipids. Representative examples of phospholipids include, but are not limited to, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine, dipalmitoyl phosphatidylcholine, dioleoyl phosphatidylcholine, distearoyl phosphatidylcholine, or dilinoleoyl phosphatidylcholine. Other compounds that do not contain phosphorus, such as sphingomyelin, the glycosphingolipid families, diacylglycerols, and beta-acyloxylic acid can be referred to amphiphilic lipids. In addition, the amphiphilic lipid described above may be mixed with other lipids including triglyceride and sterols.

The term "diacylglycerol" refers to a compound having 2-fatty acyl chains, wherein both $R^1$ and $R^2$ may independently has between 2 and 30 carbons bonded to the 1- and 2-position of glycerol by ester linkages. The acyl group may be saturated or has varying degrees of unsaturation. The diacylglycerol has the following Formula 54:

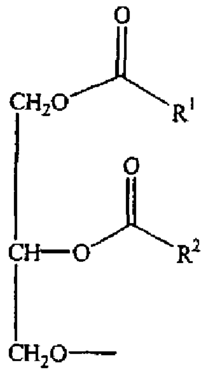

(Formula 54).

The term "diacylglycerol-coupled polyethylene glycol" i.e., a diacylglycerol-polyethylene glycol conjugate (DAG-PEG conjugate or PEG-DAG conjugate), can be the aggregation-inhibiting conjugated lipid in the present disclosure. In a preferred embodiment, the DAG-PEG conjugate may be dilaurylglycerol (C12)-PEG conjugate, ditetradecylglycerol (C14)-PEG conjugate (DMG), dipalmitoyl glycerol (C16)-PEG conjugate, or distearylglycerol (C18)-PEG conjugate (DSG). Those of skill in the art will readily appreciate that other diacylglycerols can be used for the DAG-PEG conjugate of the present disclosure. Suitable DAG-PEG conjugate for the present disclosure and method of preparation and use thereof them are disclosed in U.S. application No. 10/136,707, published as U.S.P.A 2003/0077829, and PCT patent application No. CA 02/00669, the entire content of which is incorporated herein by reference.

The term "dialkoxypropyl" refers to a compound having 2-alkyl chains, wherein each $R^1$ and $R^2$ may independently has between 2 to 30 carbons. The alkyl group may be saturated or has varying degrees of unsaturation. The dialkoxypropyl group has the following Formula 55:

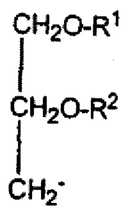

(Formula 55).

The term "dialkoxypropyl-coupled PEG," i.e., a dialkoxy-propyl conjugate (PEG-DAA conjugate), can be the aggregation-inhibiting conjugated lipid in the present disclosure. In a preferred embodiment, the PEG-DAA conjugate has the following Formula 56:

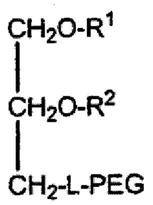

(Formula 56).

In Formula 56, $R^1$ and $R^2$ may independently be selected and may be a long chain alkyl group having about 10 to 22 carbon atoms. The long chain alkyl group may be saturated or unsaturated. Suitable alkyl groups include, but are not limited to, lauryl (C12), tetradecyl (C14), hexadecyl (C16), octadecyl (C18), and icosyl (C20). In a preferred embodiment, $R^1$ and $R^2$ may be the same, i.e., $R^1$ and $R^2$ may both be tetradecyl (i.e., ditetradecyl), $R^1$ and $R^2$ may both be octadecyl (i.e., dioctadecyl), and the like. In Formula 56, PEG may be a polyethylene glycol having an average molecular weight of about 550 to 10000 Daltons and may optionally be substituted at the terminal hydroxyl position with an alkyl, alkoxy, acyl, or aryl group. In a preferred embodiment, the PEG may have an average molecular weight of about 1000 to 5000 Daltons, more preferably, an average molecular weight of about 1,000 to 3,000 Daltons and even more preferably, an average molecular weight of about 2000 Daltons. PEG may be optionally substituted with alkyl, alkoxy, acyl, or aryl groups. In Formula 56, L is a linker moiety. Any linker moiety suitable for coupling PEG to a dialkoxypropyl backbone may be used. Suitable linker moieties include, but are not limited to, amido (-C (O) NH-), amino (-NR-), carbonyl (-C(O)-), carbonate (O-C(O)O-), carbamate (-NHC (O) O-), urea (-NHC(O)NH-), succinyl ($-(O)CCH_2CH_2C(O)-$), ether, disulfide, and combinations thereof. Other suitable linker moieties are well known in the art.

Phosphatidylethanolamines, which have various acyl chain groups with different chain lengths and degrees of saturation, can be conjugated to polyethylene glycol to form a bilayer stabilizing component as a particle aggregation-inhibiting conjugated lipid in the present disclosure. These phosphatidylethanolamines are commercially available or can be isolated or synthesized using conventional techniques known to those of skill in the art. Preferably, phosphatidylethanolamines comprise saturated or unsaturated fatty acids and have carbon chain lengths in the range of C10-C20. Phosphatidylethanolamines with mono- or di-unsaturated fatty acids and mixtures of saturated and unsaturated fatty acids can also be used. Suitable phosphatidylethanolamines include, but are not limited to, dimyristoyl phosphatidylethanolamine (DMPE), dipalmitoyl phosphatidylethanolamine (DPPE), dioleoyl phosphatidylethanolamine (DOPE), and distearoyl phosphatidylethanolamine (DSPE).

Ceramides, similar to phosphatidylethanolamines, having various acyl chain groups with different chain lengths and degrees of saturation, can be conjugated to polyethylene glycol to form a bilayer stabilizing component as a particle aggregation-inhibiting conjugated lipid in the present disclosure. It should be appreciated to those of skill in the art that ceramides, in contrast to phosphatidylethanolamine, have only one acyl group, which can be easily varied based on its chain length and degree of saturation. Ceramides suitable for use of the present disclosure are commercially available. In addition, ceramides may be isolated, for example, from eggs and brain using well-known seperation techniques, or synthesized using the methods and techniques disclosed in U.S. Patent No. 5,820,873, which is incorporated herein by reference. Using the synthetic routes set forth in the aforementioned applications, ceramides may be prepared with saturated or unsaturated fatty acids having carbon chain lengths in the range of C2-C31.

The term "ATTA" or "polyamide" refers to, but is not limited to, compounds disclosed in U.S. Patent Nos. 6,320,017 and 6,586,559, which are incorporated herein by reference. These compounds include a compound having the following Formula 57:

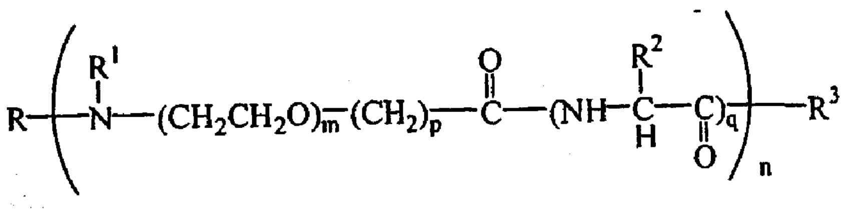

(Formula 57);

Wherein: R may be selected from the group consisting of hydrogen, alkyl, and acyl; $R^1$ may be selected from the group consisting of hydrogen and alkyl; or optionally, R and $R^1$ and the nitrogen atom to which they are bound may form an azido moiety; $R^2$ may be selected from the group of hydrogen, optionally substituted alkyl, optionally substituted aryl, and a side chain of an amino acid; $R^3$ may be selected from the group consisting of hydrogen, halogen, hydroxy, alkoxy, mercapto, hydrazino, amino, and $NR^4R^5$, wherein $R^4$ and $R^5$ may independently be hydrogen or alkyl; N is 4 to 80. M is 2 to 6; p is 1 to 4; q is 0 or 1. It will be appreciated to those of skill in the art that other polyamides may be used in the compounds of the present disclosure.

The term " homologue" refers to an analog that has the same or similar function, or a derivative of the same parent that have the same or similar function.

As used herein, the terms "mRNA" or "messenger polyribonucleotide" or "messenger RNA" are used interchangeably and refer to a single-stranded polyribonucleotide transcribed from one strand of DNA as a template, carrying genetic information guiding protein synthesis.

As used herein, the terms "sgRNA" or "small guide RNA" or "gRNA" are used interchangeably, and refer to a small noncoding RNA that is capable of guiding the insertion or deletion of uridine residues into the kinetoplast (kinetoplastid) during RNA editing and can be paired with pre-mRNA. gRNA can edit RNA molecules, have a length of approximately 60-80 nucleotides and are transcribed from a single gene.

As used herein, the terms "circRNA" or "circular RNA" or "circular polyribonucleotide" are used interchangeably and refer to polyribonucleotide molecule having a structure without free ends (i.e., without free 3' and/or 5' ends) formed in a circular or loop structure, for example, by a covalent or non-covalent bond.

As used herein, the terms "microRNA" or "miRNA" are used interchangeably and refer to a non-coding single-stranded polyribonucleotide with a length of approximately 22 nucleotides and free 3' and 5' ends. It can regulate the biological function of cells by binding to the 3 '-untranslated region (3'-UTR) of mRNA of the target gene to inhibit the protein translation of the target gene.

As used herein, the term "ASO" or "antisense oligonucleotide" are used interchangeably and refer to a synthetic, single-stranded poly (deoxy) ribonucleotide that is complementary to a segment of the target gene or mRNA and binds to a target gene/mRNA by the base-complementary principle to block the expression of the gene. It includes antisense DNA and antisense RNA.

As used herein, the terms "siRNA" or "small interference" or "short interference RNA" or "small interfering RNA" or "short interfering RNA" or "silencing RNA" are used interchangeably and refer to a class of double stranded RNA molecules that are 20 to 25 nucleotides in length and are capable of inducing degradation of the mRNA of a target gene.

As used herein, the term "ecDNA" or "extrachromosomal circular DNA" are used interchangeably and refer to DNA that is detached from a chromosome and exits in a circular structure.

The term "nucleic acid derivatives" refers to modifications or substitutions to nucleic acid sequence, including but not limited to chemical modifications to residues, substitutions of nucleotides or deoxynucleotides, modifications to sequence that improve half-life or stability, label modifications. For example, chemical modifications include, but are not limited to, phosphorylation, methylation, amination, sulfhydrylation, substitution of oxygen with sulfur, substitution of oxygen with selenium, or isotopolization of any one or more bases. Substitutions of nucleotides or deoxynucleotides include, but are not limited to, nucleic acid analogs that replace the sugar-phosphate backbone is replaced with a polypeptide or other backbone (replacing DNA or RNA with PNA). Modifications to the sequence that improve half-life or stability include, but are not limited to, attachment to PEG, fluorine modifications. Label modifications include, but are not limited to, attachment of fluorophores, amino groups, biotins, digoxigenin, small peptides, and the like.

The term "artificial nucleic acid" refers to an artificially modified nucleic acid molecule, which modification includes, but is not limited to, base modification, ribose modification, PNA, and the like.

The term "nucleic acid" refers to a polymer comprising at least two deoxynucleotides or nucleotides in single or double stranded form. Unless specifically limited, the term encompasses nucleic acids comprising known analogs of natural nucleotides that have similar binding properties as the reference nucleic acid and are metabolized in a manner similar to natural nucleotides. Unless otherwise indicated, a particular nucleic acid sequence also implicitly encompasses conservatively modified variants thereof (e.g., degenerate codon substitutions), alleles, orthologs, SNPs and complementary sequences and as well as the sequence explicitly indicated. Specifically, degenerate codon substitutions may be obtained by generating sequences in which the third position of one or more selected (or all) codons is replaced by mixed bases and/or deoxyinosine residues (Batzer et al., Nucleic Acid Res.19:5081(1991); Ohtsuka et al., J. Biol.Chem.260:2605-2608(1985); and Cassol et al. (1992); Rossolini et al., Mol.Cell.Probes 8:91-98(1994)). "Nucleotides" contain a sugar deoxyribose (DNA) or ribose (RNA), a base, and a phosphate group. Nucleotides are connected by phosphate groups. "Bases" include purines and pyrimidines, which further include the following natural compounds: adenine, thymine, guanine, cytosine, uracil, inosine, and natural analogs, and synthetic derivatives of purines and pyrimidines, which include, but are not limited to, modifications to replace new reactive groups such as, but not limited to, amines, alcohols, thiols, carboxylates, and haloalkanes. The DNA may be in the form of antisense, plasmid DNA, parts of a plasmid DNA, pre-compressed DNA, product of a Polymerase Chain Reaction (PCR), vectors (P1, PAC, BAC, YAC, artificial chromosome), expression cassettes, chimeric sequences, chromosomal DNA, or derivatives of these groups. The term nucleic acid is used interchangeably with gene, cDNA, mRNA encoded by a gene, and an interfering RNA molecule.

The term "gene" refers to a nucleic acid (e.g., DNA or RNA) sequence that includes partial length or full length coding sequences necessary for the production of a polypeptide or a polypeptide precursor (e.g., polypeptides or polypeptide precursors from hepatitis virus A, B, C, D, E, or G virus; or herpes simplex virus).

As used herein, the term "gene product" refers to a product of a gene such as a transcript of DNA, including, e.g.,

mRNA.

The phrase "silencing the expression of a target gene" refers to the capability of an siRNA of the present disclosure to silence the expression of a target gene when it is activated. To determine the extent of gene silencing, a sample or assay of cells in a target organism or culture expressing a particular construct is compared to a control sample that does not express the construct. The control samples (lacking expression of the construct) were set as relative values of 100%. Inhibition of expression of the target gene is successfully obtained when the test value relative to the control is about 90%, preferably 50%, more preferably 25-0%. Suitable assays include, for example, detection of protein or mRNA levels using techniques known to those of skill in the art such as spotting, northern blotting, in situ hybridization, ELISA, immunoprecipitation, enzymatic action, and phenotypic assays known to those of skill in the art.

A "therapeutically effective amount" or "effective amount" of an siRNA refers to an amount sufficient to produce a desired effect, e.g., a reduction in expression of the target sequence as compared to the normal expression level detected in the absence of the siRNA.

As used herein, the term "aqueous solution" refers to a composition comprising, in whole or in part, water.

As used herein, the term "organic lipid solution" refers to a composition comprising, in whole or in part, an organic solvent with a lipid.

As used herein, "systemic delivery" refers to delivery of a compound resulting in a broad biodistribution in an organism. Some techniques of administration may result in systemic delivery of some compounds, but not other compounds. Systemic delivery refers to the contact of an effective, preferably therapeutic amount of the compound with the bulk of the body. In order to achieve broad biodistribution, blood survival is often required so that the compound is not rapidly degraded or cleared (such as by initial passage through an organ (liver, lung, and the like)) or by rapid, non-specific cellular binding) before reaching the site of disease distal to the site of administration. Systemic delivery of a drug-lipid particle can be carried out in any manner known in the art, including, for example, intravenous delivery, subcutaneous delivery, and intraperitoneal delivery. In a preferred embodiment, systemic delivery of a drug-lipid particle may be achieved by intravenous delivery.

As used herein, "local delivery" refers to the delivery of a compound directly to a target site in an organism. For example, the compound may be delivered locally by direct injection to a disease site such as a tumor or other target site such as a site of inflammation or a target organ such as the liver, heart, pancreas, kidney, and the like.

**Metal-chelated phospholipid complex**

In the present disclosure, a metal-chelated phospholipid complex consists of a phospholipid molecular moiety, a linker molecular moiety, and a metal ion moiety.

Regarding the phospholipid molecular moiety, it should be noted that the cis and the trans isomers of the phospholipid molecular moiety have no influence on the technical effects to be achieved by the present disclosure

Regarding the linker molecular moiety, the phospholipid molecular moiety of the metal-chelated phospholipid complex is derived primarily from natural plant extracts, such as curcumin, and has a wide range of biological effects, including antibacterial, antiviral, antifungal, antioxidant and anti-inflammatory activities. In addition, it is an effective immunomodulator, and can regulate the activity of various immunocytes such as T cells, B cells, macrophages, neutrophils, natural killer cells and dendritic cells, promote the balance of immunity, and enhance the immunity of the organism. Based on potential immune enhancement, anti-inflammation, anti-oxidation and anti-sars-cov-2 functions of curcumin molecules, the curcumin molecule is expected to become a potential auxiliary treatment means for resisting COVID-19. In addition, curcumin molecules have high safety, are listed in food additives and medical auxiliary materials, which is beneficial to clinical drug registration of drug-lipid integration for shortening the time length of clinical drug registration.

Regarding the metal ion moiety, the coordination bond between the linker molecular moiety and the metal ion moiety in the metal-chelated phospholipid complex is broken at a low pH (pH = 5.0) of lysosomes, and the metal ion is detached from the metal-chelated phospholipid complex.

The proportion of each component in the metal-phospholipid complex can be adjusted according to the structure of the specific metal-phospholipid complex components. The basis that the proportion of each component can be adjusted in that the phospholipid molecules and linker molecules are connected by hydrogen bonds, as long as phospholipid molecules contain multiple phosphate groups, when the phospholipid molecules and linker molecules are synthesized into the phospholipid complex, the proportions of phospholipid molecules and linker molecules can be adjusted according to the number of phosphate groups contained in the phospholipid molecule. That is, when the phospholipid molecule contains two phosphate groups, the ratio of phospholipid molecule to linker molecule can be adjusted to 1:2. When the phospholipid molecule contains three phosphate groups, the ratio of phospholipid molecule to linker molecule can be adjusted to 1:3. Because the hydroxyl group of the linker molecule is connected to the metal ion by a coordination bond, as long as the linker molecule contains multiple binding sites, the ratio of the linker molecule to the metal ion can be adjusted according to the number of binding sites contained in the linker molecule.

The phospholipid molecule with phosphate groups is first connected to the linker molecule to obtain a phospholipid complex, and then the phospholipid complex is connected to the metal ion to obtain the metal-phospholipid complex. Specifically, the phospholipid molecule and the linker molecule are dissolved in the appropriate amount of ethanol according to a predefined molar ratio, and the reaction is carried out at about 65°C. The phospholipid complex can be precipitated by adding n-hexane. The phospholipid complex is then dissolved with metal ions (such as $FeCl_3$, etc.) in an appropriate amount of ethanol according to a predefined molar ratio, the reaction is carried out at about 60°C, and the metal-phospholipid complex can be obtained.

**Metal-chelated phospholipid complex nanoparticles (MPP)**

The principle of nucleic acid loading by the metal-chelated phospholipid complex nanoparticle assembled from the metal-chelated phospholipid complex is in that the phospholipid molecule is connected with the linker molecules by hydrogen bonds, and the linker molecules are connected with metal ion(s) through coordination bond(s) to form a metal-chelated phospholipid complex, and the metal ion(s) of the metal-chelated phospholipid complex is connected with negative charged drug(s) through coordination bond(s), so that the negative charged drug(s) is loaded into the nanoparticles (MPP) while the metal-chelated phospholipid complex and other components (the particle aggregation-inhibiting conjugated lipid and the non-cationic lipid or the non-ionizable lipid other than the metal-chelated phospholipid complex and the particle aggregation-inhibiting conjugated lipid) are self-assembled into the MPP. As used herein, the term "non-cationic lipid or non-ionizable lipid other than the metal-chelated phospholipid complex and the particle aggregation-inhibiting conjugated lipid" refers to component (iii) of the metal-chelated phospholipid complex nanoparticle.

In some embodiments, the particle aggregation-inhibiting conjugated lipid refers to conjugated lipid that inhibits aggregation of drug-lipid particles, the primary function of which is to prevent aggregation of drug-lipid particles, such as dialkoxypropyl-coupled PEG, diacylglycerol-coupled PEG, phosphatidylethanolamine-coupled PEG, and ceramide-coupled PEG, preferably PEG-lipid conjugates. Wherein the cis and the trans isomers of the lipids have no influence on the technical effects to be achieved by the present disclosure.

In some embodiments, the molar proportion of the metal-chelated phospholipid complex in starting material may be 5% to less than 10%, 10% to 40%, or more than 40% to 50%, for example, may be 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or 50%.

In some preferred embodiments, the molar proportion of the metal-chelated phospholipid complex in starting material may be 7%~40%, for example, may be 10% to 40%, 7% to 30%, 15% to 25%, 20% to 30%, further preferably be 15%, 25%, 7% or 30%.

In some embodiments, the molar proportion of the particle aggregation-inhibiting conjugated lipid in starting material may be 2% to 10%, for example, may be 2%, 3%, 4%, 5%, 6%, 7%, 8%, 9%, or 10%. For example, the molar proportion of the particle aggregation-inhibiting conjugated lipid in starting material may be 3% to 10%, 4% to 10%, 5% to 10%, and further preferably be 3%, 4% or 10%.

In some embodiments, the non-cationic lipid or the non-ionizable lipid may be cholesterol, and the molar proportion of cholesterol in starting material may be 15% to less 35%, 35% to 75%, or more than 75% to 80%, for example, may be 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% or 80%. For example, the molar proportion of cholesterol in starting material in starting material may be 35% to 75%, 15% to 56%, 40% to 46% or 35% to 55%, and further preferably be 15%, 40%, 46% or 56%.

In some embodiments, the metal-chelated phospholipid complex nanoparticle may comprise, in addition to cholesterol, other non-cationic lipids or non-ionizable lipids, and the molar proportion of the other non-cationic lipids or non-ionizable lipids in starting material may be 0% to 40% or more than 40% to 51%, for example, may be 0%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50% or 51%. For example, the molar proportion of the other non-cationic lipids or non-ionizable lipids in starting material may be 5% to 30%, 25% to 35% 34% to 45% or 20% to 25%, and further preferably be 25%, 35%, 34% or 45%.

**Drug-lipid particles**

Drug-lipid particles described herein typically include a drug (which is a negatively charged molecule that may be selected from the group consisting of nucleic acids, proteins, polypeptides, small molecules, nucleic acid analogs, protein analogs, polypeptide analogs, and combinations thereof, the nucleic acids being selected from the group consisting of mRNA, siRNA, cyclic RNA, microRNA, DNA, ecDNA, artificial nucleic acids, and combinations thereof), the metal-chelated phospholipid complex, the non-cationic lipid or the non-ionizable lipid, and bilayer stabilizing component, such as

the particle aggregation-inhibiting conjugated lipid. Furthermore, the nucleic acid encapsulated in the drug-lipid particles of the present disclosure is resistant to degradation by nucleases in aqueous solution.

In some embodiments, the drug is sufficiently encapsulated inside the metal-chelated phospholipid complex nanoparticle to avoid degradation of the drug, enabling the drug to be delivered into the cell.

In some embodiments, the drug-lipid particle provided by the present disclosure may have a small diameter suitable for systemic delivery with a particle size of 30 to 400 nm; the surface potential of the drug-lipid particle may be -10 to 10 mV; the stability of the drug-lipid particle may be at least 3 days, preferably more than 7 days; the cellular delivery efficiency of the drug-lipid particle may be at least 40%, for example, at least 50%, at least 55%, at least 60%, at least 65%, at least 70%, at least 75%, at least 80%, at least 85%, at least 90%, or at least 95%.

In some embodiments, the drug of the drug-lipid particle is preferably a nucleic acid, the nucleic acid component may typically comprise mRNA, interfering RNA (i.e., siRNA) which may be provided in several forms including, for example, one or more isolated small-interfering RNA (siRNA) duplex, longer double-stranded RNA (dsRNA), or siRNA or dsRNA translated from a transcription cassette in a DNA plasmid.

An RNA population can be used to provide long precursor RNAs, or long precursor RNAs that have substantial or complete identity to a selected target sequence that can be used to prepare siRNA. The RNAs can be isolated from cells or tissues, synthesized, and/or cloned according to methods well known to those of skill in the art. The RNA may be a mixed population (obtained from cells or tissues, transcribed from cDNA and the like), or may represent a single target sequence. RNA may be naturally occurring, e.g., isolated from a tissue or cell sample, synthesized in vitro, e.g., using T7 or SP6 polymerase and PCR products or cloned cDNA; or chemically synthesized.

In order to form long dsRNAs, for synthetic RNAs, the complements can also be transcribed in vitro and hybridized to form dsRNAs. If a natural RNA population is used, the RNA complements are also provided (e.g., to form dsRNA for digestion by E. *coli* RNAse III or cutting enzymes), e.g., by transcribing cDNAs corresponding to the RNA population, or by use of an RNA polymerase . The precursor RNAs are then hybridized to form double stranded RNAs for digestion. The dsRNAs can be encapsulated directly in SNALPs or can be digested in vitro prior to encapsulation.

Alternatively, one or more DNA plasmids encoding one or more siRNA templates can be encapsulated in the nucleic acid-lipid particle. siRNA can be transcribed as sequences that automatically fold into duplexes with hairpin loops from DNA templates in plasmids having an RNA polymerase III transcription unit, for example, based on the naturally ccurring transcription units for small nuclear RNA U6 or human RNase P RNA H1 (see Brummelkamp, et al., Science 296: 550(2002); Donzé,et al, Nucleic AcidsRes.30: e46(2002); Paddison, et al., Genes Dev.16: 948(2002); Yu, et al., Proc.Natl.Acad.Sci.99: 6047(2002); Lee, et al., Nat.Biotech.20: 500(2002); Miyagishi, et al., Nat.Biotech.20: 497(2002); Paul, et al., Nat.Biotech.20: 505(2002); and Sui, et al., Proc.Natl.Acad.Sci.99: 5515(2002)). Typically, the transcription unit or cassette will comprise an RNA transcription promoter sequence, such as the H1-RNA or U6 promoter, operably linked to a template for transcription of a desired siRNA sequence, and a termination sequence comprising 2-3 uridine residues and a poly-thymidine (T5) sequence (polyadenylation signal) (Brummelkamp, Science, supra). The selected promoter may provide for constitutive or inducible transcription. Compositions and methods for DNA-directed transcription of RNA interference molecules are described in detail in U.S. Patent No.6,573,099, which is incorporated herein by reference. Preferably, the synthetic or transcribed siRNA has 3' overhangs of about 1-4 nucleotides, preferably of about 2-3 nucleotides and 5' phosphate termini (Elbashir, et al, Genes Dev.15: 188 (2001); Nykänen, et al, Cell 107: 309 (2001)). The transcriptional unit is incorporated into a plasmid or DNA vector from which the interfering RNA is transcribed. Plasmids suitable for in vivo delivery of genetic material for therapeutic purposes are described in detail in U.S. Patent No.5,962,428 and 5,910,488, both of which are incorporated herein by reference. The selected plasmid can provide for transient or stable delivery of the target cell. It will be clear to those of skill in the art that plasmids originally designed to express desired gene sequences may be modified to contain a transcription unit cassette for transcribing siRNA.

Methods for isolating RNA, synthesizing RNA, hybridizing nucleic acids, preparing and screening cDNA libraries, and performing PCR are well known in the art (see, e.g., Gubler & Hoffman, Gene 25: 263-269 (1983); Sambrook et al, supra; Ausubel et al, supra), as are PCR methods (see, e.g., U.S. Pat. Nos. 4,683,195 and 4,683,202; PCR Protocols: A Guide to methods and Applications (Innis et al, eds., 1990)). Expression libraries are also well known to those of skill in the art. Additional basic books disclosing the general methods used in the present disclosure include Sambrook et al., Molecular Cloning, A Laboratory Manual (2nd ed.1989); Kriegler, Gene Transfer and Expression: A Laboratory Manual (1990); and Current Protocols in Molecular Biology (Ausubel et al., eds., 1994)).

**Use of the metal-chelated phospholipid complex, the metal-chelated phospholipid complex nanoparticle, the drug-lipid particle**

In some embodiments, when the drug is a nucleic acid, the metal-chelated phospholipid complex and the metal-chelated phospholipid complex nanoparticle can be used to promote the drug escape from lysosomes and promote the expression of nucleic acid. The metal-chelated phospholipid complex and the metal-chelated phospholipid complex nanoparticle can also be used for delivering medicaments and introducing the medicaments into cells, and thus realizing

the prevention and treatment of applicable diseases or disorders by the medicaments.

In some embodiments, the present disclosure provides use of the drug-lipid particle, for example, in a composition that can effectively deliver a drug or introduce a drug into a cell. The composition is, for example, a medicament that can silence expression of the target sequence in a mammalian subject, deliver a drug in the mammal body (e.g., a drug to treat a tumor, an imaging agent and the like), deliver a drug into a mammalian cell from the body, or treat a disease or disorder in the mammal. In the medicament, the drug-lipid particle is the main active ingredient. the medicament may be prepared into different formulations according to actual needs through different pharmaceutically acceptable auxiliary materials or preparation processes, such as solid formulation (powder, granule, pill, tablet and gel), semisolid formulation (external ointment and paste), liquid formulation (decoction, mixture, syrup, medicated wine and injection), gas formulation (aerosol and smoke) and the like. For example, the medicament may be prepared into a formulation for gastrointestinal administration, a formulation for rectal administration, a formulation for parenteral administration, and the like.

In some embodiments, the present disclosure provides a product made from the above metal-chelated phospholipid complex, the metal-chelated phospholipid complex nanoparticle, and the drug-lipid particle. The product has the above-described functions and uses of the metal-phospholipid complex, the metal-phospholipid complex nanoparticle, and the drug-lipid particle, in specific types such as, but not limited to, kit, medicament, and the like, optionally with other excipients.

Generally, for a target gene for drug-lipid particle, it is desirable to deliver drug-lipid particle such that translation (i.e., expression) of the product of the target gene is down - regulated or silenced. Suitable classifications of gene products include, but are not limited to, genes associated with viral infection and survival, genes associated with metabolic diseases and disorders (e.g., diseases and disorders in which the liver is the target, and liver diseases and disorders), genes associated with tumorigenesis and cell transformation, angiogenic genes, immunomodulator genes such as those associated with inflammation and autoimmune responses, ligand receptor genes, and genes associated with neurodegenerative disorders.

Genes associated with viral infection and survival may include those that bind, enter, and replicate in cells through viral expression, in particular, viral sequences associated with chronic viral diseases. For example, viral sequences include sequences of hepatitis viruses (Hamasaki, et al., FEBS Lett.543: 51(2003); Yokota, et al, EMBO Rep.4: 602(2003); Schlomai, et al., Hepatology 37: 764(2003); Wilson, et al., Proc.Natl.Acad.Sci.100: 2783(2003) ; Kapadia , et al. , Proc.Natl.Acad.Sci.100: 2014(2003) ; and FIELDSVIROLOGY(Knipe et al.eds.2001)); human immunodeficiency virus (HIV) (HIV)(Banerjea, etal., Mol Ther.8: 62(2003); Song, et al., J.Virol.77: 7174(2003); StephensonJAMA 289: 1494(2003); Qin, et al., Proc.Natl.Acad.Sci.100: 183(2003)), herpes virus (Jia, et al., J.Virol.77: 3301(2003)) and human papillomavirus (HPV)(Hall, et al., J.Virol.77: 6066(2003); Jiang, et al., Oncogene 21: 6041(2002)). Exemplary hepatitis virus nucleic acid sequences that can be silenced include, but are not limited to: nucleic acid sequences involved in transcription and translation (e.g., En1, En2, X, P), nucleic acid sequences encoding structural proteins (e.g., core proteins including C and C related proteins; capsid and envelope proteins including S, M, and/or L proteins, or fragments thereof) (see, e.g., FIELDS VIROLOGY, 2001, supra). Hepatitis C nucleic acid sequences that may be silenced include, but are not limited to: serine proteases (e.g., NS3/NS4), helicases (e.g., NS3), polymerases (e.g., NS5B), and envelope proteins (e.g., E1, E2, and p 7). Hepatitis A nucleic acid sequences are mentioned, for example, in Genbank Accession No. NC_001489; hepatitis B nucleic acid sequences are mentioned, for example, in Genbank Accession No. NC_ 003977; hepatitis C nucleic acid sequences are mentioned, for example, in Genbank Accession No. NC_004102; hepatitis D nucleic acid sequences are mentioned, for example, in Genbank Accession No. NC_001653; hepatitis E nucleic acid sequences are mentioned, for example, in Genbank Accession No. NC_001434; and the hepatitis G nucleic acid sequence is mentioned, for example, in Genbank Accession No. NC_001710. Silencing sequences encoding genes associated with viral infection and survival may be suitably used in conjunction with administration of conventional agents for treating viral diseases.

Genes associated with metabolic diseases and disorders (e.g., disorders in which the liver is targeted and liver diseases and disorders) may include, for example, genes expressed in dyslipidemia (e.g., liver X receptor (e.g., LXR α and LXR β Genbank Accession No. NM _007121)), Farnesoid X Receptor (FXR) (Genbank Accession No. NM _005123), Sterol Regulatory Element Binding Protein (SREBP), site-1 protease (S1P), 3-hydroxy-3-methylglutaryl coenzyme-a reductase (HMG coenzyme-a reductase), apolipoprotein (ApoB), and apolipoprotein (ApoE)) and diabetes (e.g., glucose-6-phosphate) (see, for example, Forman et al., Cell 81: 687(1995); Seol et al., Mol.Endocrinol.9: 72(1995), Zavacki et al., PNAS USA 94: 7909(1997); Sakai, et al., Cell85: 1037-1046(1996); Duncan, et al., J.Biol.Chem.272: 12778-12785(1997); Willy, et al., Genes Dev.9(9): 1033-45(1995); Lehmann, et al., J.Biol.Chem.272(6): 3137-3140(1997); Janowski, et al., Nature 383: 728-731(199; Peet, et al., Cell 93: 693-704(1998)). Those of skill in the art will appreciate that genes associated with metabolic diseases and disorders (e.g., diseases and disorders in which the liver is targeted, and liver diseases and disorders) include genes expressed in the liver itself, as well as genes expressed in other organs and tissues. Silencing sequences encoding genes associated with metabolic diseases and disorders may be suitably used in conjunction with the administration of conventional agents for treating the diseases or

disorders.

Examples of genes associated with tumorigenesis and cellular transformation may include translocation sequences such as MLL fusion gene, BCR-ABL (Wilda, et al, Oncogene, 21: 5716 (2002); Scherr, et al, Blood 101: 1566), TEL-AML1, EWS-FLI1, TLS-FUS, PAX3-FKHR, BCL-2, AML1-ETO, and AML1-MTG8(Heidenreich, et al, Blood 101: 3157 (2003)); overexpressed sequences such as multidrug resistance Genes (Nieth, et al., FEBS Lett.545: 144 (2003); Wu, et al, Cancer Res.63: 1515(2003)), cyclin (Li, et al, Cancer Res.63: 3593 (2003); Zou, et al, Genes Dev.16: 2923(2002)), β -catenin (Verma, et al., Clin Cancer Res.9: 1291(2003)), telomere terminal transferase Genes (Kosciolek, et al, Mol Cancer Ther.2: 209(2003)), c-MYC, N-MYC, BCL-2, ERBB1, and ERBB2 (Nagy, al. Exp. Cell Res. 285: 39 (2003)); and mutant sequences such as RAS (reviewed in Tuschl and Borkhardt, Mol. Interventions, 2: 158 (2002)). Silencing the sequence that encodes a DNA repair enzyme may used in conjunction with the administration of chemotherapeutic agents (Collis, et al, Cancer Res.63: 1550 (2003)). Genes encoding proteins associated with tumor migration, such as integrins, selectins and metalloproteinases, may also be target sequences of interest. Any complete or partial gene sequence that facilitates or promotes tumorigenesis or cell transformation, tumor growth or tumor migration may be included as a template sequence.

The angiogenic gene can promote the formation of new blood vessels. Vascular Endothelial Growth Factor (VEGF) is the direction of intense research (Reich, et al, Mol. Vis.9: 210 (2003)).

An immunomodulator gene is a gene that modulates one or more immune responses. Examples of immunomodulator genes may include cytokines such as growth factors (e.g., TGF- α, TGF- β, EGF, FGF, IGF, NGF, PDGF, CGF, GM-CSF, SCF and the like), interleukins (e.g., IL-2, IL-4, IL-12(Hill, et al, J.Immunol.171: 691(2003)), IL-15, IL-18, IL-20 and the like), interferons (e.g., IFN- α, IFN- β, IFN-γ and the like), and TNF. Fas and Fas ligand genes are also target immunomodulator sequences of interest (Song, et al, Nat. Med. 9: 347 (2003)). Genes encoding secondary signaling molecules in hematopoietic and lymphoid cells are also included in the present disclosure, for example, Tec family kinases such as Bruton's tyrosine kinase (Btk) (Heinonen, et al, FEBS lett.527: 274 (2002)).

Cell receptor ligands may include ligands that bind to cell surface receptors (e.g., insulin receptors, EPO receptors, G-protein coupled receptors, receptors with tyrosine kinase activity, cytokine receptors, growth factor receptors and the like) to modulate (e.g., inhibit, activate and the like) the physiological pathway(s) to which the receptors are involved (e.g., glucose level regulation, blood cell development, mitogenesis and the like). Examples of cell receptor ligands may include cytokines, growth factors, interleukins, interferons, erythropoietin (EPO), insulin, glucagon, G-protein coupled receptor ligands, and the like. Templates encoding trinucleotide repeat sequence expansion (e.g., CAG repeat sequence) are found to silence pathogenic sequences in neurodegenerative diseases caused by expansion of trinucleotide repeat sequence, such as spinal and bulbar muscular atrophy and Huntington's disease (Caplen, et al, Hum. Mol. Gene.11: 175 (2002)).

Regarding injectable delivery, in some cases, as described in U.S. Patent No. 5,543,158, U.S. Patent No. 5,641,515 and U.S. Patent No. 5,399,363, it may be desirable to deliver the drug-lipid particle disclosed herein parenterally, intravenously, intramuscularly, subcutaneously, intradermally, or intraperitoneally. The drug-lipid particle may be injected locally to a target site (e.g., a disease site such as inflammation or tumor formation or to a target organ or tissue) or systemically for broad distribution to an organism. A solution of the drug-lipid particle may be prepared in water, suitably mixed with a surfactant. Dispersion may also be prepared in glycerol, liquid polyethylene glycols and mixtures thereof, and in oils. Optionally, these formulations may contain a preservative to prevent the growth of microorganisms. Typically, when administered intravenously, the drug-lipid particle formulation may be formulated with a suitable pharmaceutical carrier. Typically, ordinary buffered saline (135-150mM NaCl) may be used as the pharmaceutical carrier, but other suitable carriers may suffice. Other suitable vectors are described, for example, in REMINGTON'S PHARMACEUTICAL SCIENCES, Mack Publishing Company, Philadelphia, PA, 17th ed. (1985). As used herein, the term "carrier" may include any and all solvents, dispersion media, medium, coatings, diluents, antibacterial and antifungal agents, isotonic and absorption delaying agents, buffers, carrier solutions, suspensions, colloids, and the like. The phrase "pharmaceutically acceptable" refers to molecular entities and compositions that do not produce allergic or similar adverse reaction when administered to a human. The formulation of aqueous compositions, which comprise a protein as active ingredient, is well understood in the art. Alternatively, these compositions are prepared as injections, liquid solutions or suspensions; solid forms suitable for solution in a liquid or suspension prior to injection may also be prepared. The formulation may also be emulsified.

The drug-lipid particle may be sterilized by conventional liposome sterilization techniques, such as filtration. The drug-lipid particle may contain pharmaceutically acceptable auxiliary substances which are suitable physiological conditions such as pH adjusting and buffering agents, toxicity adjusting agents, wetting agents and the like. These compositions may be sterilized using the techniques referred to above, or alternatively, they may be produced under sterile conditions. The resulting aqueous solutions may be packaged for use or filtered under sterile conditions and lyophilized, the lyophilized preparation being combined with a sterile aqueous solution prior to administration.

Prophylactic or therapeutic treatment: In some embodiments, the drug-lipid particle may be used for prophylactic or therapeutic treatment of a subject (e.g., a mammalian subject) suffering from a disease or disorder associated with

expression or overexpression of a target sequence. The drug-lipid particle may be administered to the subject in an amount sufficient to elicit a therapeutic response in the patient. An amount adequate to accomplish this is defined as a "therapeutically effective dose or amount" or an "effective dose or amount". In determining the effective amount of the drug-lipid particle to be administered in the treatment or prophylaxis of conditions owing to expression or overexpression of the target gene, the physician evaluates circulating plasma levels of the drug-lipid particle, drug-lipid particle toxicity, and progression of the disease associated with expression or overexpression of the target gene. Administration may be accomplished in single or divided doses.

For example, the drug-lipid particle may be administered to a subject infected or at risk of being infected by a pathogenic microorganism. The drug may preferably correspond to a sequence which plays an essential role in the lifecycle of the microorganism, and should also be unique to the microorganism (or at least absent in the natural genome of a patient undergoing therapy). The drug-lipid particle is introduced into the target cell, tissue or organ, either ex vivo or by intravenous injection at a therapeutically effective dose. Silencing of sequences encoding genes associated with pathogenic infections may be suitably used in conjunction with the administration of conventional agents for the treatment of pathogenic diseases. The treatment may be administered prophylactically to persons at risk of being infected with pathogenic microorganism or to persons already infected with the pathogenic microorganism.

In a preferred embodiment, the drug-lipid particle of the present disclosure may be conveniently used to treat cancer, viral infections, autoimmune diseases, diabetes, Alzheimer’s disease. Viral infections may include hepatitis A, hepatitis B, hepatitis C, SARS-Cov-2, HIV, HPV, influenza, smallpox, syphilis. For example, suitable sites for inhibition on the hepatitis B virus may include nucleic acid sequences encoding S, C, P and X proteins, PRE, EnI, and EnII (see, e.g., FIELDS VIROGY, 2001, supra). Those of skill in the art will appreciate that silencing of genes associated with hepatitis infection may be combined with conventional treatments for hepatitis such as, for example, immunoglobulins, interferons (e.g., pegylated and unpegylated interferon a) (see, for example, Medina et al, Antiviral Res.60 (2): 135-143 (2003); ribavirin (see, for example, Hugle and Cerny, Rev. Med. Virol.13 (6): 361-71 (2003)), adefovir and lamivudine (see, for example, Kock et al, Hepatoloy 38 (6): 1410-8 (2003)); prenylation inhibitors (see, for example, Bordier et al, J.Clin.invest.112 (3): 407-414 (2003)); famciclovir (see, for example, Yurdaydin et al, J Hepatol. 37 (2): 266-71 (2002) and saikosaponins c and d (see, Chiang et al., Planta Med.69(8): 705-9(2003).

In another embodiment, the drug-lipid particle of the present disclosure may be conveniently used to treat diseases and disorders characterized by expression or overexpression of a gene or group of genes. In some aspects, the drug-lipid particle of the present disclosure may be used to treat metabolic diseases and disorders (e.g., diseases and disorders in which the liver is a target and liver diseases and disorders) such as, for example, dyslipidemia and diabetes. Those of skill in the art will appreciate that silencing of genes associated with metabolic diseases and disorders may be combined with conventional treatments of these diseases. For example, silencing of genes involved in dyslipidemia may be combined with treatment with statins, bile acid sequestrants/resins and cholesterol absorption inhibitors such as ezetimibe, plant stanols/sterols, polyphenols, and nutraceuticals such as oat bran, linseed and soybean protein, phytostanol analogs, squalene synthetase inhibitors, bile acid transport inhibitors SREBP Cleavage Activator Protein (SCAP) activating ligands, niacin (nicotinic acid), acipimox, high dose fish oils, antioxidants and policosanol, microsomal triacylglycerol transfer protein (MTP) inhibitors, acylcoenzyme A: cholesterol acyltransferase (ACAT) inhibitors, gemcabene, lifibrol, pantothenic acid analogs, nicotinic acid-receptor agonists, anti-inflammatory agents (such as Lp-PLA (2) antagonists and AGI1067) functional oils, PPAR-$\alpha$, $\gamma$, $\delta$ agonists, and the dual PPAR-$\alpha$,/$\gamma$ and ’pan’ PPAR-$\alpha$/$\gamma$,/ $\delta$ agonists, cholesteryl ester transfer protein (CETP) inhibitors (such as torcetrapib), CETP vaccines, upregulators of ATP-binding cassette transporter (ABC) A1, lecithin cholesterol acyltransferase (LCAT) and scavenger receptor class B type 1(SRB1), and synthetic apolipoprotein (Apo) E-related peptides, extended-release niacin/lovastatin, atorvastatin/amlodipine, ezetimibe/simvastatin, atorvastatin/CETP inhibitors, statin/PPAR agonists, extended-release niacin/simvastatin and pravastatin/aspirin are under development, and anti-obesity agents (see, e.g., Bayes and Stein, Expert Opin. Pharmacother.4 (11): 1901-38 (2003)). Likewise, silencing of genes involved in diabetes can be combined with treatment with insulin as well as diet modifications and exercise.

Analogous methods are used for inhibiting expression of endogenous recipient cell genes associated with tumorigenesis and cell transformation, tumor growth and tumor migration; angiogenic genes; immunomodulator genes, such as those associated with inflammatory and autoimmune responses; ligand receptor gene; gene associated with neurodegenerative disorders; and additional genes associated with viral infection and survival. Target gene sequences of particular interest are described above.

Detection of Particles: The drug-lipid particles herein may be detected using any method known in the art. For example, a label may be coupled directly or indirectly to components of drug-lipid particle or other lipid-based carrier system using methods well known in the art. A wide variety of labels may be used, with the selection being made based on the sensitivity required, ease of conjugation to the drug-lipid particle component, stability requirements and available instrumentation and disposal provisions. Suitable labels may include, but are not limited to, spectral labels such as fluorescent dyes (e.g. fluorescein and derivatives such as fluorescein isothiocyanate (FITC) and Oregon Green™; rhodamine and derivatives, such as Texas Red, tetrahydrodiine isothiocynate (TRITC) and the like, digoxigenin, biotin,

phycoerythrin, AMCA, CyDyes™ and the like); radiolabels, such as $^3H$, $^{125}I$, $^{35}S$, $^{14}C$, $^{32}P$, $^{33}P$ and the like; enzymes such as horse radish peroxidase, alkaline phosphatase and the like; spectral colorimetric labels, such as colloidal gold or colored glass or plastic beads such as polystyrene, polypropylene, latex and the like. The label may be detected using any means known in the art.

Detection of Nucleic acids : Nucleic acids may be detected and quantified herein by any of a number of means well known to those of skill in the art. Detection of nucleic acids may be performed by methods well known in the art such as Southern analysis, northern analysis, gel electrophoresis, PCR, radiolabelling, scintillation counting and affinity chromatography. Additional analytic biochemical methods such as spectrophotometry, radiography, electrophoresis, capillary electrophoresis, high performance liquid chromatography (HPLC), thin layer chromatography (TLC), hyperdiffusion chromatography may also be employed.

The sensitivity of hybridization assays may be increased by using nucleic acid amplification system that multiplies the target nucleic acid being detected. In vitro amplification techniques suitable for amplifying sequences for use as molecular probes or for generating nucleic acid fragments for subsequent subcloning are known. Examples of techniques sufficient to direct persons of skill through such in vitro amplification methods, including the polymerase chain reaction (PCR), the ligase chain reaction (LCR), Qβ-replicase amplification, and other RNA polymerase mediated techniques (e.g., NASBA™) are found in Sambrook, et al., Molecular Cloning: A Laboratory Manual, Cold Spring Harbor Laboratory Press, 2000, and Ausubel et al. , SHORT PROTOCOLS IN MOLECULAR BIOLOGY, eds., Current Protocols, a joint venture between Greene Publishing Associates, Inc. and John Wiley & Sons, Inc., (2002), and Mullis et al.(1987), U.S. Patent No. 4,683,202; PCR Protocols A Guide to Methods and Applications (Innis et al. eds) Academic Press Inc. San Diego, CA (1990)(Innis); Arnheim & Levinson (October 1, 1990), C&EN 36; The Journal Of NIH Research, 3: 81(1991); (Kwoh et al., Proc. Natl. Acad. Sci. USA, 86: 1173(1989); Guatelli et al., Proc. Natl. Acad. Sci. USA, 87: 1874(1990); Lomell et al., J. Clin. Chem., 35: 1826(1989); Landegren et al., Science, 241: 1077(1988); Van Brunt, Biotechnology, 8: 291(1990); Wu and Wallace, Gene, 4: 560(1989); Barringer et al., Gene, 89: 117(1990), and Sooknanan and Malek, Biotechnology, 13: 563(1995). Improved methods of cloning in vitro amplified nucleic acids are described in Wallace et al., U.S. Patent No. 5,426,039. Other methods described in the art are the nucleic acid sequence based amplification (NASBA™, Cangene, Mississauga, Ontario) and Q Beta replicase systems.

Oligonucleotides for use as probes, e.g., in vitro amplification methods, for use as gene probes, or as inhibitor components are typically synthesized chemically according to the solid phase phosphoramidite triester method described by Beaucage and Caruthers, Tetrahedron letts, 22 (20): 1859 1862 (1981), e.g., by using an automated synthesizer, as described in Needham VanDevanter et al., Nucleic Acids Res., 12: 6159(1984). Purification of oligonucleotides, where necessary, is typically carried out by either native acrylamide gel electrophoresis or by anion exchange HPLC as described in Pearson and Regnier, J. Chrom., 255: 137 149 (1983). The sequence of synthetic oligonucleotides can be verified using chemical degradation method of Maxam and Gilbert (1980) in Grossman and Moldave (eds.) Academic Press, New York, Methods in Enzymology, 65: 499.

The following examples are provided to illustrate, but not to limit the disclosure. Those of skill in the art will readily recognize a variety of noncritical parameters which can be changed or modified to yield essentially the same results.

Drug-lipid particle to be claimed by the present disclosure refers to drug-lipid particle other than those containing cationic/ionizable lipids, i.e., drug-loaded metal-chelated phospholipid complex nanoparticles (drug@MPP).

## Embodiment 1. Preparation of drug-loaded metal-chelated phospholipid complex nanoparticles

### Example 1. Preparation of phospholipid complex

A phospholipid molecule having a phosphate group was connected with a linker molecule: distearoylphosphatidylcholine (DSPC, formula 46) and curcumin (formula 19) were added at a molar ratio of 1: 1 into a reaction bottle, a proper amount of ethanol was added for dissolving, and the reaction was carried out at 65 °C for 2 hours. The product was concentrated, and n-hexane was added. Phospholipid complex was precipitated, filtered, and vacuum dried. The structure of the phospholipid complex is shown below:

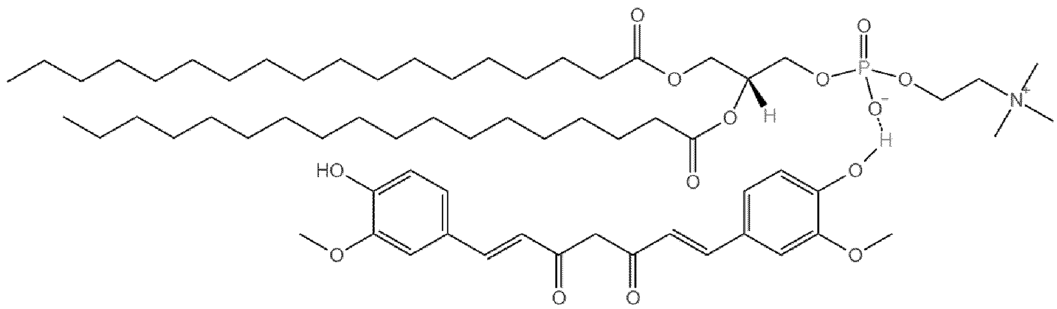

Result analysis: the yield of the target product obtained was 94% when curcumin was reacted with DSPC at 65°C for 2 hours.

**Example 2. Preparation of metal-chelated phospholipid complex**

Example 2.1 Preparation of metal-chelated phospholipid complex with Fe $^{3+}$ as metal ion

The phospholipid complex prepared in example 1 was connected to a metal ion moiety: the phospholipid complex and the $FeCl_3$ was added at a molar ratio of 1: 1 into to a reaction bottle, ethanol was added for dissolving, and the reaction was carried out at 60 °C for 2 hours. The reaction solution was rotary-evaporated, and the product was washed with ultrapure water and dried in vacuum to obtain the metal-chelated phospholipid complex. The structure of the metal-chelated phospholipid complex is shown below.

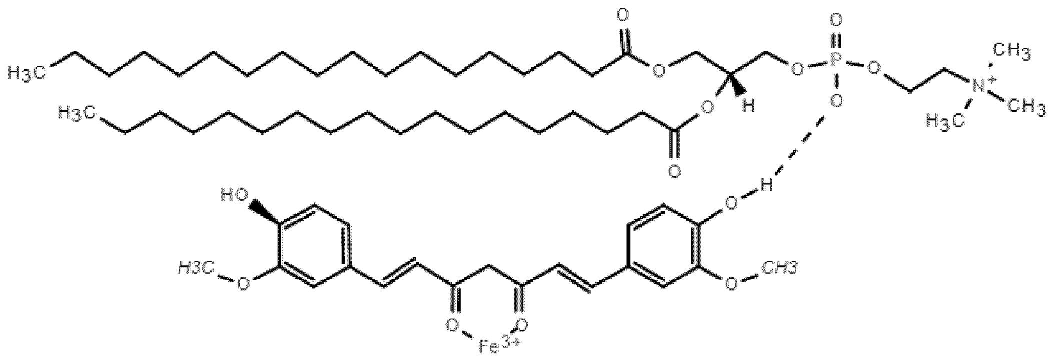

Result analysis: the yield of the target product obtained was 95% when the phospholipid complex was reacted with $FeCl_3$ at 60 °C for 2 hours, the feeding concentration of the phospholipid complex was 4.5mg/mL, and the feeding ratio of the phospholipid complex to the $FeCl_3$ was 1: 1.

Example 2.2. Preparation of metal-chelated phospholipid complex with $Al^{3+}$ as metal ion

The present Example differs from Example 2.1 in that $FeCl_3$ is replaced by $Al(NO_3)_3 \cdot 9H_2O$.The structure of the prepared metal-chelated phospholipid complex is shown below.

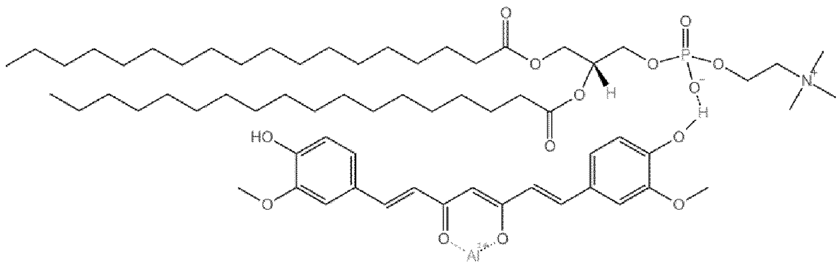

Result analysis: the yield of the target product obtained was 95% when the phospholipid complex was reacted with $Al(NO_3)_3 \cdot 9H_2O$ at 60 °C for 2 hours, the feeding concentration of the phospholipid complex was 4.5mg/mL, and the

feeding ratio of the phospholipid complex to the $Al(NO_3)_3 \cdot 9H_2O$ was 1: 1.

**Example 3. Preparation of mRNA-loaded metal-chelated phospholipid complex nanoparticles (mRNA@MPP)**

Preparation of mRNA-loaded metal-chelated phospholipid complex nanoparticle with $Fe^{3+}$ as metal ion

A metal-chelated phospholipid complex was prepared according to the method in Example 2.1, wherein DSPC, curcumin and $FeCl_3$ were fed according to a feeding ratio of 1:1:1, and the metal-chelated phospholipid complex, distearoylphosphatidylcholine (DSPC, Formula 46, as a non-cationic lipid or a non-ionizable lipid), cholesterol (CHOL, Formula 40, as a non-cationic lipid or a non-ionizable lipid), and DSPE-PEG2000 (Formula 53, as a particle aggregation-inhibiting conjugated lipid ) were dissolved in ethanol in different molar proportions as an organic phase. Among those, the metal-chelated phospholipid complex, DSPC, CHOL and DSPE-PEG2000 accounted for 15%, 35%, 46% and 4%, respectively. mRNA was dissolved at a concentration of 20 μg/mL in enzyme-free PBS buffer (PBS consists of 0.137M sodium chloride, 0.0027M potassium chloride, 0.01M disodium hydrogen phosphate and 0.0018M potassium dihydrogen phosphate) as an aqueous phase. The metal-chelated phospholipid complex was mixed in a microfluidic chip with the mRNA at a mass ratio of 40:1. The volume ratio of the aqueous phase to the organic phase was 3:1. The flow rate of the organic and aqueous phases in the microfluidic chip was 12 ml/min. Among those, the drug mRNA was the mRNA encoding the fluorescent protein eGFP, and its sequence was SEQ ID NO.1 (720nt). eGFP-mRNA@MPP was prepared. The eGFP-mRNA@MPP was incubated with 293T cells at a concentration of 2 μg/mL (the concentration of mRNA contained), and the control group was incubated with MPP without drug loading, and the cell suspension was collected after 48 h, and the percentage of eGFP-positive cells was detected by flow cytometry.

The particle size, surface potential and stability of the eGFP-mRNA@MPP prepared in Example 3 were measured, and the efficiency of nucleic acid encapsulation of eGFP-mRNA@MPP was calculated.

A method for detecting particle size and criteria for judging the results: the particle size of nanoparticles was tested by using the Malvern laser particle size analyzer Zetasizer, and the particle size was considered acceptable in the range of 30~400 nm.

A method for detecting surface potential and criteria for judging the results: the surface potential of nanoparticles was tested by using the Malvern laser particle size analyzer Zetasizer, and the potential was considered acceptable in the range of -10-10 mV.

A method for detecting stability and criteria for judging the results: the nanoparticles were placed at 4 °C for 7 days, and the particle size and surface potential of the nanoparticles were tested by the Malvern laser particle size analyzer Zetasizer, and the stability was considered to be good when the particle size and surface potential did not change significantly within 3-7 days.

A method for calculating nucleic acid encapsulation efficiency: agarose gel electrophoresis was used. Firstly, the feed amount of nucleic acid of each group of lipid nanoparticles was set at 10 μg/mL, and the mass ratio of metal-chelated phospholipid complex with $Fe^{3+}$ as metal ion to mRNA was 40:1, and the same concentration of nucleic acids was dissolved in PBS buffer as a positive control, and the negative control was PBS buffer. The agarose gel had a concentration of 1.5%, where the void of gel permitted only free nucleic acids to pass through, blocking lipid nanoparticles. Electrophoresis was halted once the free nucleic acid bands were distinctly separated and visible. The gray values of free nucleic acids in different groups were calculated by software Image J, the positive control group was set as 100%, and the proportion of free nucleic acids in each group to the positive control was a relative amount of free nucleic acids, and then the encapsulation efficiency of each group was calculated as (100- relative amount of free nucleic acids) %. A nucleic acid encapsulation efficiency of more than 50% was considered to be acceptable.

Cell culture method: Human embryonic kidney cell line 293T was cultured with DMEM medium containing 10% FBS and 1% penicillin-streptomycin at a condition of 37°C and 5% $CO_2$.

A method to analyze the percentage of eGFP-positive cells by a flow cytometry method: 293T cells were seeded on 24-well plates at a density of $5\times10^5$ cells/well, and when the cell density reached 80%, cells were incubated with 1 mL of MPP or eGFP-mRNA@MPP, wherein the eGFP-mRNA@MPP had a concentration of 2 μg/mL. After 48 hours, the cell suspension was collected, 20,000 cells were collected with FITC channel of the flow cytometry, and the percentage of eGFP-positive cells was analyzed, and the calculation Formula was as follows: eGFP-positive cell rate = number of eGFP-expressing cells/total number of cells ×100%. A percentage of eGFP-positive cells above 40% was considered acceptable.

**Preparation of a mRNA-loaded metal-chelated phospholipid complex nanoparticle with $Al^{3+}$ as metal ion**

A metal-chelated phospholipid complex was prepared according to the method in Example 2.2, wherein DSPC, curcumin and $Al(NO_3)_3 \cdot 9H_2O$ were fed according to a feeding ratio of 1:1:1, and the metal-chelated phospholipid complex, distearoylphosphatidylcholine (DSPC, Formula 46, as a non-cationic lipid or a non-ionizable lipid), cholesterol (CHOL, Formula 40, as a non-cationic lipid or a non-ionizable lipid), and DSPE-PEG2000 (Formula 53, as a particle aggregation-inhibiting conjugated lipid ) were dissolved in ethanol in different molar ratios as an organic phase. Among those, the metal-chelated phospholipid complex, DSPC, CHOL and DSPE-PEG2000 accounted for 7%, 34%, 56% and 3%, respectively. mRNA was dissolved at a concentration of 20 μg/mL in PBS buffer (PBS consists of 0.137M sodium chloride, 0.0027M potassium chloride, 0.01M disodium hydrogen phosphate and 0.0018M potassium dihydrogen phosphate) as an aqueous phase. The metal-chelated phospholipid complex was mixed with the mRNA in a mass ratio of 13.3:1 in a microfluidic chip. The volume ratio of the aqueous phase to the organic phase is 3:1. The flow rate of the organic and aqueous phases in the microfluidic chip was 12 ml/min. Among those, the drug mRNA is the mRNA encoding the fluorescent protein eGFP, and its sequence was SEQ ID NO.1 (720nt). eGFP-mRNA@MPP was prepared. eGFP-mRNA@MPP was incubated with 293T cells at a concentration of 2 μg/mL (the concentration of mRNA contained), the control group was incubated with MPP without drug loading, the cell suspension was collected after 48 h, and the percentage of eGFP-positive cells was detected by flow cytometry.

The particle size, surface potential and stability of the eGFP-mRNA@MPP prepared in Example 3 were measured, and the nucleic acid encapsulation efficiency of eGFP-mRNA@MPP was calculated.

A method for detecting particle size and criteria for judging the results: the particle size of nanoparticles was tested by using the Malvern laser particle size analyzer Zetasizer, and the particle size was considered acceptable in the range of 30~400 nm.

A method for detecting surface potential and criteria for judging the results: the surface potential of nanoparticles was tested by using the Malvern laser particle size analyzer Zetasizer, and the potential was considered acceptable in the range of -10-10 mV.

A method for detecting stability and criteria for judging the results: the nanoparticles were placed at 4 °C for 7 days, and the particle size and surface potential of the nanoparticles were tested by the Malvern laser particle size analyzer Zetasizer, and the stability was considered to be good when the particle size and surface potential did not change significantly within 3-7 days.

A method for calculating nucleic acid encapsulation efficiency: agarose gel electrophoresis was used. Firstly, the feed amount of nucleic acid of each group of lipid nanoparticles was set at 10 μg/mL, and the mass ratio of metal-chelated phospholipid complex with $Al^{3+}$ as metal ion to mRNA was 13.3:1, and the same concentration of nucleic acids was dissolved in PBS buffer as a positive control, and the negative control was PBS buffer. The agarose gel was at a concentration of 1.5%, where the void of gel permitted only free nucleic acids to pass through, blocking lipid nanoparticles. Electrophoresis was halted once the free nucleic acid bands were distinctly separated and visible. The gray values of free nucleic acids in different groups were calculated by software Image J, the positive control group was set as 100%, and the proportion of free nucleic acids in each group to the positive control was a relative amount of free nucleic acids, and then the encapsulation efficiency of each group was calculated as (100- relative amount of free nucleic acids)%. A nucleic acid encapsulation efficiency of more than 50% is considered to be acceptable.

Cell culture method: Human embryonic kidney cell line 293T was cultured with DMEM medium containing 10% FBS and 1% penicillin-streptomycin at a condition of 37°C and 5% $CO_2$.

A method to analyze the percentage of eGFP-positive cells by a flow cytometry method: 293T cells were seeded on 24-well plates at a density of $5\times10^5$ cells/well, and when the cell density reached 80%, cells were incubated with 1 mL of MPP or eGFP-mRNA@MPP wherein the eGFP-mRNA@MPP has a concentration of 2 μg/mL. After 48 hours, the cell suspension was collected, 20,000 cells were collected with FITC channel of the flow cytometry, and the percentage of eGFP-positive cells was analyzed, and the calculation Formula was as follows: eGFP-positive cell rate = number of eGFP-expressing cells/total number of cells $\times$100%. A percentage of eGFP-positive cells above 40% was considered acceptable.

The principle of loading nucleic acids in metal-chelated phospholipid complex nanoparticles (MPP) assembled by metal-chelated phospholipid complex is as follows: curcumin is connected to DSPC through hydrogen bond, meanwhile curcumin is connected to $Fe^{3+}$ or $Al^{3+}$ through coordination bond, forming the metal-chelated phospholipid complex, and the $Fe^{3+}$ or $Al^{3+}$ of the metal-chelated phospholipid complex is linked to nucleic acids through coordination bond. This ensures that the metal-chelated phospholipid complex and other components self-assemble into MPPs while loading nucleic acids into nanoparticles. There are two possibilities for the contribution of curcumin in the loading of nucleic acids in MPP: (1) curcumin interacts with nucleic acids to assist in the loading of nucleic acids in MPPs, for example, curcumin assists in loading nucleic acids by being inserted into minor grooves of nucleic acids; (2) curcumin may also not interact directly with nucleic acids.

### Example 3.1 Feeding ratios of components of metal-chelated phospholipid complex

DSPC, curcumin and $FeCl_3$ in Example 3 were fed into different feeding ratios (1: 1:1, 3: 3:2, 2:2: 1), and following the other procedures which are same as in Example 3, different eGFP-mRNA@MPP were prepared and their nucleic acid encapsulation rates were detected respectively.

Result analysis: as shown in Table 1-1, when the feeding ratio among DSPC, curcumin and $FeCl_3$ was 1:1:1, the

eGFP-mRNA encapsulation efficiency of the prepared drug-lipid particles was 97%; when the feeding ratio among DSPC, curcumin and $FeCl_3$ was 3:3:2, the eGFP-mRNA encapsulation efficiency of the prepared drug-lipid particles was 70%; when the feeding ratio among DSPC, curcumin and $FeCl_3$ was 2:2:1, the eGFP-mRNA encapsulation efficiency of the prepared drug-lipid particles was 60%. The function of $Fe^{3+}$ in drug-lipid particles is to connect phospholipid complex and nucleic acids, and each $Fe^{3+}$ has up to three complexing sites, so the feeding ratio among DSPC, curcumin and $FeCl_3$ in the drug-lipid particles should be 1:1:1, so as to ensure that the drug-lipid particles can encapsulate as many nucleic acids as possible. The experimental results also confirmed that when the feeding ratio among DSPC, curcumin and $FeCl_3$ was 1:1:1, the mRNA encapsulation efficiency of drug-lipid particles prepared therefrom was the highest. When the feeding ratios among DSPC, curcumin and $FeCl_3$ were in the range of 1:1:1 to 2:2:1, the nucleic acid encapsulation efficiency of the drug-lipid complex nanoparticles were all more than 60%.

Table 1-1 Feeding ratios of components of metal-chelated phospholipid complexes and the functions of the drug-lipid particles prepared therefrom

| feeding ratio among DSPC, curcumin and $FeCl_3$ | mRNA encapsulation efficiency of drug-lipid particles |
|---|---|
| 1: 1: 1 | 87% |
| 3: 3: 2 | 70% |
| 2: 2: 1 | 60% |

The DSPC, curcumin and Al $(NO_3)_3 \cdot 9H_2O$ in Example 3 were added in different ratios (1:1:1, 3:3:2, 2:2:1), and the other steps were the same as in Example 3 to prepare different eGFP-mRNA@MPPs, and their nucleic acid encapsulation efficiency were detected respectively.

Result analysis: as shown in Table 1-2, when the ratio among DSPC, curcumin and $Al(NO_3)_3 \cdot 9H_2O$ was 1:1:1, the eGFP-mRNA encapsulation efficiency of the prepared metal-chelated phospholipid complex nanoparticles was 98%; when the ratio among DSPC, curcumin and $Al(NO_3)_3 \cdot 9H_2O$ was 3:2:2, the eGFP-mRNA encapsulation efficiency of the prepared metal-chelated phospholipid complex nanoparticles was 72%; when the ratio among DSPC, curcumin and $Al(NO_3)_3 \cdot 9H_2O$ was 2:2:1, the eGFP-mRNA encapsulation efficiency of the prepared metal-chelated phospholipid complex nanoparticles was 58%. The function of $Al^{3+}$ in the metal-chelated phospholipid complex nanoparticles is to connect the phospholipid complex with the nucleic acids, and each $Al^{3+}$ has a maximum of three complexing sites, so the feeding ratio among DSPC, curcumin and $Al(NO_3)_3 \cdot 9H_2O$ in the drug-lipid particles should be 1:1:1 to ensure that the metal-chelated phospholipid complex nanoparticles can encapsulate as many nucleic acids as possible. The results of the experiment also confirmed that when the ratio feeding ratio among DSPC, curcumin and $Al(NO_3)_3 \cdot 9H_2O$ was 1:1:1, the eGFP-mRNA encapsulation efficiency of metal-chelated phospholipid complex nanoparticles prepared from the same was the highest. When the ratio among DSPC, curcumin and $Al(NO_3)_3 \cdot 9H_2O$ was in the range of 1:1:1 to 2:2:1, the nucleic acid encapsulation efficiency of the metal-chelated phospholipid complex nanoparticles was above 60%.

Table 1-2 Feeding ratios of components of the metal-chelated phospholipid complexes when the metal ions are $Al^{3+}$ and the functions of the prepared metal-chelated phospholipid complex nanoparticles

| Feeding ratio among DSPC, curcumin and $Al(NO_3)_3 \cdot 9H_2O$ | mRNA encapsulation efficiency of metal-chelated phospholipid complex nanoparticles |
|---|---|
| 1: 1: 1 | 92% |
| 3: 3: 2 | 72% |
| 2: 2: 1 | 58% |

**Example 3.2 Proportions of metal-chelated phospholipid complex, distearoylphosphatidylcholine (DSPC), DSPE-PEG2000 and cholesterol (CHOL) in the preparation of drug-lipid particles**

Compared with Example 3, the ratios of metal-chelated phospholipid complex, distearoylphosphatidylcholine (DSPC), DSPE-PEG2000, cholesterol (CHOL) are shown in Tables 1-3 (with $Fe^{3+}$ as metal ion) and Tables 1-4 (with $Al^{3+}$ as the metal ion), and the other conditions were the same.

As shown in Table 1-3, when the proportion of metal-chelated phospholipid complex (with $Fe^{3+}$ as metal ion) was in the range of (10-40)%, the proportion of DSPC was in the range of (0-40)%, the proportion of CHOL was in the range of (35-75)%, and the proportion of DSPE-PEG2000 was in the range of (2-10)%, the particle size of the drug-lipid particles was in the range of 50~400nm, the surface potential was in the range of -10-10 mV, the stability in vitro was more than 3

days, and the mRNA encapsulation efficiency was more than 50%, and the eGFP protein positive cell rate was more than 70%. Among those, when the metal-chelated phospholipid complex accounted for 15%, distearoylphosphatidylcholine (DSPC) accounted for 35%, cholesterol (CHOL) accounted for 46%, and DSPE-PEG2000 accounted for 4%, the performance of drug-lipid particles was optimal, that is, the particle size was 110 nm and the surface potential was -2.04 mV, and the stability in vitro was >7 days, the mRNA encapsulation efficiency was 87%, and the eGFP protein positive cell rate was 97%. Because mRNA@MPP mainly rely on the metal-chelated phospholipid complex to adsorb nucleic acids, the proportion of metal-chelated phospholipid complex should not be too low. When the content of DSPC is in the range of 0-40%, the stability of nanoparticles prepared therefrom is within the acceptable range; when the content of DSPC is 0%, because the metal-phospholipid complex contains DSPC, the stable properties of its nanoparticles are maintained. The function of DSPE-PEG2000 is to prevent aggregation of nanoparticles and increase the circulation time in vivo, and its content in the range of 2-10% would provide better performance. The function of CHOL is to enhance the fluidity of nanoparticles, a certain content of CHOL is conducive to the stability of nanoparticles.

The above results suggest that mRNA@MPP has better drug loading performance when the proportion of metal-chelated phospholipid complex (metal ion is $Fe^{3+}$) is in the range of (10-40)%, the proportion of DSPC is in the range of (0-40)%, the proportion of CHOL is in the range of (35-75)%, and the proportion of DSPE-PEG2000 is in the range of (2-10)%.

Tables 1-3 Performance tests of eGFP-mRNA@MPP ($Fe^{3+}$) with different component proportions

| Factors | Metal-chelated phospholipid complexes | DSPC | CHOL | DSPE-PEG2000 | Particle size (nm) | Surface potential (mV) | Stability (days) | mRNA encapsulation efficiency | eGFP-positive cell rate |
|---|---|---|---|---|---|---|---|---|---|
| 1 | 25% | 25% | 40% | 10% | 120 | -1.70 | >7 | 84% | 97% |
| 2 | 40% | 0 | 55% | 5% | 300 | -0.51 | 3 | 80% | 91% |
| 3 | 20% | 20% | 50% | 10% | 110 | -4.03 | >7 | 65% | 78% |
| 4 | 10% | 30% | 58% | 2% | 100 | -0.41 | 3 | 60% | 73% |
| 5 | 30% | 0 | 60% | 10% | 180 | -4.27 | 5 | 81% | 90% |
| 6 | 30% | 15% | 50% | 5% | 150 | -0.57 | 5 | 77% | 82% |
| 7 | 30% | 5% | 55% | 10% | 160 | -3.81 | >7 | 78% | 81% |
| 8 | 20% | 0 | 75% | 5% | 150 | -1.18 | 5 | 68% | 75% |
| 9 | 20% | 30% | 40% | 10% | 98 | -1.55 | 5 | 75% | 79% |
| 10 | 15% | 40% | 43% | 2% | 120 | -1.05 | 3 | 70% | 75% |
| 11 | 20% | 40% | 35% | 5% | 130 | -1.96 | 5 | 72% | 74% |
| 12 | 15% | 35% | 46% | 4% | 110 | -2.04 | >7 | 87% | 97% |

Result analysis: as shown in Tables 1-4, when the proportion of metal-chelated phospholipid complex (metal ion is $Al^{3+}$) was in the range of (5-50)%, the proportion of DSPC was in the range of (0-51)%, the proportion of CHOL was in the range of (15-80)%, and the proportion of DSPE-PEG2000 was in the range of (2-10)%, the particle size of the drug-lipid particles was in the range of 50~400nm, the surface potential was in the range of -10-10 mV, the stability in vitro was more than 3 days, and the mRNA encapsulation efficiency was more than 50%, and the eGFP protein positive cell rate was more than 70%. Among those, when the metal-chelated phospholipid complex accounted for 7%, distearoylphosphatidylcholine (DSPC) accounted for 34%, cholesterol (CHOL) accounted for 56%, and DSPE-PEG2000 accounted for 3%, the performance of the drug-lipid particles was optimal, that is, the particle size was 100 nm and the surface potential was -1.57 mV, and the stability in vitro was >7 days, the mRNA encapsulation efficiency was 92%, and the eGFP protein positive cell rate was 98%. Because the mRNA@MPP mainly relies on metal-chelated phospholipid complex to adsorb nucleic acids, the proportion of metal-chelated phospholipid complexes should not be too low; when the content of DSPC was in the range of 0-51%, the stability of its nanoparticles was within an acceptable range; when the content of DSPC is 0%, because the metal-phospholipid complex contains DSPC, the stable properties of its nanoparticles are maintained. The function of DSPE-PEG2000 is to prevent nanoparticle aggregation and increase the circulation time in the body, and its content in the range of 2-10% would have better performance; the function of CHOL is to enhance the fluidity of nanoparticles, and maintaining a certain content is conducive to the stability of nanoparticles.

The above results suggest that the mRNA@MPP has better drug loading performance when the metal-chelated phospholipid complex (metal ion is $Al^{3+}$) accounts for (5-50)%, DSPC accounts for (0-51)%, CHOL accounts for (15-80)%, and DSPE-PEG2000 accounts for (2-10)%,.

Table 1-4 Performance tests of eGFP-mRNA@MPP ($Al^{3+}$) with different component proportions

| Factors | Metal-chelated phospholipid complexes | DSPC (Formula 51) | CHOL (Formula 59) | DSPE-PEG2000 (Formula 58) | Particle size (nm) | Surface potential (mV) | Stability (days) | mRNA encapsulation efficiency | eGFP-positive cell rate |
|---|---|---|---|---|---|---|---|---|---|
| 1 | 20% | 0% | 74% | 6% | 130 | -1.76 | 3 | 84% | 77% |
| 2 | 13% | 0% | 80% | 7% | 85 | -3.69 | 5 | 80% | 72% |
| 3 | 50% | 0% | 40% | 10% | 87 | -4.17 | 3 | 88% | 78% |
| 4 | 5% | 47% | 44% | 4% | 160 | -0.97 | 5 | 75% | 73% |
| 5 | 30% | 45% | 15% | 10% | 245 | -4.27 | 5 | 81% | 90% |
| 6 | 7% | 34% | 56% | 3% | 100 | -1.57 | >7 | 92% | 98% |
| 7 | 8% | 50% | 55% | 4% | 200 | -3.44 | 3 | 70% | 81% |
| 8 | 5% | 50% | 43% | 2% | 140 | -1.01 | 3 | 70% | 75% |
| 9 | 25% | 25% | 40% | 10% | 268 | -4.57 | >7 | 75% | 70% |
| 10 | 25% | 51% | 15% | 9% | 220 | -1.55 | >7 | 72% | 76% |
| 11 | 20% | 40% | 35% | 5% | 190 | -3.75 | 5 | 74% | 73% |

**Example 3.3 Types of non-cationic lipid or non-ionizable lipid for preparation of eGFP-mRNA@MPP**

Compared with Example 3, the replacement of distearoylphosphatidylcholine (DSPC) is shown in Tables 1-5 and 1-6, and the other conditions are the same.

Result analysis: in order to explore whether DSPC in eGFP-mRNA@MPP can be replaced by other non-cationic lipids or non-ionizable lipids. Three other non-cationic lipids or non-ionizable lipids, that is, DSPE, DSPA and DSPG were selected to replace DSPC respectively, and the particle size, surface potential, stability and mRNA encapsulation efficiency were detected to prove that DSPC in eGFP-mRNA@MPP can be replaced by other non-cationic lipids or non-ionizable lipids, and their function after replacement are equivalent to that of eGFP-mRNA @ MPP containing DSPC (Table 1-5 (metal ion is $Fe^{3+}$) and Table 1-6 (metal ion is $Al^{3+}$). Because the main function of non-cationic lipid or non-ionizable lipids DSPC in eGFP-mRNA@MPP is to make liposome membrane more fusible, more stable and less toxic, and other non-cationic lipids or non-ionizable lipids also have the function of making liposome membrane more fusible, more stable and less toxic, so DSPC in drug-lipid particles can be replaced by other non-cationic lipid or non-ionizable lipid other than the metal-chelated phospholipid complex and the particle aggregation-inhibiting conjugated lipid, and their efficacy would not be affected.

The structures of the three other non-cationic lipids or non-ionizable lipids (DSPE, DSPA and DSPG) are shown as follows.

DSPE

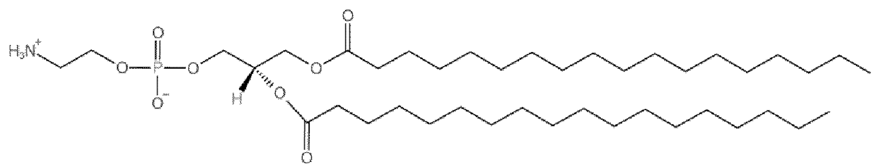

(Formula 47)

DSPA

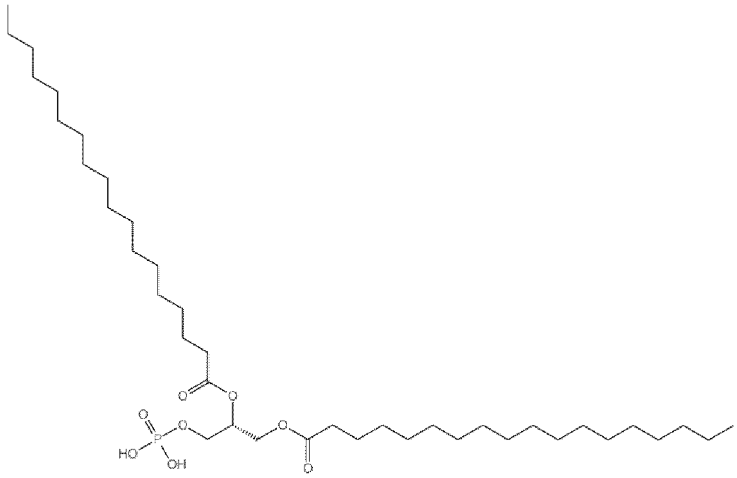

(Formula 48)

DSPG

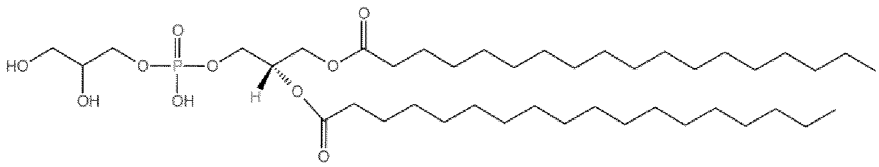

(Formula 49)

Table 1-5 Performances of eGFP-mRNA@MPPs ($Fe^{3+}$) containing different non-cationic lipids or a non-ionizable lipids other than the metal-chelated phospholipid complex and the particle aggregation-inhibiting conjugated lipid

| Non-cationic lipids or non-ionizable lipids | DSPC | DSPE | DSPA | DSPG |
|---|---|---|---|---|
| Particle size (nm) | 110 | 130 | 125 | 140 |
| Surface potential (mV) | -2.04 | -1.81 | -2.05 | -1.77 |
| Stability (days) | >7 | >7 | >7 | >7 |
| mRNA encapsulation efficiency (%) | 87 | 75 | 78 | 76 |
| eGFP-positive cell rate (%) | 97 | 92 | 94 | 85 |

Table 1-6 Performances of eGFP-mRNA@MPPs ($Al^{3+}$) containing different non-cationic lipids or non-ionizable lipids other than the metal-chelated phospholipid complex and the particle aggregation-inhibiting conjugated lipid

| Non-cationic lipids or non-ionizable lipids | DSPC | DSPE | DSPA | DSPG |
|---|---|---|---|---|
| Particle size (nm) | 100 | 110 | 105 | 120 |
| Surface potential (mV) | -1.57 | -2.80 | -1.07 | -1.93 |
| Stability (days) | >7 | >7 | >7 | >7 |
| mRNA encapsulation efficiency (%) | 92 | 79 | 78 | 81 |
| eGFP-positive cell rate (%) | 98 | 91 | 92 | 90 |

**Example 3.4 Types of particle aggregation-inhibiting conjugated lipids for preparation of eGFP-mRNA@MPP**

Compared with Example 3, the replacement of DSPE-PEG2000 (Formula 53) is shown in Table 1-7 (metal ion is $Fe^{3+}$) and Table 1-8 (metal ion is $Al^{3+}$), and the other conditions are the same. The three particle aggregation-inhibiting conjugated lipids are DSPE-PEG700 (Formula 50), DSPE-PEG500 (Formula 52) and DSPE-PEG1000 (Formula 51).

Result analysis: in order to explore whether DSPE-PEG2000 in eGFP-mRNA@MPP can be replaced by other

particle aggregation-inhibiting conjugated lipids, three other particle aggregation-inhibiting conjugated lipids, namely DSPE-PEG700, DSPE-PEG5000 and DSPE-PEG1000, were selected to replace DSPE-PEG2000 respectively, and the particle size, surface potential, stability and mRNA encapsulation efficiency were tested to prove that DSPE-PEG2000 in eGFP-mRNA@MPP can be replaced by other particle aggregation-inhibiting conjugated lipids, and their function after replacement are equivalent to the efficacy of eGFP-mRNA@MPP containing DSPE-PEG2000 (Tables 1-7 and 1-8). Because the main function of DSPE-PEG2000 in eGFP-mRNA@MPP is to inhibit aggregation, and other particle aggregation-inhibiting conjugated lipids also have the function of inhibiting aggregation, DSPE-PEG2000 in eGFP-mRNA@MPP can be replaced by other particle aggregation-inhibiting conjugated lipids, and their efficacy would not be affected.

Table 1-7 Performances of eGFP-mRNA@MPPs ($Fe^{3+}$) containing different particle aggregation-inhibiting conjugated lipids

| Particle aggregation-inhibiting conjugated lipid | DSPE-PEG2000 | DSPE-PEG700 | DSPE-PEG 1000 | DSPE-PEG5000 |
|---|---|---|---|---|
| Particle size (nm) | 110 | 110 | 128 | 124 |
| Surface potential (mV) | -2.04 | -1.66 | -2.01 | -1.97 |
| Stability (days) | >7 | >7 | >7 | >7 |
| mRNA encapsulation | 87 | 80 | 76 | 78 |
| efficiency (%) | | | | |
| eGFP-positive cell rate (%) | 97 | 92 | 95 | 80 |

Table 1-8 Performances of eGFP-mRNA@MPPs ($Al^{3+}$) containing different particle aggregation-inhibiting conjugated lipids

| Particle aggregation-inhibiting conjugated lipid | DSPE-PEG2000 | DSPE-PEG700 | DSPE-PEG 1000 | DSPE-PEG5000 |
|---|---|---|---|---|
| Particle size (nm) | 100 | 95 | 90 | 120 |
| Surface potential (mV) | -1.57 | -1.50 | -1.98 | -1.80 |
| Stability (days) | >7 | >7 | >7 | >7 |
| mRNA encapsulation efficiency (%) | 92 | 86 | 85 | 88 |
| eGFP-positive cell rate (%) | 98 | 95 | 95 | 90 |

**Example 3.5 Preparation and effect characterization of mRNA@MPP**

**Example 3.5.1 Preparation and effect characterization of mRNA@MPP with $Fe^{3+}$ as metal ion**

The mRNA in Example 3 was replaced with the other two mRNAs, and three mRNA@MPPs containing mRNA sequences of different target proteins were prepared respectively according to the method of Example 3. The three different mRNA sequences are: (1) the mRNA sequence encoding the fluorescent protein eGFP shown in SEQ ID NO.1 (720nt); (2) the mRNA sequence encoding the receptor binding domain (RBD) of S1 subunit of novel coronavirus shown in SEQ ID NO.2 (669nt); (3) the mRNA sequence encoding tumor antigen NY-ESO-1 shown in SEQ ID NO.3 (543nt). The rest of preparation process of drug (mRNA)-lipid particles is the same as that in Example 3, and eGFP-mRNA@MPP, RBD-mRNA@MPP, and NY-ESO-1-mRNA@MPP were obtained respectively.

eGFP-mRNA@MPP was incubated with 293T cells at a concentration of 2 μg/mL (the concentration of mRNA contained), and the control group was incubated with MPP. After 48 hours, the cell suspension was collected, and the percentage of eGFP-positive cells was detected by flow cytometry. The results are shown in Figure 1-1; RBD-mRNA@MPP was incubated with 293T cells at a concentration of 2 μg/mL (the concentration of mRNA contained), and the control group was incubated with MPP. After 24 h, the supernatant was frozen at -20°C for later use after centrifugation. The expression level of the novel coronavirus antigen RBD protein in the cell supernatant was detected using a commercially available SARS-COV-2 antigen RBD ELISA detection kit. The results are shown in Figure 1-2.

ELISA method for detecting RBD expression level:

1. Sample collection: the cell supernatant was placed at room temperature for 2 hours, centrifuged at 1000×g for 20 minutes, and the supernatant was taken;
2. Sample addition: blank wells, standard wells, and test sample wells were set on the coated plate. 100μL of sample diluent was added to the blank wells, and serially diluted standard samples were added to the standard sample wells, and 100 μL of the sample to be tested was added to the test sample wells, which were all incubated at 37°C for 60 minutes.
3. The liquid in the wells was discarded. The plate was washed by 3 times, and soaked for 1-2 minutes each time. 100μL of the prepared biotin-labeled anti-RBD antibody working solution was added to each well, mixed well, and incubated at 37°C for 60 minutes.
4. The liquid in the wells was discarded, the plate was washed by 3 times, and soaked for 1-2 minutes each time.
5. 100 μL of the prepared streptavidin HRP working solution was added to each well, mixed well, and incubated at 37°C for 45 min.
6. The liquid in the wells was discarded, the plate was washed by 3 times, and soaked for 1-2 minutes each time.
7. 100 μL of TMB substrate solution was added to each well, and incubated at 37°C in the dark for 15 minutes.
8. 100 μL of stop solution was added to each well to terminate the reaction.
9. The optical density (OD) value of each well was measured at a wavelength of 450 nm.

Data analysis: The standard curve was drawn with the concentration of the standard samples as the horizontal axis and the OD value as the vertical axis.

The experimental animals were randomly divided into 2 groups (experimental group and control group), with 5 mice in each group. Among those, the animal model of RBD-mRNA@MPP was BALB/c mouse. Each mouse was given the first intramuscular administration on the first day and the second intramuscular administration on the 14th day. The experimental group was injected with RBD-mRNA@MPP, and the control group was injected with metal-chelated phospholipid complex nanoparticles (MPP) without mRNA loading. The dose of each administration was 100 μL, and the RBD-mRNA@MPP preparation in the experimental group contained 30 mg of mRNA. On the 28th day after the first administration, the blood of mice was collected, the serum was separated and diluted in series, and the total IgG antibodies against the RBD of S1 subunit of the SARS-COV-2produced in the body of mice were detected by a commercially available ELISA kit. The results are shown in Figures 1-3.

The animal model of NY-ESO-1-mRNA@MPP was C57BL/6 mice. Each mouse was intramuscularly administered four times on days 1, 7, 14, and 21. The experimental group was injected with NY-ESO-1-mRNA@MPP, and the control group was injected with metal-chelated phospholipid complex nanoparticles (MPP) without mRNA loading. The dose of each administration was 100 μL, and the NY-ESO-1-mRNA@MPP preparation in the experimental group contained 30 mg of mRNA. On the 28th day after the first administration, the blood of mice was collected, the serum was separated and diluted in series, and the total anti-NY-ESO-1 IgG antibodies produced in the body of mice were detected by ELISA. The results are shown in Figures 1-4.

Method for detecting total anti-NY-ESO-1 IgG antibodies in mice:

Preparation of reagents used in ELISA method:

1. Coating solution: 8.4g $NaHCO_3$ was accurately weighed and dissolved in 1L distilled water (DDW). After the solid was completely dissolved, the pH of the entire solution was adjusted to 9.6 with 1M NaOH solution, and the prepared coating solution was stored at 4°C for later use.
2. Washing solution: 0.5mL Tween-20 was added to 1L 0.01M PBS solution, mixed well and placed at room temperature.
3. Blocking solution: 20g BSA was accurately weighed and added to 1L 0.01M PBS solution. The undissolved BSA powder was ultrasonicated in the solution. When the solid was completely dissolved in the solution which then turned to light yellow, the solution was stored in a refrigerator at 4°C for later use.
4. Antibody diluent: 2.5g BSA was accurately weighed and dissolved in 250mL 0.01M PBS solution. After the solid was completely dissolved, 1.25mL Tween-20 was added, mixed well and stored at 4°C for later use.
5. Color development solution:

(A) 0.1M citric acid: 19.2g citric acid was added to DDW water to 1000mL
(B) 0.2M disodium hydrogen phosphate: 28.4g anhydrous disodium hydrogen phosphate was added to DDW water to 1000mL

24.3mL of 0.1M citric acid solution (A) was mixed with 25.7mL of 0.2M phosphate buffer (B) and added with 50mL of DDW water. 50mg OPD (o-phenylenediamine) and 0.15mL 30% $H_2O_2$ was added before use.
6. Stop solution: 2M $H_2SO_4$: 55.5mL concentrated sulfuric acid was added with DDW to 500mL.

ELISA method was used to determine the titer of antibodies in mouse serum:

1. Coating: NY-ESO-1 antigen was diluted to 1 μg/mL with coating solution, added to 96-well plate, 50μL/well, and coated overnight at 4°C.
2. Blocking: the coating solution in the well plate was dried by shaking, then washed with the blocking solution three times, for 5 minutes each time, and shaken to dry. Each well was filled with 150μL of blocking solution and incubated at 37°C for 2 hours.
3. Drying: the blocking solution was dried by shaking, and incubated at 37°C for 1 to 2 hours, until the liquid at the bottom of the well plate had completely evaporated.
4. Immunization: The serum samples were first diluted to a 1:1000 ratio using antibody diluent and then underwent serial 1:2 dilutions. These diluted serum samples were dispensed into a blocked 96-well plate with 100 μL per well, and incubated at 37°C for 2 hours. The liquid in the wells was dried by shaking, and each well was washed with 300 μL of washing solution, followed by gentle shaking for 40 seconds and this step was repeated three times. A biotinylated goat anti-mouse IgG antibody, diluted to 1:1000, was introduced to the well plate with 100 μL per well and incubated for 1 hour at 37°C. The liquid in the well plate was dried by shaking, then washing solution was added, and the washing step was repeated. 100 μL of freshly prepared streptavidin-labeled horseradish peroxidase HRP working solution was added to each well and incubated at 37°C for 1 hour. The liquid in the well plate was dried by shaking, then washing solution was added, and the washing step was repeated. 100 μL of color development solution was added per well and allowed to react at room temperature for 5 minutes. The color development was halted by adding 50 μL of stop solution per well. Absorbance at 450nm was then measured using an ELISA reader.

On the 28th day following the administration of RBD-mRNA@MPP, spleens from healthy mice were harvested, and a single-cell suspension was prepared under sterile conditions. 100,000 spleen cells per well were plated in a cell culture plate, followed by the addition of RBD protein at a final concentration of 10 mg/mL and incubation for 48 hours. The supernatant was removed by centrifugation, and the expression levels of IFN-γ, IL-2, and IL-4 were determined by ELISA kits. The results are shown in Figures 1-5.

On the 28th day following the administration of NY-ESO-1-mRNA@MPP, spleens from normal mice were harvested, and a single-cell suspension was prepared under sterile conditions. 100,000 spleen cells per well were plated in a cell culture plate, followed by the addition of NY-ESO-1 protein at a final concentration of 10 mg/mL and incubation for 48 hours. The supernatant was removed by centrifugation, and the expression levels of IFN-γ, IL-2, and TNF-α were determined by ELISA kits. The results are shown in Figures 1-6.

Result analysis: as depicted in Figure 1-1, the experimental group treated with eGFP-mRNA@MPP exhibited a 97% rate of eGFP-positive cells, while the MPP control group showed no detectable eGFP signal. According to Figure 1-2, the concentration of RBD protein encoded by the RBD-mRNA encapsulated within MPP reached 193.3 ng/mL in the supernatant of 293T cells, whereas the RBD protein level in the supernatant of 293T cells transfected with the empty vector MPP was 0. The results indicate that mRNA-MPP can encapsulate and deliver any mRNA, which can directly encode polypeptides in cells. Figures 1-3 and 1-4 demonstrate that both RBD-mRNA@MPP and NY-ESO-1-mRNA@MPP can effectively induce humoral immunity in mice, generating high levels of antigen-specific antibodies. Among those, the IgG antibody titer in mice treated with RBD-mRNA@MPP reached 117268.8; the IgG antibody titer in mice treated with NY-ESO-1-mRNA@MPP reached 5319.52. As shown in Figures 1-5 and 1-6, both RBD-mRNA@MPP and NY-ESO-1-mRNA@MPP can effectively induce cellular immunity in mice, that is, activate immune cells and produce a large number of cytokines. Among those, RBD-mRNA@MPP made the expression of cytokines IFN-γ, IL-2, and IL-4 reach 252.8 pg/mL, 207.6 pg/mL, and 56.6 pg/mL, respectively; NY-ESO-1-mRNA@MPP made the expression of cytokines IFN-γ, IL-2, and TNF-α reach 70.79 pg/mL, 75.29 pg/mL, and 75.27 pg/mL, respectively. The results suggest that the drug-loaded metal-chelated phospholipid complex nanoparticles (mRNA@MPP) can encapsulate and deliver any mRNA, thereby promoting the expression of target proteins (antigens), and then effectively inducing humoral immunity and cellular immunity in mice, further producing high levels of antigen-specific binding antibodies and cytokines, and playing the role of anti-SARS-COV-2 mRNA vaccines and anti-tumor mRNA vaccines.

**Example 3.5.2 Preparation and effect characterization of mRNA@MPP with $Al^{3+}$ as metal ions**

The difference between this example and Example 3.5.1 is that the metal ion $Fe^{3+}$ in Example 3.5.1 was replaced by $A1^{3+}$.

Result analysis: Figures 1-7 show that the rate of eGFP-positive cells in the eGFP-mRNA@MPP experimental group reached 98.02%, whereas no eGFP signal was detected in the MPP control group. As depicted in Figures 1-8, the RBD protein encoded by RBD-mRNA encapsulated by MPP measured 212.6 ng/mL in the supernatant of 293T cells, compared to 0 ng/mL in the supernatant of 293T cells transfected with the empty vector MPP. The results suggest that mRNA-MPP can encapsulate and deliver any mRNA and directly encode polypeptides in cells. As shown in Figures 1-9 and 1-10, both RBD-mRNA@MPP and NY-ESO-1-mRNA@MPP can effectively induce humoral immunity in mice and produce high levels of antigen-specific binding antibodies. The IgG antibody titer in mice treated with RBD-mRNA@MPP reached 129113; the IgG antibody titer in mice treated with NY-ESO-1-mRNA@MPP reached 6507.4. As shown in Figures 1-11 and 1-12, both RBD-mRNA@MPP and NY-ESO-1-mRNA@MPP can effectively induce cellular immunity in mice, i.e., activate immune cells and produce a large number of cytokines. Among those, RBD-mRNA@MPP made the expression of cytokines IFN-$\gamma$, IL-2, and IL-4 reach 271.8 pg/mL, 234.6 pg/mL, and 64.8 pg/mL, respectively; NY-ESO-1-mRNA @MPP made the expression of cytokines IFN-$\gamma$, IL-2, and TNF-$\alpha$ reach 83.8 pg/mL, 98 pg/mL, and 97.8 pg/mL, respectively. The results suggest that mRNA@MPP can encapsulate and deliver any mRNA, thereby promoting the expression of target proteins (antigens), and then effectively induce humoral immunity and cellular immunity in mice, produce high levels of antigen-specific binding antibodies and cytokines, and play the role of anti-SARS-COV-2 mRNA vaccines and anti-tumor mRNA vaccines.

**Example 3.6 Preparation and effect of siRNA-loaded metal-chelated phospholipid complex nanoparticles (siRNA @MPP)**

**Example 3.6.1 Preparation and effect of siRNA-loaded metal-chelated phospholipid complex nanoparticles (siRNA @MPP) with $Fe^{3+}$ as metal ion**

The mRNA in Example 3 was replaced with siRNA, and three siRNA@MPPs containing different siRNAs were prepared respectively according to the method of Example 3. The genes targeted by the three different siRNA, sequences and corresponding random control sequences thereof are: (1) the sequence of siRNA targeting Bcl-2 gene (Bcl-2-siRNA) shown in SEQ ID NO.4 (antisense chain) and SEQ ID No.21 (sense chain) (19 bp), and its random control sequence shown in SEQ ID NO.5 (antisense chain) and SEQ ID No.22 (sense chain) (19 bp); (2) the sequence of siRNA targeting PLK1 gene (PLK1-siRNA) shown in SEQ ID NO.6 (antisense chain) and SEQ ID No.23 (sense chain) (21 bp), and its random control sequence shown in SEQ ID NO.7 (antisense chain) and SEQ ID No.24 (sense chain) (19 bp); (3) the sequence of siRNA targeting Gal-1 gene (Gal-1-siRNA) shown in SEQ ID NO.8 (19 bp), its random control sequence shown in SEQ ID NO.9 (19 bp). The rest of preparation process of siRNA@MPP is the same as that of Example 3.

The sequence of Bcl-2-siRNA is as follows:

Antisense: 5'-CAGCUUAUAAUGGAUGUAC-3' (SEQ ID No.4);
Sense: 5'-GUACAUCCAUUAUAAGCUG-3' (SEQ ID No.21) (19 bp).

The random control sequence of Bcl-2-siRNA is as follows:

Antisense: 5'-ACGUGACACGUUCGGAGAA-3' (SEQ ID No.5);
Sense: 5'-UUCUCCGAACGUGUCACGU-3' (SEQ ID No.22) (19 bp).

The sequence of PLK1-siRNA is as follows:

Antisense: 5'-UAAGGAGGGUGAUCUUCUUCA-3' (SEQ ID No.6);
Sense: 5'-UGAAGAAGAUCACCCUCCUUA-3' (SEQ ID No.23) (21 bp).

The random control sequence of PLK1-siRNA is as follows:

Antisense: 5'-CUUACGCUGAGUACUUCGA-3' (SEQ ID No.7);
Sense: 5'-UCGAAGUACUCAGCGUAAG-3' (SEQ ID No.24) (19 bp).

The sequence of Gal-1-siRNA is as follows:
5'-GCUGCCAGAUGGAUACGAA-3' (SEQ ID No.8) (19 bp).

The random control sequence of Gal-1-siRNA is as follows:
5'-GGAAAUCCCCCAACAGUGA-3' (SEQ ID No.9) (19 bp).

Cell culture: U251 human glioblastoma cells were grown as monolayers in a medium containing high glucose (4.5 g/L) DMEM + 10% fetal bovine serum (FBS), 1% penicillin/streptomycin and 2 mml-glutamine (Bio Industries), and cultured at 37°C, 5% $CO_2$, and passaged twice a week.

After U251 cells were seeded in a 6-well plate at a density of $1\times10^6$ cells per well for about 24 hours, the cells in each well were incubated with siRNA@MPPs containing the above siRNAs (where the concentration of siRNA was 2 μg/mL) for 72 hours, and then the cells were collected and the total cell RNA was extracted. The mRNA expression levels of the target genes (Bcl-2, PLK1, and Gal-1) were detected by RT-PCR technology, and the capability of siRNA@MPPs to silence the cell target genes was statistically analyzed.

Specific RT-PCR process:

Total RNA extraction: the culture medium was discarded from the six-well plate, and rinsed with PBS buffer three times. Each well was added with 1mL of Trizol to lyse the cells. 200μL of chloroform was added, mixed thoroughly, and left at room temperature for 10 minutes. The mixture was centrifuged at 13000 rpm and 4°C for 15 minutes resulting in layered three-phase liquid, with RNA dissolved in the upper aqueous phase. The upper phase was pipetted and placed in a new enzyme-free 1.5 ml centrifuge tube, 500 μL of isopropanol was added and the solution was allowed to stand at room temperature for 10 minutes. After centrifugation at 13000 rpm and 4°C for 15 minutes, the RNA precipitate was obtained. The supernatant was discarded, and 1 mL of 75% (v/v) ethanol newly prepared with RNase-free water was added to each tube. The RNA precipitate at the bottom was gently resuspended, followed by centrifugation at 7500 rpm and 4°C for 10 minutes. The supernatant was discarded, and the liquid at the bottom was aspirated as thoroughly as possible. The RNA precipitate was air-dried at room temperature with the lid open. 50 μL of enzyme-free water was added to dissolve the precipitate. The purity and concentration of the extracted RNA were measured using an ultra-micro UV-visible spectrophotometer.

cDNA reverse transcription: RNA was reverse transcribed into cDNA using the Ta Ka Ra Prime Script™ RT reagent Kit with gDNA Eraser. The genomic DNA (gDNA) was removed before the reverse transcription step to make the results more accurate and reliable. The total RNA reverse transcription reaction system was prepared on ice: 1μL Prime Script RT Enzyme MixI, 1μL RT Primer Mix, 4μL 5×Prime Script Buffer 2, 4μL RNase Free $dH_2O$. The prepared reaction mixture was incubated at 37°C for 15 minutes, and then placed at 85°C for 5 seconds to halt the reaction, and then stored at 4°C for later use.

RT-PCR operation: this detection method was the SYBR Green dye method, and no probe was required. Specifically, real-time PCR reaction was performed using cDNA from different samples as templates. The reaction solution was prepared on ice: 5μL SYBR Premix Dimer Eraser (2×), 0.3μL PCR Forward Primer (10 μM), 0.3μL PCR Reverse Primer (10 μM), 0.2μL ROX Reference DyeII(50×), 1μL cDNA template obtained in the previous step and 3.2μL dH2O. 10μL of sample was added to each well of the plate, and centrifuged (1000 rpm, 5min) to eliminate liquid sticking to the wall and bubbles in the reaction solution. ABI ViiA7 real-time fluorescence quantitative PCR instrument was used for real-time PCR reaction detection, the reaction program was 95°C, 30 sec (1 cycle) → 95°C, 5 sec; 55°C, 30 sec; 72°C, 30 sec (40 cycles) → 60°C-95°C, 2min (1 cycle). The experiment was repeated three times, and the average value was taken to obtain Ct value of each group, and the expression fold change between the experimental group and the control group was calculated. The control gene was GAPDH. The RT-PCR primers were as follows:

(1) Bcl-2 primer: forward: 5'-AGGATTGTGGCCTTCTTTGAG-3',
reverse: 5'-AGACAGCCAGGAGAAATCAAAC-3';
(2) PLK1 primer: forward:5'-ACCAGCACGTCGTAGGATTC-3',
reverse: 5'-CAAGCAATTTGCCGTAGG-3';
(3) Gal-1 primer: forward: 5'-CAATCAT GGCCTGTGGTCTG-3',
reverse: 5'- GTG TAGGCACAGGTTGTTGCTG-3';
(4) GAPDH primer: forward: 5'-TCAGGGGTTTCACATTTGGCA-3',
reverse: 5'-GG AGCGGAA AACCA-3'.

The expression level of each target gene was represented by the RQ value ($2^{-\Delta\Delta CT}$). The Formula was as follows:

$$\text{Fold Change} = 2^{-\Delta\Delta Ct}$$

Wherein,

$$\Delta\Delta Ct = \Delta Ct_{experimental\ group} - \Delta Ct_{control\ group},\ \Delta Ct = Ct_{target\ gene} - Ct_{reference\ gene}$$

Calculation method of gene silencing efficiency: 100% - gene expression level of experimental group / gene expression level of control group.

Result analysis: As shown in Figures 1-13, 1-14, and 1-15 (where scr siRNA is a random control sequence), the three siRNA@MPP can significantly interfere with their corresponding target genes. The inhibition rate of Bcl-2-siRNA@MPP on the target gene Bcl-2 reached 76%; the inhibition rate of PLK1-siRNA@MPP on the target gene PLK1 reached 86%; and the inhibition rate of Gal-1-siRNA@MPP on the target gene Gal-1 reached 73%. The results suggest that siRNA@MPP can load any siRNA for intervention therapy of target genes and play the role of siRNA-loaded drugs, vaccines or other products.

**Example 3.6.2 Preparation and effect of siRNA-loaded metal-chelated phospholipid complex nanoparticles (siRNA @MPP) with $Al^{3+}$as metal ion**

The difference between this example and Example 3.6.1 is that the metal ion $Fe^{3+}$ in Example 3.6.1 was replaced by $A1^{3+}$.

Result analysis: As shown in Figures 1-16, 1-17, and 1-18, the three siRNA@MPPs can significantly interfere with their corresponding target genes. The inhibition rate of Bcl-2-siRNA@MPP on the target gene Bcl-2 reached 81%; the inhibition rate of PLK1-siRNA@MPP on the target gene PLK1 reached 90%; the inhibition rate of Gal-1-siRNA@MPP on the target gene Gal-1 reached 79%. The results suggest that siRNA@MPP can load any siRNA for intervention therapy of target genes, and play the role of siRNA-loaded drugs, vaccines or other products.

**Example 3.7 Preparation and effect of ASO-loaded metal-chelated phospholipid complex nanoparticles (ASO@MPP)**

**Example 3.7.1 Preparation and effect of ASO-loaded metal-chelated phospholipid complex nanoparticles (ASO@MPP) with $Fe^{3+}$ as metal ion**

The mRNA in Example 3 was replaced with ASO, and three drug-loaded metal-chelated phospholipid complex nanoparticles (ASO@MPP) containing different ASOs were prepared respectively according to the method of Example 3. The genes targeted by the three different ASO, sequences and corresponding random control sequences thereof are: (1) the sequence of the ASO targeting STAT3 gene (STAT3-ASO) shown in SEQ ID NO.10 (17nt), and its random control sequence shown in SEQ ID NO.11 (18nt); (2) The sequence of the ASO targeting the α-syn gene (α-syn-ASO) shown in SEQ ID NO.12 (16nt), and its random control sequence shown in SEQ ID NO.13 (16nt); (3) The sequence of the ASO targeting the Bcl-2 gene (Bcl-2-ASO) shown in SEQ ID NO.14 (18nt), and its random control sequence shown in SEQ ID NO.15 (20nt). The rest of preparation process of ASO-loaded metal-chelated phospholipid complex nanoparticles is the same as that of Example 3. Different ASO@MPPs were incubated with different cells: U251 human glioblastoma cells were incubated with ASO@MPP targeting STAT3 gene; SH-SY5Y human neuroblastoma cells were incubated with ASO@MPP targeting α-syn gene; Daudi human lymphoma cells were incubated with ASO@MPP targeting Bcl-2 gene. After about 24 hours of inoculation in a 6-well plate at a density of $1\times10^6$ cells per well, each well of cells was incubated with ASO@MPP containing the above ASO (where the concentration of ASO was 1μg/mL) for 48 hours, and then the cells were collected, total cell RNA was extracted, and the mRNA expression of target genes (STAT3, α-syn, Bcl-2) was detected by RT-PCR technology, and the capability of ASO@MPP to silence cell target genes was calculated.

SEQ ID No.10 sequence (sequence of STAT3-ASO) is as follows:
5'-GCTCCAGCATCTGCTTC-3' (17nt).

SEQ ID No.11 sequence (random control sequence of STAT3-ASO) is as follows:
5'-GAAGCAGCAGATGCTGGA-3' (18nt).

SEQ ID No.12 sequence (sequence of α-syn-ASO) is as follows:
5'-GCTCCCTCCACTGTCT-3' (16nt).

SEQ ID No. 13 sequence (random control sequence of α-syn-ASO) is as follows:
5'-ACTCCCGAACCTGTCT-3' (16nt).

SEQ ID No.14 sequence (sequence of Bcl-2-ASO) is as follows:
5'-TCTCCCAGCGTGCGCCAT-3' (18nt).

SEQ ID No.15 sequence (random control sequence of Bcl-2-ASO) is as follows:
5'-CAGCGTGCGCCATCCTTCCC-3' (20nt).

Cell culture: (1) U251 human glioblastoma cells were grown in a monolayer in a medium containing high glucose (4.5 g/L) DMEM + 10% fetal bovine serum (FBS), 1% penicillin/streptomycin and 2 mml-glutamine (Bio Industries), and cultured at 37°C, 5% $CO_2$, and passaged twice a week; (2) SH-SY5Y human neuroblastoma cells were grown in a monolayer in a medium containing high glucose (4.5 g/L) DMEM + 10% fetal bovine serum (FBS), 1% penicillin/streptomycin and 2 mml-glutamine (Bio Industries), and cultured at 37°C, 5% $CO_2$, and passaged twice a week; (3) Daudi human lymphoma cells were grown in a medium containing RPMI 1640 + 10% fetal bovine serum (FBS), 1% penicillin/streptomycin and 2 mml-glutamine (Bio Industries), and cultured at 37°C, 5% $CO_2$, and passaged twice a week.

Specific RT-PCR process:

Total RNA extraction: the culture medium was discarded from the six-well plate, and rinsed with PBS buffer three times. Each well was added with 1mL of Trizol to lyse the cells. 200μL of chloroform was added, mixed thoroughly, and left at room temperature for 10 minutes. The mixture was centrifuged at 13000 rpm and 4°C for 15 minutes resulting in layered three-phase liquid, with RNA dissolved in the upper aqueous phase. The upper phase was pipetted and placed in a new enzyme-free 1.5 ml centrifuge tube, 500 μL of isopropanol was added and the solution was allowed to stand at room temperature for 10 minutes. After centrifugation at 13000 rpm and 4°C for 15 minutes, the RNA precipitate was obtained. The supernatant was discarded, and 1 mL of 75% (v/v) ethanol newly prepared with RNase-free water was added to each tube. The RNA precipitate at the bottom was gently resuspended, followed by centrifugation at 7500 rpm and 4°C for 10 minutes. The supernatant was discarded, and the liquid at the bottom was aspirated as thoroughly as possible. The RNA precipitate was air-dried at room temperature with the lid open. 50 μL of enzyme-free water was added to dissolve the precipitate. The purity and concentration of the RNA were measured using an ultra-micro UV-visible spectrophotometer.

cDNA reverse transcription: RNA was reverse transcribed into cDNA using the Ta Ka Ra Prime Script™ RT reagent Kit with gDNA Eraser. The genomic DNA (gDNA) was removed before the reverse transcription step to make the results more accurate and reliable. The total RNA reverse transcription reaction system was prepared on ice: 1μL Prime Script RT Enzyme MixI, 1μL RT Primer Mix, 4μL 5×Prime Script Buffer 2, 4μL RNase Free $dH_2O$. The prepared reaction mixture was incubated at 37°C for 15 minutes, and then placed at 85°C for 5 seconds to halt the reaction, and then stored at 4°C for later use.

RT-PCR operation: this detection method was the SYBR Green dye method, and no probe was required. Specifically, real-time PCR reaction was performed using cDNA from different samples as templates. The reaction solution was prepared on ice: 5μL SYBR Premix Dimer Eraser (2×), 0.3μL PCR Forward Primer (10 μM), 0.3μL PCR Reverse Primer (10 μM), 0.2μL ROX Reference DyeII(50×), 1μL cDNA template obtained in the previous step and 3.2μL dH2O. 10μL of sample was added to each well of the plate, and centrifuged (1000 rpm, 5min) to eliminate liquid sticking to the wall and bubbles in the reaction solution. ABI ViiA7 real-time fluorescence quantitative PCR instrument was used for real-time PCR reaction detection, the reaction program was 95°C, 30 sec (1 cycle) → 95°C, 5 sec; 55°C, 30 sec; 72°C, 30 sec (40 cycles) → 60°C-95°C, 2min (1 cycle). The experiment was repeated three times, and the average value was taken to obtain Ct value of each group, and the expression fold change between the experimental group and the control group was calculated. The control gene is GAPDH. The RT-PCR primers were as follows:

(1) STAT3 primer: forward: 5'- TGATCACCTTTGAGACCGAGG -3',
reverse: 5'- GATCACCACAACTGG CAA GG -3';
(2) α-syn primer: forward:5'- TGACGGGTGTGACAGCAGTAG -3',
reverse: 5'- CAGTGGCTGCTGCAATG -3';
(3) Bcl-2 primer: forward: 5'- AGGATT GTG GCCTTCTTTGAG -3',
reverse: 5'- AGACAGCCAGGAGAAATCAAAC -3';
(4) GAPDH primer: forward: 5'- TCAGGGG TTTCACATTTGGCA -3',
reverse: 5'- GGAGCGGAA AACCA -3'.

The expression level of each target gene was represented by the RQ value ($2^{-\Delta\Delta CT}$). The Formula was as follows:

$$\text{Fold Change} = 2^{-\Delta\Delta Ct}$$

Wherein,

$$\Delta\Delta Ct = \Delta Ct_{\text{experimental group}} - \Delta Ct_{\text{control group}},\ \Delta Ct = Ct_{\text{target gene}} - Ct_{\text{reference gene}}$$

Calculation method of gene silencing efficiency:

100% - gene expression level of experimental group/gene expression level of control group.

Result analysis: As shown in Figures 1-19, 1-20, 1-21 (where scr ASO is a random control sequence), the three ASO@MPPs can significantly interfere with their corresponding target genes, among which STAT3-ASO@MPP had an inhibition rate of 75% on the target gene STAT3; α-syn-ASO@MPP had an inhibition rate of 71% on the target gene α-syn; and Bcl-2-ASO@MPP had an inhibition rate of 66% on the target gene Bcl-2. The results suggest that the ASO@MPP can load any ASO for target gene intervention therapy, and play the role of ASO-loaded drugs, vaccines or other products.

**Example 3.7.2 Preparation and effect of ASO-loaded metal-chelated phospholipid complex nanoparticles (ASO@MPP) when the metal ion is $Al^{3+}$**

The difference between this example and Example 3.7.1 is that the metal ion $Fe^{3+}$ in Example 3.7.2 is replaced by $A1^{3+}$.

Result analysis: as shown in Figures 1-22, 1-23, and 1-24, the three ASO@MPPs can significantly interfere with their corresponding target genes, among which STAT3-ASO@MPP has an inhibition rate of 79% on the target gene STAT3; α-syn-ASO@MPP has an inhibition rate of 80% on the target gene α-syn; Bcl-2-ASO@MPP has an inhibition rate of 74% on the target gene Bcl-2. The results suggest that ASO@MPP can load any ASO for interventional treatment of target genes, and play the role of ASO-loaded drugs, vaccines or other products.

**Example 3.8 Preparation of drug (different types of nucleic acids)-loaded metal-chelated phospholipid complex nanoparticles and their effects**

**Example 3.8.1 Preparation of drug (different types of nucleic acids)-loaded metal-chelated phospholipid complex nanoparticles with $Fe^{3+}$ as metal ion and their effects**

The mRNA in Example 3 was replaced with double-stranded RNA (siRNA), single-stranded DNA (ASO), single-stranded RNA (mRNA), double-stranded DNA, and single-stranded DNA, respectively. The different types of nucleic acid sequences are: (1) the sequence of double-stranded RNA (Bcl-2-siRNA) shown in SEQ ID NO.4 (antisense strand) and SEQ ID No.21 (sense strand) (19bp), and its random control sequence shown in SEQ ID NO.5 (antisense strand) and SEQ ID No.22 (sense strand) (19bp); (2) the sequence of single-stranded DNA (STAT3-ASO) shown in SEQ ID NO.10 (17nt), and its random control sequence shown in SEQ ID NO.11 (18nt); (3) the sequence of single-stranded RNA (mRNA encoding wild-type SARS-COV-2 S protein) shown in SEQ ID NO.16 (3822nt); (4) the sequence of double-stranded DNA (dsDNA) shown in SEQ ID NO.17 (antisense strand) and SEQ ID NO.25 (sense strand) (22bp) (the 3' end of the sequence is labeled with fluorescent probe Cy3); (5) the sequence of single-stranded DNA (ssDNA) shown in SEQ ID NO.18 (22nt) (the 3' end of the sequence is labeled with fluorescent probe Cy3). The drug-loaded metal-chelated phospholipid complex nanoparticles (Bcl-2-siRNA@MPP, STAT3-ASO@MPP, S-mRNA@MPP, dsDNA@MPP, ssDNA@MPP) containing the above-mentioned different types of nucleic acids were prepared respectively according to the method of Example 3. The rest of preparation process of the drug-lipid particles was the same as that of Example 3.

The sequences of double-stranded DNA are as follows:

Antisense: 5'-TAGCTTATCAGACTGATGTTGA-3'(SEQ ID No.17),
Sence: 5'-TCAACATCAGTCTGATAAGCTA-3'(SEQ ID No.25)(22bp).

The sequence of SEQ ID No.18 (the sequence of single-stranded DNA) is as follows:
5'-TCAACATCAGTCTGATAAGCTA-3'(22nt).

U251 cells were seeded in a 12-well plate at a density of $1\times10^5$ cells per well for about 24 hours. Each well of cells was incubated with siRNA@MPP (wherein the concentration of siRNA was 2μg/mL) or ASO@MPP (wherein the concentration of ASO was 2μg/mL) for 72 hours, the cells were collected, the total RNA of the cells was extracted, and the mRNA expression of the target genes (Bcl-2, STAT3) was detected by RT-PCR technology, and the capability of siRNA@MPP or ASO@MPP to silence the target genes in the cells was calculated. The results are shown in Figures 1-13 in Example 3.6 and Figures 1-19 in Example 3.7.

S-mRNA@MPP was incubated with 293T cells at a concentration of 2 μg/mL (the concentration of contained mRNA), and the control group was incubated with MPP. After 24 hours, the supernatant was centrifuged and stored at -20°C for later use; the cell pellet was resuspended in 100 μL PBS buffer solution, frozen and thawed twice, ultrasonicated for 10 min, and then centrifugated to obtain the supernatant. The expression levels of S protein in both the cell supernatant and the cell lysate were detected using a commercially available SARS-COV-2 S protein ELISA detection kit. The results

are shown in Figures 1-25.

A549 lung cancer cells were incubated with ds-DNA@MPP at a concentration of 100 nM (the concentration of DNA contained) for 2 hours, and then the rest of drug-lipid particles were removed. The cells were washed twice with PBS, and the cell nuclei were stained with Hochest33342 dye for 3 minutes, and then the dye was removed. The cells were washed twice with PBS, and the cells were observed using a high-content imaging system, and the efficiency of drug-lipid particle transfection of DNA was calculated. The results are shown in Figures 1-26.

HT22 mouse hippocampal neurons were incubated with ss-DNA@MPP at a concentration of 200 nM (the concentration of DNA contained) for 2 hours, and then the rest of drug-lipid particles were removed. The cells were washed twice with PBS, observed using a high-content imaging system, and the efficiency of drug-lipid particle transfection of DNA was calculated. The results are shown in Figure 1-26.

The culture method of human glioblastoma U251 cells is the same as in Example 3.6.

The culture method of 293T cells is the same as in Example 3.

The culture method of HT22 mouse hippocampal neurons: neurons were cultured in DMEM medium containing 10% FBS and 1% penicillin-streptomycin at 37°C and 5% $CO_2$.

The RT-PCR method is the same as in Example 3.6.

ELISA detection of S protein expression level: the "anti-RBD antibody working solution" in the ELISA detection of RBD in Example 3.5 was replaced with the "anti-S protein antibody working solution", and the rest of steps are the same as in Example 3.5.

The calculation method of gene silencing efficiency is the same as in Example 3.6.

The calculation method of transfection efficiency: 3-5 fields of view were randomly select using a high-content imaging system to obtain the cell morphology under ordinary light source, the fluorescence signal when the excitation/emission light is 550nm/570nm (excitation light of fluorescent dye Cy3 for labeled DNA) in the same field of view, and the fluorescence signal when the excitation/emission light is 352nm/461nm (excitation light of fluorescent dye Hoechst 33342 for labeling cell nuclei), and the ratio of the number of cells with Cy3 fluorescence signal in the randomly selected field of view to the number of cells with Hochest 33342 fluorescence signal in the same field of view was calculated, which is the transfection efficiency.

Result analysis: as shown in Figure 1-13 of Example 3.6.1, the inhibition rate of the drug (double-stranded RNA)-loaded metal-chelated phospholipid complex nanoparticles (Bcl-2-siRNA@MPP) on the target gene Bcl-2 reached 76%; as shown in Figure 1-19 of Example 3.7.1, the inhibition rate of the drug (single-stranded DNA)-loaded metal-chelated phospholipid complex nanoparticles (STAT3-ASO@MPP) on the target gene STAT3 reached 75%; as shown in Figure 1-25, the S protein expression level in the supernatant of 293T cells transfected with drug (single-stranded RNA)-loaded metal-chelated phospholipid complex nanoparticles (S-mRNA@MPP) was 161.3 ng/mL, while the S protein content in the supernatant of 293T cells transfected with empty vector MPP was 0; the efficiency of transfecting double-stranded DNA into cells by drug (double-stranded DNA)-loaded metal-chelated phospholipid complex nanoparticles (dsDNA@MPP) was 100 % (Figure 1-26); the efficiency of transfecting single-stranded DNA into cells by drug (single-stranded DNA)-loaded metal-chelated phospholipid complex nanoparticles (ssDNA@MPP) was 100% (Figure 1-26). The results suggest that the drug-loaded metal-chelated phospholipid complex nanoparticles can encapsulate any nucleic acid (double-stranded RNA, single-stranded RNA, double-stranded DNA, single-stranded DNA) and realize its function, wherein the length of the nucleic acid ranges from 16-3822 nt.

### Example 3.8.2 Preparation of drug (different types of nucleic acid)-loaded metal-chelated phospholipid complex nanoparticles with $Al^{3+}$ as the metal ion and their effects

The difference between this example and Example 3.8.1 is that the metal ion $Fe^{3+}$ in Example 3.8.1 was replaced by $A1^{3+}$.

Result analysis: as shown in Figure 1-16 of Example 3.6.2, the inhibition rate of the drug (double-stranded RNA)-loaded metal-chelated phospholipid complex nanoparticles (Bcl-2-siRNA@MPP) on the target gene Bcl-2 reached 81%; as shown in Figure 1-22 of Example 3.7.2, the inhibition rate of the drug (single-stranded DNA)-loaded metal-chelated phospholipid complex nanoparticles (STAT3-ASO@MPP) on the target gene STAT3 reached 79%; as shown in Figure 1-27, the S protein expression level in the supernatant of 293T cells transfected with drug (single-stranded RNA)-loaded metal-chelated phospholipid complex nanoparticles (S-mRNA@MPP) was 178.7 ng/mL, while the S protein content in the supernatant of 293T cells transfected with empty vector MPP was 0; the efficiency of transfecting double-stranded DNA into cells by drug (double-stranded DNA)-loaded metal-chelated phospholipid complex nanoparticles (dsDNA@MPP) was 100 % (Figure 1-28); the efficiency of transfecting single-stranded DNA into cells by drug (single-stranded DNA)-loaded metal-chelated phospholipid complex nanoparticles (ssDNA@MPP) was 100% (Figure 1-28). The results suggest that drug-loaded metal-chelated phospholipid complex nanoparticles can encapsulate any nucleic acid (double-stranded RNA, single-stranded RNA, double-stranded DNA, single-stranded DNA) and realize its function, where the length of the nucleic acid ranges from 16-3822 nt.

**Embodiment 2. Performance characterization of drug-loaded metal-chelated phospholipid complex nanoparticles**

**Example 4 Synthesis characterization of metal-chelated phospholipid complex**

**Example 4.1 Synthesis characterization of metal-chelated phospholipid complex with $Fe^{3+}$ as metal ion**

The connection of DSPC and curcumin is characterized by differential scanning calorimetry. The measurement conditions were as follows: 3-5 mg substance to be detected was weighed, the heating rate was 10 °C/min, and the temperature range was 30~300 °C. Curcumin, DSPC and phospholipid complex were scanned, and curves were drawn according to the obtained data, as shown in Figure 2-1. The curves show that curcumin had a lattice structure and an obvious melting peak appeared at 185°C; DSPC is a mixture, and there were many depressions on the curve, which might be the heat changes of different components under different temperatures; in the phospholipid complex, there was no peak near the curcumin melting peak, but basically a straight line, indicating that curcumin and DSPC were combined and existed in an amorphous form, which proved that the phospholipid complex was successfully prepared.

The connection of the phospholipid complex and $Fe^{3+}$ is characterized by spectrophotometry: as shown in Figure 2-2, after the phospholipid complex (CUR-HSPC) binds $Fe^{3+}$, its maximum absorption wavelength shifted from 420nm to 375nm, and the conjugated structure of the phospholipid complex changed, proving that curcumin was successfully complexed with $Fe^{3+}$.

**Example 4.2 Synthesis and characterization of metal-chelated phospholipid complex with $Al^{3+}$ as metal ion**

The present example differs from example 4.1 in that the phospholipid complex and $Al^{3+}$ are connected and characterized by spectrophotometry: as shown in Figure 2-3, after the phospholipid complex (CUR-HSPC) binds $Al^{3+}$, its maximum absorption wavelength shifted from 420nm to 433nm, and the conjugated structure of the phospholipid complex changed, proving that curcumin was successfully complexed with $Al^{3+}$.

**Example 5. Characterization of $Fe^{3+}$ detaching from metal-chelated phospholipid complex under low pH conditions when metal ion is $Fe^{3+}$**

The phospholipid complex in the metal-chelated phospholipid complex binds $Fe^{3+}$ through coordination bonds. Under the low pH conditions of lysosomes, the coordination bonds between the phospholiplid complex and $Fe^{3+}$ will be protonated (absorb hydrogen ions) and broken. To prove that the $Fe^{3+}$ in the metal-chelated phospholipid complex indeed detached from the lipid complex through the above mechanism, we designed the following experiment: observe the color of the metal-chelated phospholipid complex under physiological pH (pH = 7.4) and the lysosome low pH (pH = 5.0) conditions. As shown in Figure 2-4, the metal-chelated phospholipid complex changed from brown-red to bright yellow under the lysosome low pH (pH = 5.0) condition, indicating that $Fe^{3+}$ has detached from the complex. The results suggest that $Fe^{3+}$ can detach from the metal-chelated phospholipid complex under the lysosome low pH condition.

The principle of $Fe^{3+}$ detaching from the metal-chelated phospholipid complex under low pH conditions is that the coordination bond between curcumin and $Fe^{3+}$ is protonated under low pH conditions (pH=5.0), that is, curcumin will bind a large number of protons ($H^+$) from the solution, resulting in the rupture of the coordination bond between $Fe^{3+}$ and curcumin, thereby separating $Fe^{3+}$ from curcumin, and finally separating $Fe^{3+}$ from the metal-chelated phospholipid complex (Figure 2-4).

**Example 6. Elemental analysis of MPP in drug-loaded metal-chelated phospholipid complex nanoparticles**

**Example 6.1. Elemental analysis of MPP in drug-loaded metal-chelated phospholipid complex nanoparticles with $Fe^{3+}$ as metal ion**

The drug-loaded metal-chelated phospholipid complex nanoparticles siRNA@MPP were prepared according to Example 3 except that the mRNA in Example 3 was replaced with sulfhydryl modified siRNA. Elemental analysis was carried out by transmission electron microscopy. The results are shown in Figure 2-5. C, N, O and P were common elements. The analysis diagram of Fe element shows that $Fe^{3+}$ was uniformly distributed on the lipid nanoparticles, and the analysis diagram of S element could specifically represent the position of siRNA due to the modification of sulfhydryl groups on siRNA. It can be seen from the diagram that siRNA was well complexed near $Fe^{3+}$. It was proved that the drug-lipid nanoparticles successfully encapsulated siRNA.

**Example 6.2. E**lectron microscopy **analysis of MPP in drug-loaded metal-chelated phospholipid complex nanoparticles with $Al^{3+}$ as metal ion**

The drug-loaded metal-chelated phospholipid complex nanoparticles MPP were prepared according to Example 3. Morphological analysis was carried out by transmission electron microscopy. The results are shown in Figure 2-6. The form of MPP in drug-loaded metal-chelated phospholipid complex nanoparticles was standard spherical with uniform particle size and the size is about 100 nm.

**Example 7. Efficiency of metal-chelated phospholipid complex nanoparticles MPP encapsulating nucleic acids (siRNA and mRNA) with $Fe^{3+}$ or $Al^{3+}$ as metal ion and its comparison with LNP**

The mRNA in Example 3 was replaced with siRNA targeting the Bcl-2 gene (SEQ ID NO.4, 21 bp) and mRNA encoding the receptor binding domain (RBD) of the SARS-COV-2 S1 subunit (SEQ ID NO.2, 669 nt), respectively, to prepare drug-loaded metal-chelated phospholipid complex nanoparticles siRNA@MPP and mRNA@MPP encapsulating nucleic acid, respectively. The rest of preparation process of the drug-loaded metal-chelated phospholipid complex nanoparticles was the same as that in Example 3.

siRNA@LNP and mRNA@LNP were prepared according to the same drug loading amount as siRNA@MPP in Example 3.6 and mRNA@MPP in Example 3.5. The specific method was as follows: an organic phase solution was prepared according to the Onpattro lipid nanoparticle Formula, that is, by dissolving ionizable lipid ALC0315, DSPE-PEG2000, DSPC and cholesterol in ethanol at a molar ratio of 50%:1.5%:10%:38.5%. Bcl-siRNA or RBD-mRNA was added to the aqueous phase (0.1M acetic acid-sodium acetate buffer solution, pH=4.0). Among those, the ratio of amino lipids to phosphate-containing nucleotides (N/P) was 6:1. Meanwhile the nucleic acid loading amount was ensured to be the same as that of the above-mentioned siRNA@MPP and mRNA@MPP. The aqueous phase and the organic phase were quickly mixed at a flow rate of 14mL/min at a volume ratio of 3:1. After mixing, the mixture was diluted tenfold with enzyme-free PBS buffer solution, and the mixture was concentrated to one-tenth using a 100kDa ultrafiltration tube. After repeated dilution and concentration operations for 3 times, the ethanol concentration in the mixture was reduced to below 0.0005%, and the pH value of the solution was increased to the normal pH value of PBS buffer solution (7.2-7.4), and siRNA@LNP and mRNA@LNP were obtained respectively.

Agarose gel electrophoresis was used to detect the nucleic acids (siRNA and mRNA) encapsulation efficiency of siRNA@MPP, mRNA@MPP, siRNA@LNP and mRNA@LNP respectively. The encapsulation efficiency was determined as follows: the amount of nucleic acids (siRNA and mRNA) in each group of lipid nanoparticles was set at 10 $\mu$g/mL, the mass ratio of lipid to nucleic acid was 40:1. The nucleic acids were dissolved in PBS buffer solution as the positive control group, and the negative control was a PBS buffer solution without nucleic acids. The concentration of agarose gel was 1.5%. At this time, the void in the gel only allowed free nucleic acids to pass through, but not lipid nanoparticles. When the free nucleic acid bands were clearly distinguishable, the electrophoresis was stopped to prevent nucleic acid degradation due to long electrophoresis time. The Image J software was employed to quantify the grayscale values of free nucleic acids in various groups. The positive control group was set as 100%, and the ratio of free nucleic acids in each group to the positive control was the relative amount of free nucleic acids. The encapsulation efficiency of each group was (100-relative amount of free nucleic acids) %.

Result analysis: as shown in Figures 2-7, the encapsulation efficiency of MPP ($Fe^{3+}$) for siRNA and mRNA was 85.86% and 87.11%, respectively; the encapsulation efficiency of MPP ($Al^{3+}$) for siRNA and mRNA was 89.73% and 92.23%, respectively; the encapsulation efficiency of LNP for siRNA and mRNA was 84.98% and 79.12%, respectively. The results suggest that there is no significant difference in the nucleic acid encapsulation efficiency between MPP and LNP.

**Example 8. Capability of nucleic acids of metal-chelated phospholipid complex nanoparticles MPP with $Fe^{3+}$ or $Al^{3+}$ as metal ion to escape from lysosomes and its comparison with LNP**

The Bcl-2-siRNA (SEQ ID NO.4) in Example 3.6 was replaced with Cy5-labeled Bcl-2-siRNA to prepare Cy5-siRNA@MPP (the concentration of siRNA contained was 100 nM); the Bcl-2-siRNA (SEQ ID NO.4) in Example 7 was replaced with Cy5-labeled Bcl-2-siRNA to prepare Cy5-siRNA@LNP (the concentration of siRNA contained was 100 nM); the eGFP-mRNA (SEQ ID NO.1) in Example 3.5 was replaced with Cy5-labeled eGFP-mRNA to prepare Cy5-mRNA@MPP (the concentration of mRNA contained was 2 $\mu$g/mL); the RBD-mRNA in Example 7 was replaced with Cy5-labeled RBD-mRNA to prepare Cy5-mRNA@LNP (the concentration of mRNA contained was 2 $\mu$g/mL). After incubating them with A549 cells and the cell lysosome probe Lysotracker Green for 3 hours, the overlap of the Cy5 fluorescence signal (red) and the Lysotracker Green fluorescence signal (green) were observed using a high-content imaging system to assess the capability of the drug-lipid particles to promote nucleic acid escape from lysosomes.

Criteria for determining the capability of drug-loaded metal-chelated phospholipid complex nanoparticles to

promote nucleic acids to escape from lysosomes: after incubating cells with drug-lipid nanoparticles for 3 hours, the overlap of the Cy5 fluorescence signal (red) and the Lysotracker Green fluorescence signal (green) were observed using a high-content imaging system, and the overlap rate of the red fluorescence signal and the green fluorescence signal was statistically analyzed using image J software. After the drug-loaded metal-chelated phospholipid complex nanoparticles were incubated with cells for 3 hours, the overlap rate of the red fluorescence signal and the green fluorescence signal was less than 50%, indicating that the nucleic acids could escape from the cell lysosomes quickly. The lipid nanoparticles had a good capability to promote nucleic acids escape from lysosomes.

Result analysis: as shown in Figure 2-8, when Cy5-siRNA@MPP ($Fe^{3+}$) and Cy5-mRNA@MPP ($Fe^{3+}$) were incubated in A549 cells for 3 hours, the overlap rates of the red fluorescence signal and the green fluorescence signal were 36.05% and 43.07%, respectively, that is, the rates of escape from lysosomes were 63.95% and 56.93%, respectively; when Cy5-siRNA@MPP ($Al^{3+}$) and Cy5-mRNA @MPP ($Al^{3+}$) were incubated in A549 cells for 3 hours, the overlap rates of red fluorescence signal and green fluorescence signal were 32.39% and 40.17%, respectively, that is, the rates of escape from lysosomes were 67.61% and 59.83%, respectively; while when Cy5-siRNA@LNP and Cy5-mRNA@LNP were incubated in A549 cells for 3 hours, the overlap rates of red fluorescence signal and green fluorescence signal were 76.89% and 86.87%, respectively, that is, the rates of escape from lysosomes were 23.11% and 13.13%, respectively. This indicates that the drug-lipid nanoparticle MPP has a good capability to promote nucleic acids escape from lysosomes, and the capability of MPP to promote escaping from lysosomes is significantly stronger than that of LNP.

### Example 9. capability of metal-chelated phospholipid complex nanoparticles MPP with $Fe^{3+}$ or $Al^{3+}$ as metal ion to promote nucleic acid expression and its comparison with LNP

The RBD-mRNA (SEQ ID NO.2) in Example 7 was replaced with mRNA encoding the fluorescent protein eGFP, and the rest of the preparation method was the same as that in Example 7 to obtain eGFP-mRNA@LNP.

The eGFP-mRNA@MPP prepared in Example 3 and the above-mentioned eGFP-mRNA@LNP (the concentration of mRNA contained was 2 μg/mL) were incubated in 293T cells respectively, and the control group was incubated with MPP or LNP. After 48 hours, the cell suspensions were collected and the percentages of eGFP-positive cells were detected by flow cytometry.

The method for analyzing the eGFP-positive cell rate by flow cytometry is as described in Example 3.

Result analysis: as shown in Figures 2-9, the percentages of eGFP-positive cells after MPP ($Fe^{3+}$), MPP ($Al^{3+}$) and LNP treatment of 293T cells were 97.2%, 98.1% and 63.03%, respectively. The results suggest that MPP is better than LNP in promoting nucleic acid expression. The possible reason is that as described in Example 8, MPP has a stronger capability than LNP in promoting nucleic acids to escape from lysosomes, so more nucleic acids loaded by MPP can be effectively released into the cytoplasm and translated into proteins.

### Example 10. capability of drug-loaded metal-chelated phospholipid complex nanoparticles MPP to promote humoral immunity and cellular immunity when metal ion is $Fe^{3+}$ or $Al^{3+}$ and their comparison with LNP

The RBD-mRNA@MPP in Example 3.5 and the RBD-mRNA@LNP in Example 7 were incubated with 293T cells at a concentration of 2μg/mL (the concentration of mRNA contained), and the control group was incubated with MPP. After 24 hours, the supernatant was centrifuged and frozen at -20°C for later use; the cell pellet was resuspended in 100μL PBS buffer solution, frozen and thawed twice, and ultrasonicated for 10 minutes before centrifugation to obtain the supernatant. The expression levels of RBD protein in both the cell supernatant and the cell lysate were detected using a commercially available SARS-COV-2 antigen RBD ELISA detection kit. The results are shown in Figures 2-10.

The method for detecting the expression level of RBD by ELISA is as described in Example 3.5.

The experimental animals were randomly divided into 3 groups (experimental group and control group), with 5 animals in each group. The animal model was BALB/c mice. Each mouse was given the first intramuscular administration on the first day and the second intramuscular administration on the 14th day. The experimental groups were injected with RBD-mRNA@MPP ($Fe^{3+}$), RBD-mRNA@MPP($Al^{3+}$) or RBD-mRNA@LNP, respectively, and the control group was injected with MPP and LNP without mRNA loaded. The dose of each administration was 100 μL, wherein the RBD-mRNA@MPP ($Fe^{3+}$), RBD-mRNA@MPP($Al^{3+}$) and RBD-mRNA@LNP preparations in the experimental group each contained 30 mg of mRNA. The blood of mice was collected on the 28th day after the first administration, and the serum was separated and diluted stepwise. The titer of the total IgG antibody against the RBD of S1 subunit of the SARS-COV-2 produced in mice was detected by a commercially available ELISA kit, and the results are shown in Figures 2-11.

The method for detecting the titer of the total IgG antibody against the RBD of S1 subunit of the SARS-COV-2 by ELISA is as described in Example 3.5.

On the 28th day after administration of RBD-mRNA@MPP ($Fe^{3+}$), RBD-mRNA@MPP ($Al^{3+}$) and RBD-mRNA@LNP, spleens of normal mice were collected and prepared into a single cell suspension under sterile conditions. 100,000 spleen cells/well were plated in a cell well plate, and RBD protein with a final concentration of 10 mg/mL was

added and cultured for 48 hours. The supernatant was removed by centrifugation, and the expression levels of IFN-γ, IL-2 and IL-4 were determined by ELISA kit. The results are shown in Figures 2-12.

The method for detecting the expression levels of IFN-γ, IL-2 and IL-4 by ELISA is as described in Example 3.5. Result analysis: as shown in Figures 2-10, RBD-mRNA@MPP($Fe^{3+}$), RBD-mRNA@MPP($Al^{3+}$) and RBD-mRNA@LNP can all induce 293T cells to express a certain amount of RBD, but the capability of RBD-mRNA@MPP($Al^{3+}$) to induce cells to express RBD is significantly stronger than that of RBD-mRNA@MPP($Fe^{3+}$); the capability of RBD-mRNA@MPP($Fe^{3+}$) to induce cell expression of RBD is significantly stronger than that of RBD-mRNA@LNP. The expression level of RBD in the cell supernatant of the RBD-mRNA@MPP ($Fe^{3+}$) treatment group was 205 ng/mL, the expression level of RBD in the cell supernatant of the RBD-mRNA@MPP ($Al^{3+}$) treatment group was 230 ng/mL, and the expression level of RBD in the cell supernatant of the RBD-mRNA@LNP treatment group was 115.7 ng/mL. As shown in Figure 2-11, RBD-mRNA@MPP effectively induced humoral immunity in mice and produced high levels of antigen-specific binding antibodies. The capability of RBD-mRNA@MPP($Al^{3+}$) to induce humoral immunity in mice was clearly superior to that of RBD-mRNA@MPP($Fe^{3+}$). The capability of RBD-mRNA@MPP($Fe^{3+}$) to induce humoral immunity in mice was clearly superior to that of RBD-mRNA@LNP. The IgG antibody titer in mice treated with RBD-mRNA@MPP($Fe^{3+}$) reached 117233.8, the IgG antibody titer in mice treated with RBD-mRNA@MPP($Al^{3+}$) reached 133116; while the IgG antibody titer in mice treated with RBD-mRNA@LNP reached only 67476. As shown in Figure 2-12, RBD-mRNA@MPP($Al^{3+}$) can effectively induce cellular immunity in mice, that is, activate immune cells and produce a large number of cytokines, and the capability of mRNA@MPP($Al^{3+}$) to induce cellular immunity in mice was clearly better than that of RBD-mRNA@MPP($Fe^{3+}$), and the capability of RBD-mRNA@MPP($Fe^{3+}$) to induce cellular immunity in mice was clearly better than that of RBD-mRNA@LNP. RBD-mRNA@MPP($Fe^{3+}$) made the expression levels of cytokines IFN-γ, IL-2, and IL-4 reach 256.8 pg/mL, 207.6 pg/mL, and 61.8 pg/mL, respectively; RBD-mRNA@MPP($Al^{3+}$) made the expression levels of cytokines IFN-γ, IL-2, and IL-4 reach 298 pg/mL, 249 pg/mL, and 74.6 pg/mL; while RBD-mRNA@LNP made the expression of cytokines IFN-γ, IL-2, and IL-4 only 104.2 pg/mL, 79.2 pg/mL, and 27 pg/mL. The results suggest that the capability of mRNA@MPP($Al^{3+}$) to deliver any mRNA and realize its function is significantly better than RBD-mRNA@MPP($Fe^{3+}$), and the capability of RBD-mRNA@MPP($Fe^{3+}$) to induce cellular immunity in mice is clearly better than RBD-mRNA@LNP. RBD-mRNA@MPP can more effectively promote the expression of target proteins by cells and can more effectively activate humoral immunity and cellular immunity in vivo. Therefore, the drug (mRNA)-lipid particles are significantly better than the existing technology LNP in terms of the effect of mRNA-loaded drugs, vaccines or other products. The possible reasons are: 1) compared with LNP, MPP has a stronger capability to promote nucleic acids escape from lysosomes; 2) compared with LNP, MPP has a stronger capability to promote the expression of nucleic acids into proteins (antigens); 3) compared with LNP, after the curcumin in MPP detached with DSPC, as an immune adjuvant (also known as immunomodulator), it can activate humoral immunity and cellular immunity to enhance the effect of MPP delivery of mRNA vaccine, and can also inhibit the immune factor storm to inhibit excessive and harmful immune response to the body.

## Example 11. in vivo safety evaluation of metal-chelated phospholipid complex nanoparticles (MPP) with $Fe^{3+}$ or $Al^{3+}$ as metal ion

Using SD rats as the research subjects, a 20-day subchronic toxicity study of MPP was conducted, and a 20-day recovery period was established. The specific experimental methods are as follows:

56 SPF SD rats (220 ± 20g), half male and half female, were raised in an environment with a temperature of 25°C, a humidity of 45% - 55%, and a light exposure of 12h. After 3-5 days of adaptive feeding, they were randomly divided into groups according to gender: 32 in the experimental group and 24 in the recovery group. The blank control group (Control) consisted of 14 rats (including 8 rats in the experimental group and 6 rats in the recovery group), half of which were male and half were female; the low-dose MPP group (8 mg/kg) consisted of 14 rats (including 8 in the experimental group and 6 in the recovery group), half of which were male and half were female; the medium-dose MPP group (16 mg/kg) consisted of 14 rats (including 8 in the experimental group and 6 in the recovery group), half of which were male and half were female; the high-dose group (32 mg/kg) consisted of 14 rats (including 8 in the experimental group and 6 in the recovery group), half of which were male and half were female. The experimental group (32 rats in total) was dissected after the end of the drug administration, and the recovery group (24 rats in total) continued to be fed normally for 20 days after the end of the drug administration, before being dissected.

Administration method: The experimental animals were injected with drugs through the tail vein, once every 2 days, for a total of 20 days, and the body weight of SD rats was recorded once a week. The prepared MPPs were dissolved in DPBS, the control group was injected with an equal amount of DPBS, and the low-dose MPP group, medium-dose MPP group, and high-dose MPP group were injected with 8 mg/kg, 16 mg/kg, and 32 mg/kg of MPP, respectively.

The basis for setting the above-mentioned MPP dosage: when encapsulating 200 μg/kg mRNA (the actual required amount for mRNA animal experiments), the required amount of empty vector MPP is 8 mg/kg. In order to fully prove the safety of MPP, 1, 2, and 4 times the actual required amount of MPP animal experiments, namely 8 mg/kg, 16 mg/kg, and 32 mg/kg were selected.

Detection methods of general indicators: after each administration, the general status of the animals in each group was observed, including survival status, diet status, appearance characteristics, behavioral activities, body weight, and whether there was a local reaction to administration. During the autopsy, a gross autopsy was performed, including timely weighing the wet weight of major organs, such as brain, heart, liver, spleen, lung, and kidney, calculating the organ/body weight ratios, and recording the pathological changes of each organ. The organ/body weight ratio = wet weight of rat organ / rat body weight × 100%.

Obtaining and storing whole blood and serum from SD rats: after 20 days of administration and a 20-day recovery period, rats were dissected and blood was collected from the abdominal aorta, that is, SD rats were anesthetized with isoflurane and fixed on a dissection board, the abdomen was disinfected with 75% ethanol, the abdomen of the rat was cut open with sterile ophthalmic scissors, and the internal organs were gently opened with cotton balls to expose the abdominal aorta. Whole blood was collected using a 500μL negative pressure EDTAK2 anticoagulation blood collection tube and stored at 4°C for complete blood count. Whole blood was collected using a 5mL negative pressure ordinary blood collection tube and allowed to stand at room temperature for 30 minutes, centrifuged at 4°C, 1500rpm for 15 minutes, and the supernatant was taken in a 1.5mL centrifuge tube and stored at -20°C for the detection of blood biochemical indicators and immunology-related indicators.

Method for complete blood count: complete blood count indicators include: white blood cell count, lymphocyte count, monocyte count, neutrophil count, lymphocyte percentage, monocyte percentage, neutrophil percentage, red blood cell count, hemoglobin, hematocrit, mean corpuscular volume, mean corpuscular hemoglobin content, mean corpuscular hemoglobin concentration, coefficient of variation of red blood cell distribution width, platelet count, mean platelet volume, platelet distribution width, and platelet hematocrit. The whole blood sample was gently inverted to mix, and a small amount of whole blood was taken and the results were automatically analyzed using a fully automatic blood cell analyzer.

Method for detecting blood biochemical indicators: blood biochemical indicators include inorganic ions ($Fe^{2+}$, $Na^+$, $K^+$, $Cl^-$, $Ca^{2+}$), liver function indicators (ALT, AST, γ-GT, T-BIL, D-BIL, ALP, ALB), renal function indicators (BUN, UA, CR), cardiac function indicators (LDH, CK), glucose metabolism indicators (GSP, GLU, INS), lipid metabolism indicators (CHO, TG, LDL-C, HDL-C). The serum sample was thawed and centrifuged at 3000 rpm for 15 minutes. The supernatant was collected and prepared for use. The automatic biochemical analyzer was then configured with the appropriate parameters, the prepared working solution was added, followed by the addition of the serum to be tested. The automatic biochemical analyzer subsequently performed the measurements automatically.

Detection method of immunology-related indicators: immunology-related indicators include thyroid function indicators (TT3, TT4, TSH), cytokines (IL-1, IL-2, IL-4, IFN-γ, IFN-α, TNF-α), immunoglobulins (IgG, IgA, IgM), and serum complement (C3, CH50). The above indicators were detected by ELISA method.

Pathological examination method of main organs of SD rats: at the end of the administration period and the end of the recovery period, the rats in each group were anesthetized, and the main organs of the rats, including the whole brain, heart, liver, spleen, lung, and kidney, were removed by ophthalmic scissors, gently rinsed with 0.9% saline, fixed in 4% paraformaldehyde fixative, routinely embedded by paraffin, H&E stained, and the histopathological changes of various organs of the rats in the control group and the experimental group were observed under an optical microscope.

Result analysis: as shown in Table 2-1, at the end of the administration period and the recovery period, compared with the control group, the rats in the low, medium and high dose MPP($Fe^{3+}$) or MPP($Al^{3+}$) groups survived well, had normal diet, normal appearance and behavior, and no obvious adverse reactions were observed after administration; compared with the control group, there was no significant difference in the weight gain of male SD rats and female SD rats in the low, medium and high dose MPP groups; compared with the control group, there was no significant difference in the organ/body weight ratios of the low, medium and high dose MPP groups.

At the end of the administration period and the end of the recovery period, compared with the control group, the complete blood count indicators (white blood cell count, lymphocyte count, monocyte count, neutrophil count, lymphocyte percentage, monocyte percentage, neutrophil percentage, red blood cell count, hemoglobin, hematocrit, mean corpuscular volume, mean corpuscular hemoglobin content, mean corpuscular hemoglobin concentration, coefficient of variation of red blood cell distribution width, platelet count, mean platelet volume, platelet distribution width, platelet hematocrit) in the low, medium and high dose MPP($Fe^{3+}$) or MPP($Al^{3+}$) groups showed no abnormalities; compared with the control group, the blood biochemical indicators in the low, medium and high dose MPP groups, including inorganic ions ($Fe^{2+}$, $Na^+$, $K^+$, $Cl^-$, $Ca^{2+}$), liver function indicators (ALT, AST, γ-GT, T-BIL, D-BIL, ALP, ALB), renal function indicators (BUN, UA, CR), cardiac function indicators (LDH, CK), glucose metabolism indicators (GSP, GLU, INS), lipid metabolism indicators (CHO, TG, LDL-C, HDL-C), all showed no abnormalities; compared with the control group, the immunological related indicators of the low, medium and high dose MPP groups, including thyroid function indicators (TT3, TT4, TSH), cytokines (IL-1, IL-2, IL-4, IFN-γ, IFN-α, TNF-α), immunoglobulins (IgG, IgA, IgM), serum complement (C3, CH50), showed no abnormalities.

At the end of the administration period and the end of the recovery period, compared with the control group, the brain tissue structure of rats in the low, medium and high dose MPP($Fe^{3+}$) or MPP($Al^{3+}$) groups was intact, the tissue staining was normal, the cell morphology was intact, there was no nuclear pyconsis and inflammatory cell infiltration; the

myocardial tissue structure was intact, the myocardial cells were arranged neatly, continuously and tightly, the cell nuclei were clearly visible, and no obvious cell congestion, edema, or necrosis was observed; the hepatocyte morphology was normal, there was no inflammatory cell aggregation and necrosis; the spleen structure was normal, and the boundaries between red and white pulp were clear; the lung tissue structure was intact, the alveoli were of the same size, and there was no obvious inflammatory cell aggregation and infiltration; the kidney structure was normal.

The above results indicate that no obvious chronic toxicity was found in long-term and large-scale injections of MPP ($Fe^{3+}$ or $Al^{3+}$) into SD rats, indicating that the safety of MPP($Fe^{3+}$) or MPP($Al^{3+}$) is relatively high.

Table 2-1 In vivo safety evaluation of MPP

| | Low, medium and high doses of MPP ($Fe^{3+}$) | Low, medium and high doses of MPP ($Al^{3+}$) |
|---|---|---|
| General status (survival status, diet status, appearance characteristics, behavioral activities, body weight, and whether there was a local reaction to administration) | No abnormality | No abnormality |
| Complete blood count indicators (white blood cell count, lymphocyte count, monocyte count, neutrophil count, lymphocyte percentage, monocyte percentage, neutrophil percentage, red blood cell count, hemoglobin, hematocrit, mean corpuscular volume, mean corpuscular hemoglobin content, mean corpuscular hemoglobin concentration, coefficient of variation of red blood cell distribution width, platelet count, mean platelet volume, platelet distribution width, platelet hematocrit) | No abnormality | No abnormality |
| Inorganic ions ($Fe^{2+}$, $Na^+$, $K^+$, $Cl^-$, $Ca^{2+}$) | No abnormality | No abnormality |
| Liver function indicators (ALT, AST, γ-GT, T-BIL, D-BIL, ALP, ALB) | No abnormality | No abnormality |
| Kidney function indicators (BUN, UA, CR) | No abnormality | No abnormality |
| Cardiac function indicators (LDH, CK) | No abnormality | No abnormality |
| Glucose metabolism indicators (GSP, GLU, INS) | No abnormality | No abnormality |
| Lipid metabolism indicators (CHO, TG, LDL-C, HDL-C) | No abnormality | No abnormality |
| Thyroid function indicators (TT3, TT4, TSH) | No abnormality | No abnormality |
| Cytokines (IL-1, IL-2, IL-4, IFN-γ, IFN-α, TNF-α) | No abnormality | No abnormality |
| Immunoglobulin (IgG, IgA, IgM) | No abnormality | No abnormality |
| Serum complement (C3, CH50) | No abnormality | No abnormality |
| Organ coefficient (heart, liver, spleen, lung, kidney, brain) | No abnormality | No abnormality |

(continued)

| | Low, medium and high doses of MPP ($Fe^{3+}$) | Low, medium and high doses of MPP ($Al^{3+}$) |
|---|---|---|
| Organ tissue structure (heart, liver, spleen, lung, kidney, brain) | No abnormality | No abnormality |
| Note: ALT, alanine aminotransferase; AST, aspartate aminotransferase; γ-GT, glutamyl transpeptidase; T-BIL, total bilirubin; D-BIL, direct bilirubin; ALP, alkaline phosphatase; ALB, albumin; BUN, urea nitrogen; UA, uric acid; CR, creatinine; LDH, lactate dehydrogenase; CK, creatine phosphokinase; GSP, fructosamine; GLU, glucose; INS, insulin; CHO, cholesterol; TG, triglycerides; LDL-C, low-density lipoprotein; HDL-C, high-density lipoprotein; TT3, triiodothyronine; TT4, tetraiodothyronine; TSH, thyroid stimulating hormone; IL-1, interleukin 1; IL-2, interleukin 2; IL-4, interleukin 4; IFN-γ, interferon γ; IFN-α, interferon α; TNF-α, tumor necrosis factor α; IgG, immunoglobulin G; IgA, immunoglobulin A; IgM, immunoglobulin M; C3, complement C3; CH50, total complement CH50 | | |

**Example 12. comparison of in vivo safety between metal-chelated phospholipid complex nanoparticles (MPP) with $Fe^{3+}$ or $Al^{3+}$ as metal ion and LNP**

The main toxicity of LNP comes from its main components - cationic lipid and/or ionizable lipid. When LNP is metabolized in the body, the free cationic lipid and/or ionizable lipid will produce obvious toxicity to the body. A median lethal dose ($IC_{50}$) of cationic lipid and/or ionizable lipid to biological cells is an important parameter for evaluating toxicity of LNP to the body. The metal-chelated phospholipid complex nanoparticles (MPP) uses metal-chelated phospholipid complex to replace the cationic lipid/ionizable lipid in LNP. Therefore, we compared the difference in toxicity between LNP and MPP($Fe^{3+}$) or MPP($Al^{3+}$) by studying the median lethal dose ($IC_{50}$) of metal-chelated phospholipid complex and cationic lipid/ionizable lipid to biological cells.

After incubating 293T cells with different concentrations of the metal-chelated phospholipid complex (0, 0.1, 0.3, 0.9, 2.7, 8.1, 24.3, 72.9, 218.7 μM), cationic lipid (DOTAP, 0, 0.1, 0.3, 0.9, 2.7, 8.1, 24.3, 72.9, 218.7 μM, the structural formula is as follows) and ionizable lipid (ALC0315, 0, 0.1, 0.3, 0.9, 2.7, 8.1, 24.3, 72.9, 218.7 μM, the structural formula is as follows) for 48 hours, cell activity was detected using a CCK8 activity detection kit, and the median lethal dose $IC_{50}$ of the metal-chelated phospholipid complex, cationic lipid (DOTAP) and ionizable lipid (ALC0315) for 293T cells was calculated respectively.

DOTAP

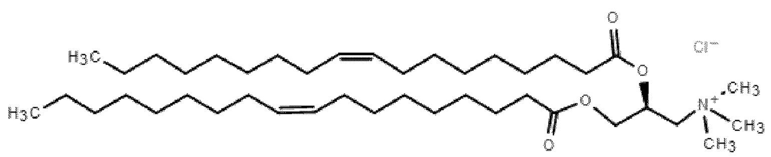

.

ALC0315

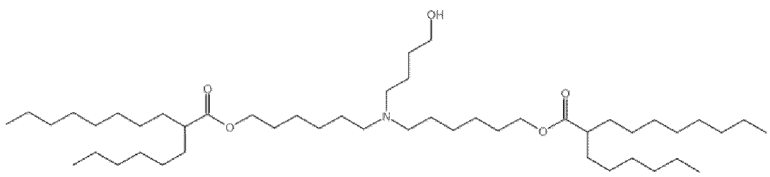

.

Detection method of CCK8:

1. Cell culture: cells were cultivated with DMEM culture medium containing 10% FBS and 1% Penicillin-Streptomycin Solution, and wait until the cell density reached 80%-90% of the culture bottle;
2. The remaining culture medium in the culture bottle was washed with PBS, the culture bottle was added with trypsin, and quickly transferred to a 37°C incubator containing 5% $CO_2$. During close observation, when the cells become slightly round, culture medium was added to stop digestion. The mixture was transferred to a centrifuge tube, centrifuged at 1500RPM for 5 minutes, and the cells were resuspended with fresh culture medium;
3. Counting: the cell suspension was diluted to 100,000 cells per 1mL according to the purpose, 100μL per well in a 96-well plate, and at least 5 replicates per group. Drug was added after 24h of incubation at 37°C, 5%$CO_2$;
4. After drug incubation for 48h, 10% CCK8 was added. Incubation is made for 1-3h, and absorbance was measured by an ELISA reader at 450nm;
5. Survival rate (%) = [A (drug added) - A (blank)] / [A (0 drug added) - A (blank)] × 100%.

$IC_{50}$ calculation method: with survival rate as the ordinate and drug concentration as the abscissa, $IC_{50}$ was calculated by Graphpad using [Inhibitor] vs. normalized response -Variable slope analysis method.

To compare the in vivo safety of MPP and LNP, MPP (8 mg/kg) and LNP (3.24 mg/kg) that can load the same amount of nucleic acid (200 μg/kg mRNA) were taken, and in vivo experiments were carried out according to the method of Example 11 to evaluate and compare the in vivo toxicity of MPP and LNP.

Result analysis: as shown in Table 2-2, the $IC_{50}$ of the metal-chelated phospholipid complex is significantly greater than that of cationic lipid (DOTAP) and ionizable lipid (ALC0315). The results indicate that the toxicity of the metal-chelated phospholipid complex is significantly less than that of cationic lipid and ionizable lipid.

As shown in Table 2-3, at the end of the administration period and the end of the recovery period, compared with the control group, the liver function (ALT, AST, ALP) and cytokine (IL-6, IL-1β) of the MPP ($Fe^{3+}$) or MPP ($Al^{3+}$) groups were not significantly abnormal. However, compared with the control group, the liver function (ALT, AST, ALP) and cytokine (IL-6, IL-1β) of the LNP group were significantly increased. The results suggest that the in vivo safety of MPP ($Fe^{3+}$) or MPP ($Al^{3+}$) is higher than that of LNP. The reason is that the core component of LNP is the artificially synthesized "cationic lipid/ionizable lipid", which has high cytotoxicity and immunogenicity, and its structure is relatively stable and difficult to decompose in the body; while the core component of MPP ($Fe^{3+}$) or MPP ($Al^{3+}$) is a metal-chelated phospholipid complex, which is composed of a phospholipid molecule, a highly safe natural small molecule curcumin (a food additive and pharmaceutical excipient approved by the FDA) and safe metal ions, and it has been decomposed into natural molecules in the body after completing drug delivery. In summary, because there is no cationic lipid/ionizable lipid in the composition of MPP ($Fe^{3+}$) or MPP ($Al^{3+}$), it will not cause toxic and side effects related to cationic lipid/ionizable lipid, so the safety of MPP ($Fe^{3+}$) or MPP ($Al^{3+}$) is higher than that of LNP.

Table 2-2 Comparison of $IC_{50}$ between metal-chelated phospholipid complexes with $Fe^{3+}$ or $Al^{3+}$ as metal ion, cationic lipid (DOTAP) and ionizable lipid (ALC0315)

| | Metal-chelated phospholipid complexe with $Fe^{3+}$ as metal ion | Metal-chelated phospholipid complexes with $Al^{3+}$ as metal ion | DOTAP | ALC0315 |
|---|---|---|---|---|
| $IC_{50}$(μM) | 640.9 | 501.6 | 40.7 | 145.1 |

Table 2-3 Comparison of chronic toxicity test indicators of MPP and LNP

| | MPP when the metal ion is $Fe^{3+}$ | MPP when the metal ion is $Al^{3+}$ | LNP |
|---|---|---|---|
| Liver function indicators(ALT, AST, ALP) | No abnormality | No abnormality | Abnormal |
| Cytokines(IL-6,IL-1β) | No abnormality | No abnormality | Abnormal |

## Embodiment 3. Clinical application and administration route of drug-loaded metal-chelated phospholipid complex nanoparticles

### Example 13. Clinical application and administration route of drug-loaded metal-chelated phospholipid complex nanoparticles with $Fe^{3+}$ or $Al^{3+}$as metal ion

The mRNA in Example 3 was replaced with siRNA targeting B7-H4 gene (B7-H4-siRNA) and its control (scr-siRNA), and mRNA encoding the receptor binding domain (RBD) of the S1 subunit of the SARS-COV-2 (RBD-mRNA).

The sequences of the above different nucleic acids are: (1) the sequence of B7-H4-siRNA shown in SEQ ID No.19 (sense chain) and SEQ ID No.26 (antisense chain) (25bp), and its random control sequence shown in SEQ ID No.20

(sense chain) and SEQ ID No.27 (antisense chain) (19bp); (2) the mRNA sequence encoding the receptor binding domain (RBD) of the SARS-COV-2 S1 subunit shown in SEQ ID No.2 (669nt). Referring to the method of Example 3, drug-loaded metal-chelated phospholipid complex nanoparticles (B7-H4-siRNA @MPP ($Fe^{3+}$), RBD-mRNA @MPP ($Fe^{3+}$), B7-H4-siRNA @MPP ($Al^{3+}$), RBD-mRNA @MPP ($Al^{3+}$)) containing the above different types of nucleic acids were prepared respectively, and the rest of preparation process of the drug-loaded metal-chelated phospholipid complex nanoparticles was the same as that of Example 3. The above two different drug-loaded metal-chelated phospholipid complex nanoparticles (B7-H4-siRNA @MPP, RBD-mRNA@MPP) were used to treat liver cancer and as mRNA vaccines to prevent the SARS-COV-2, respectively.

The sequence of B7-H4-siRNA is as follows:

sense 5'-GGG AGA CAC UCC AUC ACA GUC ACU A -3' (SEQ ID No.19).
antisense 5'-UAG UGA CUG UGA UGG AGU GUC UCC C-3' (SEQ ID No.26) (25bp).

The random control sequence of B7-H4-siRNA is as follows:

sense 5'-UUCUCCGAACGUGUCACGU-3' (SEQ ID No.20).
antisense 5'-ACGUGACACGUUCGGAGAA-3' (SEQ ID No.27) (19bp).

To evaluate the effects of B7-H4-siRNA@MPP ($Fe^{3+}$) and B7-H4-siRNA@MPP ($Al^{3+}$) in treating liver cancer, an animal model of liver cancer was established using HepG2 cells. When the tumor size increased to about 100 $mm^3$, mice with liver cancer were randomly divided into 7 groups (5 mice in each group): PBS control group, blank vector MPP ($Fe^{3+}$) group, blank vector MPP ($Al^{3+}$) group, Scr-siRNA@MPP ($Fe^{3+}$) control group, B7-H4-siRNA@MPP ($Fe^{3+}$) treatment group, Scr-siRNA@MPP ($Al^{3+}$) control group, and B7-H4-siRNA@MPP ($Al^{3+}$) treatment group. Each group of mice was intratumorally injected with PBS, MPP ($Fe^{3+}$), MPP ($Al^{3+}$), Scr-siRNA@MPP ($Fe^{3+}$), B7-H4 siRNA@MPP ($Fe^{3+}$), Scr-siRNA@MPP ($Al^{3+}$), B7-H4 siRNA@MPP ($Al^{3+}$) once every 3 days, with a dose of 200$\mu$g siRNA/kg, and 8 injections were performed. The tumor volume was measured and recorded every 3 days. The results are shown in Figure 3-1.

To evaluate the role of RBD-mRNA@MPP as an mRNA vaccine to prevent SARS-COV-2, the experimental process and experimental methods are as shown in the previous Example 3.5.

The ELISA detection method is as described in Example 3.5.

Establishment of liver cancer mouse model: HepG2 cells were collected and resuspended in PBS at a density of $1\times10^7$/mL and stored on ice before inoculation. Then 100$\mu$L of the cell suspension was injected subcutaneously into the back area near the hind legs of female Balb/c nude mice to establish a liver cancer mouse model.

Result analysis:

As shown in Figure 3-1, Scr-siRNA@MPP($Fe^{3+}$) and Scr-siRNA@MPP($Al^{3+}$) had almost no inhibitory effect on the growth of liver cancer HepG2 cells, while B7-H4-siRNA@MPP($Fe^{3+}$) and B7-H4 siRNA@MPP($Al^{3+}$) showed a high therapeutic effect and could effectively inhibit the growth of liver cancer tumors. The results suggest that drug-loaded metal-chelated phospholipid complex nanoparticles can encapsulate and deliver B7-H4 siRNA, thereby inhibiting the development of liver cancer by inhibiting the expression of target genes.

As shown in the previous Example 3.5, Figure 1-3, and Figure 1-5, RBD-mRNA@MPP($Fe^{3+}$) increased the expression level of mouse IgG antibody to 117268.8 (Figure 1-3), and the expression levels of cytokines IFN-$\gamma$, IL-2, and IL-4 reached 252.8 pg/mL, 207.6 pg/mL, and 56.6 pg/mL, respectively (Figure 1-5). RBD-mRNA@MPP($Al^{3+}$) increased the expression level of mouse IgG antibody to 129113 (Figure 1-17), and the expression levels of cytokines IFN-$\gamma$, IL-2, and IL-4 reached 271.8 pg/mL, 234.6 pg/mL, and 68.4 pg/mL, respectively (Figure 1-19). The results suggest that RBD-mRNA@MPP can effectively induce humoral immunity in mice and produce high levels of antigen-specific binding antibodies; at the same time, it can effectively induce cellular immunity in mice, that is, activate immune cells and produce a large number of cytokines. Therefore, RBD-mRNA@MPP can effectively prevent infection with the SARS-COV-2.

As shown in Figure 3-1, B7-H4-siRNA@MPP can effectively treat liver cancer by intratumoral injection; as shown in Example 3.5, Figures 1-3, 1-5, 1-9 and 1-11, RBD-mRNA@MPP can activate humoral immunity and cellular immunity by intramuscular injection, thereby preventing infection with the SARS-COV-2. The results suggest that drug-loaded metal-chelated phospholipid complex nanoparticles can be administered through a variety of routes.

**Embodiement 4. Functions of the nanoparticles when curcumin and $Fe^{3+}$ or $Al^{3+}$ are replaced by analogs**

**Example 12. Functions of the nanoparticles when curcumin and $Fe^{3+}$ or $Al^{3+}$ are replaced by analogs**

Referring to Example 1, Example 2, and Example 3, DSPC, curcumin and $Fe^{3+}$ were replaced by their analogs,

respectively. Twenty-eight different drug-loaded metal-chelated phospholipid complex nanoparticles (eGFP-mRNA@MPPs) were prepared by different combinations, wherein the concentration of mRNA contained in each eGFP-mRNA@MPP was 2μg/mL. The names and structures of DSPC, curcumin, $Fe^{3+}$ and their analogs are shown in Table 4-1, and the combinations of curcumin, $Fe^{3+}$ and their analogs in the twenty-eight mRNA@MPPs are shown in Table 4-2. Among those, the reaction temperature in Example 1 is 65°C, reaction time is 2h, the reaction temperature in Example 2 is 60°C, the reaction time is 2 h, and other conditions remain unchanged.

To compare the effects of the twenty-eight different eGFP-mRNA@MPPs and eGFP-mRNA@LNP, we prepared LNP encapsulating equal amounts of eGFP mRNA with reference to Example 9 to obtain eGFP-mRNA@LNP.

The twenty-eight different eGFP-mRNA@MPPs and the eGFP-mRNA@LNP (the concentration of the mRNA contained of each was 2 μg/mL) were incubated with 293T cells respectively, and the control group was incubated with MPP or LNP. After 48 h, the cell suspension was collected and the percentage of eGFP-positive cells was detected by flow cytometry.

The method for analyzing the eGFP-positive cell rate by flow cytometry is as described in Example 3.

The main toxicity of LNP comes from its main component, cationic lipid/ionizable lipid. When LNP is metabolized in the body, the free cationic lipid/ionizable lipid will produce obvious toxicity to the body. The median lethal dose ($IC_{50}$) of cationic lipid/ionizable lipid for biological cells is an important parameter for evaluating the toxicity of LNP to the body. The metal-chelated phospholipid complex nanoparticle (MPP) replaces the cationic lipid/ionizable lipid in LNP with metal-chelated phospholipid complex. Therefore, we compared the differences in toxicity between LNP and twenty-eight MPPs by studying the median lethal dose ($IC_{50}$) of twenty-eight metal-chelated phospholipid complexes and cationic lipid (DOTAP)/ionizable lipid (ALC0315) in Table 4-2 for biological cells.

The calculation method of $IC_{50}$ is as described in Example 12.

Result analysis: as shown in Table 4-2, the percentages of eGFP-positive cells in 293T cells treated with twenty-eight different eGFP-mRNA@MPPs were significantly higher than that of eGFP-mRNA@LNP, among which the percentage of eGFP-positive cells of mRNA@MPP composed of DSPC, curcumin and $Fe^{3+}$ was the highest. The results suggest that the function of mRNA@MPPs formed after DSPC, curcumin and $Fe^{3+}$ are replaced by their analogs is not as good as that of mRNA@MPP, but better than that of mRNA@LNP. The possible reason is that as described in Example 8, MPP has a stronger capability to promote nucleic acids escape from lysosomes than LNP, so more nucleic acids loaded by MPP can be effectively released into the cytoplasm and translated into proteins.

The above results suggest that as long as the following conditions are met, the function of the drug-loaded metal-chelated phospholipid complex nanoparticles composed of the analogs that replace DSPC, curcumin and $Fe^{3+}$ will not be affected: (1) the analogs of DSPC are amphiphilic phospholipid molecules; (2) the analogs of $Fe^{3+}$ are a metal ion; (3) the analogs of curcumin can form phospholipid complexes with the analogs of DSPC, and can be complexed with metal ions (4) the coordination bond between curcumin and $Fe^{3+}$ can break in response to the low pH environment of lysosomes.

As shown in Table 4-3, the $IC_{50}$ of the twenty-eight metal-chelated phospholipid complexes are significantly greater than those of cationic lipid (DOTAP) and ionizable lipid (ALC0315). The results suggest that the toxicity of metal-chelated phospholipid complexes is significantly lower than that of cationic lipid and ionizable lipid, that is, the safety of lipid nanoparticles (MPP) composed of DSPC, curcumin, $Fe^{3+}$ and their analogs is higher than that of LNP. The reason is that the main component of LNP is artificially synthesized "cationic lipid/ionizable lipid", which has high cytotoxicity and immunogenicity, and its structure is relatively stable and difficult to decompose and metabolize in the body; while the main component of MPP is metal-chelated phospholipid complex, which is composed of phospholipid molecules, highly safe natural small molecules (of which curcumin is a food additive and pharmaceutical excipient approved by the FDA) and safe metal ions, and it can be decomposed into natural molecules in the body after completing drug delivery. In summary, the lipid particles (MPP) composed of DSPC, curcumin, $Fe^{3+}$ and their analogs do not contain cationic lipid/ionizable lipid, and will not cause toxic and side effects related to cationic lipid/ionizable lipid, so the safety of MPP is higher than that of LNP.

Table 4-1 Names and structures of DSPC, curcumin, $Fe^{3+}$ and their analogs

| | Name | Structure |
|---|---|---|
| DSPC and its analogs | DSPC | 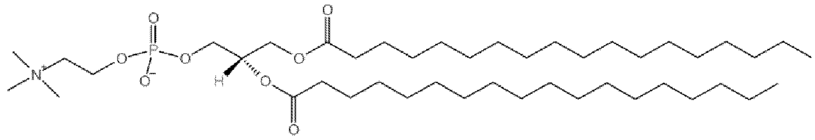 |
| | DSPE | 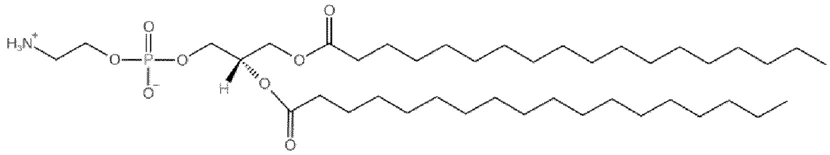 |
| | DSPA | 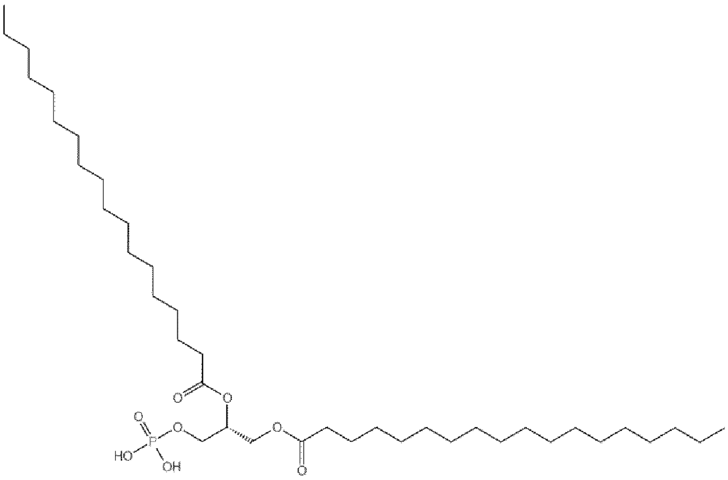 |
| | DSPG | 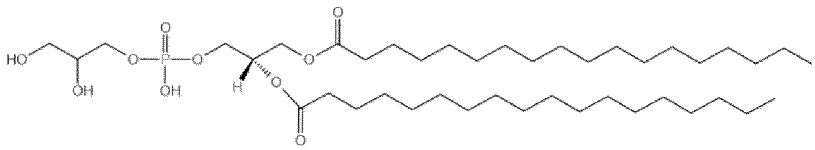 |

(continued)

| | Name | Structure |
|---|---|---|
| Curcumin and its analogs | Curcumin | 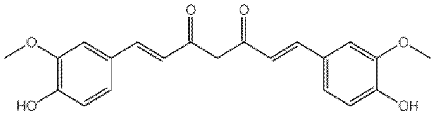 |
| | Hesperidin | 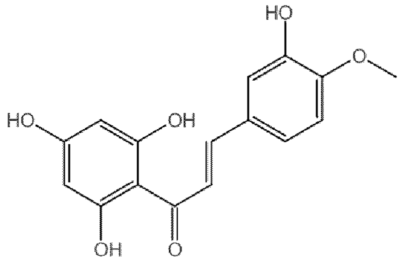 |
| | Tea polyphenol | 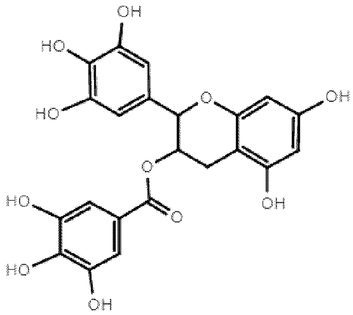 |
| $Fe^{3+}$ and its analogs | $Fe^{3+}$ | |
| | $Ca^{2+}$ | |
| | $Al^{3+}$ | |

Table 4-2 List of combinations and functions of metal-chelated phospholipid complexes in drug-lipid particles prepared from DSPC, curcumin, $Fe^{3+}$ and their analogs

| Drug-lipid nanoparticles | Main component | Percentage of eGFP-positive cells (%) |
|---|---|---|
| mRNA@LNP | ALC0315 (ionizable lipid) | 43.1 |
| mRNA@MPP1 | Metal-chelated phospholipid complex composed of DSPC, curcumin and $Fe^{3+}$ | 98.2 |
| mRNA@MPP2 | Metal-chelated phospholipid complex composed of DSPC, curcumin and $Al^{3+}$ | 98.4 |
| mRNA@MPP3 | Metal-chelated phospholipid complex composed of DSPC, curcumin and $Ca^{2+}$ | 80.1 |
| mRNA@MPP4 | Metal-chelated phospholipid complex composed of DSPC, hesperidin and $Fe^{3+}$ | 73.0 |
| mRNA@MPP5 | Metal-chelated phospholipid complex composed of DSPC, hesperidin and $Al^{3+}$ | 82.9 |
| mRNA@MPP6 | Metal-chelated phospholipid complex composed of DSPC, hesperidin and $Ca^{2+}$ | 80.8 |
| mRNA@MPP7 | Metal-chelated phospholipid complex composed of DSPC, tea polyphenol and $Fe^{3+}$ | 91.5 |
| mRNA@MPP8 | Metal-chelated phospholipid complex composed of DSPC, tea polyphenol and $Al^{3+}$ | 77.3 |

(continued)

| Drug-lipid nanoparticles | Main component | Percentage of eGFP-positive cells (%) |
|---|---|---|
| mRNA@MPP9 | Metal-chelated phospholipid complex composed of DSPC, tea polyphenol and $Ca^{2+}$ | 69.3 |
| mRNA@MPP10 | Metal-chelated phospholipid complex composed of DSPE, curcumin and $Fe^{3+}$ | 58.4 |
| mRNA@MPP11 | Metal-chelated phospholipid complex composed of DSPE, curcumin and $Al^{3+}$ | 82.4 |
| mRNA@MPP12 | Metal-chelated phospholipid complex composed of DSPE, curcumin and $Ca^{2+}$ | 77.5 |
| mRNA@MPP13 | Metal-chelated phospholipid complex composed of DSPE, hesperidin and $Fe^{3+}$ | 86.1 |
| mRNA@MPP14 | Metal-chelated phospholipid complex composed of DSPE, hesperidin and $Al^{3+}$ | 79.3 |
| mRNA@MPP15 | Metal-chelated phospholipid complex composed of DSPE, hesperidin and $Ca^{2+}$ | 59.0 |
| mRNA@MPP16 | Metal-chelated phospholipid complex composed of DSPE, tea polyphenol and $Fe^{3+}$ | 63.1 |
| mRNA@MPP17 | Metal-chelated phospholipid complex composed of DSPE, tea polyphenol and $Al^{3+}$ | 69.5 |
| mRNA@MPP18 | Metal-chelated phospholipid complex composed of DSPE, tea polyphenol and $Ca^{2+}$ | 70.2 |
| mRNA@MPP19 | Metal-chelated phospholipid complex composed of DSPA, curcumin and $Fe^{3+}$ | 72.3 |
| mRNA@MPP20 | Metal-chelated phospholipid complex composed of DSPA, curcumin and $Al^{3+}$ | 77.2 |
| mRNA@MPP21 | Metal-chelated phospholipid complex composed of DSPA, curcumin and $Ca^{2+}$ | 65.1 |
| mRNA@MPP22 | Metal-chelated phospholipid complex composed of DSPA, hesperidin and $Fe^{3+}$ | 70.1 |
| mRNA@MPP23 | Metal-chelated phospholipid complex composed of DSPA, hesperidin and $Al^{3+}$ | 81.7 |
| mRNA@MPP24 | Metal-chelated phospholipid complex composed of DSPA, hesperidin and $Ca^{2+}$ | 56.1 |
| mRNA@MPP25 | Metal-chelated phospholipid complex composed of DSPA, tea polyphenol and $Fe^{3+}$ | 70.4 |
| mRNA@MPP26 | Metal-chelated phospholipid complex composed of DSPA, tea polyphenol and $Al^{3+}$ | 75.9 |
| mRNA@MPP27 | Metal-chelated phospholipid complex composed of DSPA, tea polyphenol and $Ca^{2+}$ | 54.9 |
| mRNA@MPP28 | Metal-chelated phospholipid complex composed of DSPG, curcumin and $Fe^{3+}$ | 53.0 |

Table 4-3 IC50 of metal-chelated phospholipid complexes prepared from DSPC curcumin, $Fe^{3+}$ and their analogs

| Type | Name | $IC_{50}$ (μM) |
|---|---|---|
| Ionizable lipid | ALC0315 | 145.1 |
| Cationic lipid | DOTAP | 40.7 |

(continued)

| Type | Name | $IC_{50}$ (μM) |
|---|---|---|
| Metal-chelated polyphenol complexes | Metal-chelated phospholipid complex composed of DSPC, curcumin and $Fe^{3+}$ | 646.9 |
| | Metal-chelated phospholipid complex composed of DSPC, curcumin and $Al^{3+}$ | 704.3 |
| | Metal-chelated phospholipid complex composed of DSPC, curcumin and $Ca^{2+}$ | 642.1 |
| | Metal-chelated phospholipid complex composed of DSPC, hesperidin and $Fe^{3+}$ | 190.8 |
| | Metal-chelated phospholipid complex composed of DSPC, hesperidin and $Al^{3+}$ | 219.0 |
| | Metal-chelated phospholipid complex composed of DSPC, hesperidin and $Ca^{2+}$ | 209.3 |
| | Metal-chelated phospholipid complex composed of DSPC, tea polyphenol and $Fe^{3+}$ | 1573.7 |
| | Metal-chelated phospholipid complex composed of DSPC, tea polyphenol and $Al^{3+}$ | 1257.1 |
| | Metal-chelated phospholipid complex composed of DSPC, tea polyphenol and $Ca^{2+}$ | 1679.5 |
| | Metal-chelated phospholipid complex composed of DSPE, curcumin and $Fe^{3+}$ | 590.7 |
| | Metal-chelated phospholipid complex composed of DSPE, curcumin and $Al^{3+}$ | 582.9 |
| | Metal-chelated phospholipid complex composed of DSPE, curcumin and $Ca^{2+}$ | 657.3 |
| | Metal-chelated phospholipid complex composed of DSPE, hesperidin and $Fe^{3+}$ | 342.9 |
| | Metal-chelated phospholipid complex composed of DSPE, hesperidin and $Al^{3+}$ | 322.4 |
| | Metal-chelated phospholipid complex composed of DSPE, hesperidin and $Ca^{2+}$ | 219.1 |
| | Metal-chelated phospholipid complex composed of DSPE, tea polyphenol and $Fe^{3+}$ | 2163.5 |
| | Metal-chelated phospholipid complex composed of DSPE, tea polyphenol and $Al^{3+}$ | 1726.3 |
| | Metal-chelated phospholipid complex composed of DSPE, tea polyphenol and $Ca^{2+}$ | 1369.8 |
| | Metal-chelated phospholipid complex composed of DSPA, curcumin and $Fe^{3+}$ | 477.3 |
| | Metal-chelated phospholipid complex composed of DSPA, curcumin and $Al^{3+}$ | 443.9 |
| | Metal-chelated phospholipid complex composed of DSPA, curcumin and $Ca^{2+}$ | 516.5 |
| | Metal-chelated phospholipid complex composed of DSPA, hesperidin and $Fe^{3+}$ | 127.8 |
| | Metal-chelated phospholipid complex composed of DSPA, hesperidin and $Al^{3+}$ | 216.4 |
| | Metal-chelated phospholipid complex composed of DSPA, hesperidin and $Ca^{2+}$ | 195.1 |
| | Metal-chelated phospholipid complex composed of DSPA, tea polyphenol and $Fe^{3+}$ | 1154.5 |
| | Metal-chelated phospholipid complex composed of DSPA, tea polyphenol and $Al^{3+}$ | 1229.0 |
| | Metal-chelated phospholipid complex composed of DSPA, tea polyphenol and $Ca^{2+}$ | 1363.7 |
| | Metal-chelated phospholipid complex composed of DSPG, curcumin and $Fe^{3+}$ | 989.6 |

**Example 16. Feeding ratios of DSPC, curcumin, $Fe^{3+}$ and their analogs in different metal-chelated phospholipid complexes and function of the drug-loaded metal-chelated phospholipid complex nanoparticles prepared therefrom**

According to Example 3, metal-chelated phospholipid complexes were prepared, and curcumin was replaced by its analogs hesperidin (1 molecule of hesperidin contains 4 hydroxyls) and tea polyphenol (1 molecule of tea polyphenol contains 8 hydroxyls), respectively, to prepare three metal-chelated phospholipid complexes (mRNA@MPP1, mRNA@MPP4, mRNA@MPP29). When preparing these three metal-chelated phospholipid complexes, the ratio among DSPC, curcumin or its analogs and $FeCl_3$ was 1:1:1, 1:1:1, 1:1:2, respectively. The corresponding drug-loaded metal-chelated phospholipid complex nanoparticles were prepared using these three metal-chelated phospholipid complexes (mRNA@MPP1, mRNA@MPP4, mRNA@MPP29). The mRNA is an mRNA encoding eGFP fluorescent protein, and its sequence is shown in SEQ ID NO.1 (720nt). The mRNA encapsulation efficiency of these three drug-lipid particles and their capability to promote the expression of eGFP fluorescent protein after treating 293T cells were detected according to the experimental process and experimental methods described in Example 3.5.

Result analysis: as shown in Table 4-4, the mRNA encapsulation efficiency and the capability to promote the

expression of the target protein of the drug-loaded metal-chelated phospholipid complex nanoparticles prepared using different feeding ratios according to the chemical structures of the metal-chelated phospholipid complex components are comparable. The results suggest that the feeding ratios of the metal-chelated phospholipid complex components can be adjusted according to the structures of the specific metal-chelated phospholipid complex components. The basis for adjusting the feeding ratio is in that the DSPC analogs are hydrogen-bonded to curcumin analogs. As long as the DSPC analogs contains multiple phosphate groups, the feeding ratio of DSPC analogs to curcumin analogs in the synthesis of phospholipid complex can be adjusted according to the number of phosphate groups contained in the DSPC analogs, that is, when DSPC analogs contain two phosphate groups, the feeding ratio of DSPC analogs to curcumin analogs can be adjusted to 1:2; when DSPC analogs contain three phosphate groups, the feeding ratio of DSPC analogs to curcumin analogs can be adjusted to 1:3. The basis for adjusting the feeding ratio is further in that that the hydroxyl groups of the curcumin analogs are connected to the $Fe^{3+}$ analogs by coordination bonds. As long as the curcumin analogs contain multiple binding sites, the feeding ratios of the curcumin analogs and the $Fe^{3+}$ analogs can be adjusted according to the number of binding sites contained in the curcumin analogs.

Table 4-4 Feeding ratios of components of different metal-chelated phospholipid complexes and functions of the prepared drug-lipid particles therefrom

| Drug-lipid particles | Components of metal-chelated phospholipid complexes and their feeding ratios | mRNA encapsulation efficiency of drug-lipid particles (%) | Percentage of eGFP-positive cells (%) |
|---|---|---|---|
| mRNA@MPP1 | DSPC, curcumin and $Fe^{3+}$ were fed at a ratio of 1:1:1 | 87 | 97 |
| mRNA@MPP4 | DSPC, hesperidin and $Fe^{3+}$ were fed at a ratio of 1:1:1 | 85 | 72 |
| mRNA@MPP29 | DSPC, tea polyphenol and $Fe^{3+}$ were fed at a ratio of 1:1:2 | 85 | 92 |

**Example 17 Feeding ratios of DSPC, curcumin and its analog, and $Al^{3+}$ in different metal-chelated phospholipid complexes and function of the drug-loaded metal-chelated phospholipid complex nanoparticles prepared therefrom**

According to Example 3, metal-chelated phospholipid complexes were prepared, and curcumin was replaced by its analogs hesperidin (1 molecule of hesperidin contains 4 hydroxyls) and tea polyphenol (1 molecule of tea polyphenol contains 8 hydroxyls), respectively, to prepare three metal-chelated phospholipid complexes (mRNA@MPP2, mRNA@MPP5, mRNA@MPP30). When preparing these three metal-chelated phospholipid complexes, the feeding ratios among DSPC, curcumin or its analogs, and $Al(NO_3)_3 \cdot 9H_2O$ were 1:1:1, 1:1:1, 1:1:2, respectively. And the corresponding drug-loaded metal-chelated phospholipid complex nanoparticles were prepared using these three metal-chelated phospholipid complexes (mRNA@MPP2, mRNA@MPP5, mRNA@MPP30). Among those, mRNA is mRNA encoding eGFP fluorescent protein, and its sequence is shown in SEQ ID NO.1 (720nt). According to the experimental process and experimental method described in Example 3.5, the mRNA encapsulation efficiency of the three drug-lipid nanoparticles and their capability to promote the expression of eGFP fluorescent protein after treating 293T cells were detected.

Result analysis: As shown in Table 4-5, the mRNA encapsulation efficiency and the capability to promote the expression of the target protein of the drug-loaded metal-chelated phospholipid complex nanoparticles prepared by using different dosage ratios according to the chemical structures of the metal-chelated phospholipid complex components are equivalent. The results suggest that the feeding ratios of the metal-chelated phospholipid complex components can be adjusted according to the structures of the specific metal-chelated phospholipid complex components. The basis for adjusting the feeding ratio is in that the DSPC analogs are hydrogen-bonded to curcumin analogs. As long as the DSPC analogs contains multiple phosphate groups, the feeding ratio of DSPC analogs to curcumin analogs in the synthesis of phospholipid complex can be adjusted according to the number of phosphate groups contained in the DSPC analogs, that is, when DSPC analogs contain two phosphate groups, the feeding ratio of DSPC analogs to curcumin analogs can be adjusted to 1:2; when DSPC analogs contain three phosphate groups, the feeding ratio of DSPC analogs to curcumin analogs can be adjusted to 1:3. The basis for adjusting the feeding ratio is further in that the hydroxyl groups of the curcumin analogs are connected to the $Al^{3+}$ analogs by coordination bonds, as long as the curcumin analogs contains multiple binding sites, the feeding ratio of the curcumin analogs and the $Al^{3+}$ analogs can be adjusted according to the number of binding sites contained in the curcumin analogs.

Table 4-5 Feeding ratios of components of different metal-chelated phospholipid complexes and functions of the prepared drug-lipid particles therefrom

| Drug-lipid particles | Components of metal-chelated phospholipid complexes and their feeding ratios | mRNA encapsulation efficiency of drug-lipid particles (%) | Percentage of eGFP-positive cells (%) |
|---|---|---|---|
| mRNA@MPP2 | DSPC, curcumin and $Al^{3+}$ were fed at a ratio of 1:1:1 | 92 | 98 |
| mRNA@MPP5 | DSPC, hesperidin and $Al^{3+}$ were fed at a ratio of 1:1:1 | 86 | 78 |
| mRNA@MPP30 | DSPC, tea polyphenol and $Al^{3+}$ were fed at a ratio of 1:1:2 | 85 | 92 |

The preparation of mRNA@MPP in the above Example 3 was completed by the research group of Professor Shan Wang of Department of Pharmaceutical Engineering, College of Chemistry and Chemical Engineering, Central South University.

Unless otherwise defined, all technical and scientific terms used in the present disclosure have the same meaning as those generally understood by technicians in the technical field to which the present disclosure belongs. In the event of any inconsistency, the meaning described in the present disclosure or the meaning derived from the contents recorded in the present disclosure shall prevail. In addition, the terms used in this specification are only for the purpose of describing the embodiments of the present disclosure and are not intended to limit the present disclosure.

Note that the above are only preferred embodiments of the present disclosure and the technical principles used. Those skilled in the art will understand that the present disclosure is not limited to the specific embodiments described herein, and that various obvious changes, readjustments and substitutions can be made by those skilled in the art without departing from the scope of protection of the present disclosure. Therefore, although the present disclosure is described in more detail through the above embodiments, the present disclosure is not limited to the above embodiments, and may also include more other equivalent embodiments without departing from the technical concept of the present disclosure, all of which belong to the scope of protection of the present disclosure.

## Claims

1. A metal-chelated phospholipid complex forming by reaction of a phospholipid molecular moiety, a linker molecular moiety, and a metal ion moiety, wherein the phospholipid molecular moiety is linked to the linker molecular moiety, the linker molecular moiety is linked to the metal ion moiety through a coordination bond, and the metal-chelated phospholipid complex is not a cationic lipid or an ionizable lipid.

2. The metal-chelated phospholipid complex of claim 1, wherein the phospholipid molecular moiety is selected from the group consisting of lecithin (PC), phosphatidyl ethanolamine (PE), phosphatidyl serine (PS), phosphatidic acid (PA), phosphatidyl glycerol (PG), ceramide-1-phosphate (CP), phosphatidyl inositol (PI), phosphatidyl threonine (PT), sphingomyelin (SM), lysolecithin (LPC), lysophosphatidyl ethanolamine (LPE), lysophosphatidylserine (LPS), lysophosphatidic acid (LPA), lysophosphatidyl glycerol (LPG), lysophosphatidylinositol (LPI), lysophosphatidylthreonine (LPT), lysosphingomyelin (LSM), sphingosine 1-phosphate (S1P), derivatives thereof, and combinations thereof.

3. The metal-chelated phospholipid complex of claim 2, wherein the phospholipid molecular moiety is selected from the group consisting of

lecithin (PC)

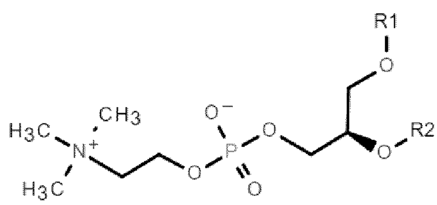

Formula 1,

phosphatidyl ethanolamine (PE)

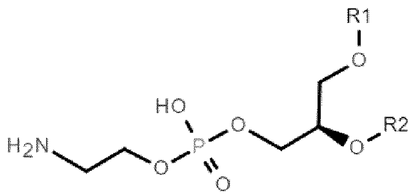

Formula 2,

phosphatidyl serine (PS)

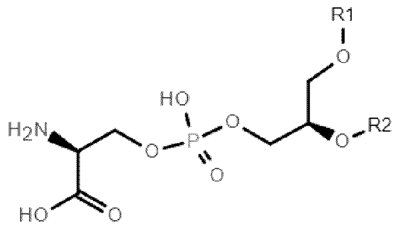

Formula 3,

phosphatidic acid (PA)

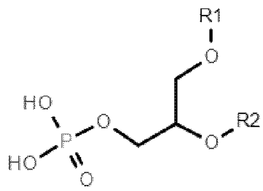

Formula 4,

phosphatidyl glycerol (PG)

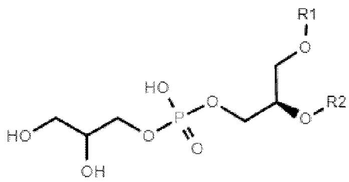

Formula 5,

ceramide-1-phosphate(CP)

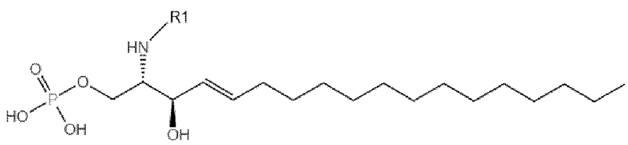

Formula 6,

phosphatidyl inositol (PI)

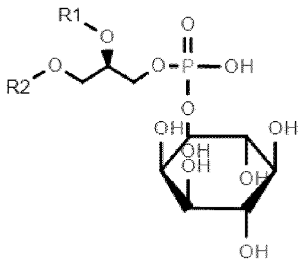

Formula 7,

phosphatidyl threonine (PT)

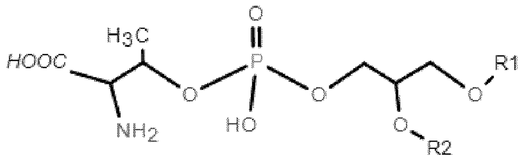

Formula 8,

sphingomyelin (SM)

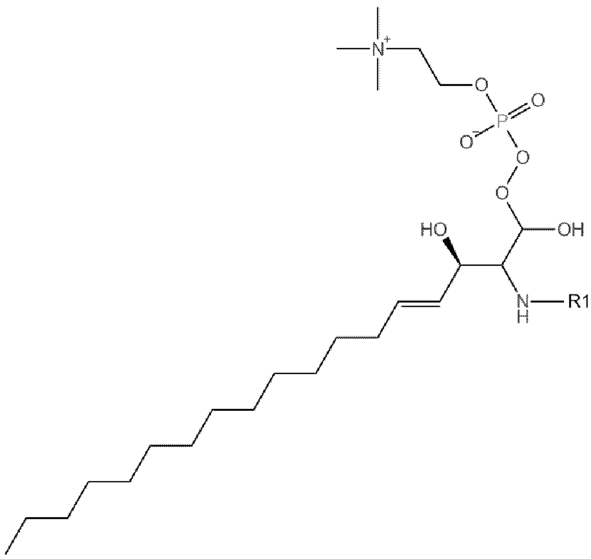

Formula 9,

lysolecithin (LPC)

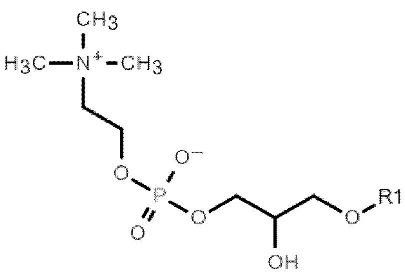

Formula 10,

lysophosphatidyl ethanolamine (LPE)

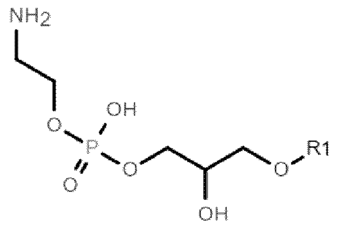

Formula 11,

lysophosphatidylserine (LPS)

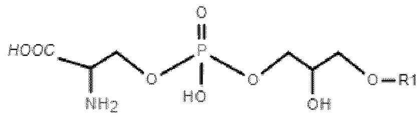

Formula 12,

lysophosphatidic acid (LPA)

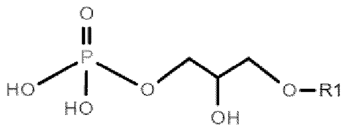

Formula 13,

lysophosphatidyl glycerol (LPG)

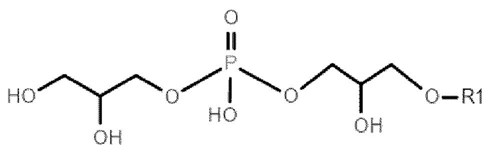

Formula 14,

lysophosphatidylinositol (LPI)

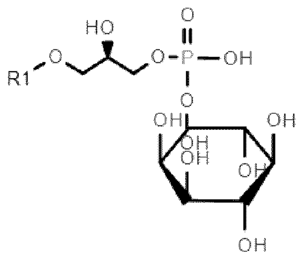

Formula 15,

lysophosphatidylthreonine (LPT)

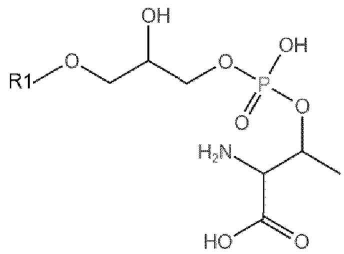

Formula 16,

lysosphingomyelin (LSM)

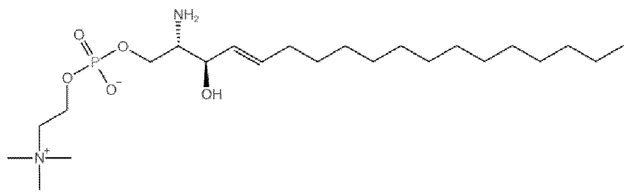

Formula 17,

sphingosine-1-phosphate (S1P)

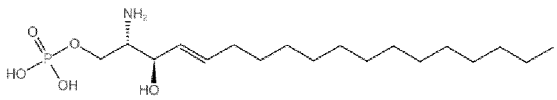

Formula 18,

derivatives thereof, and combinations thereof;

wherein, R1, R2 may each independently be:

decanoyl

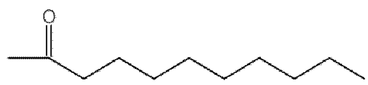

,

lauroyl

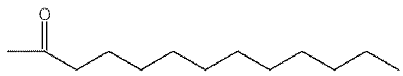

,

myristoyl

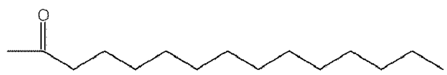

,

palmitoyl

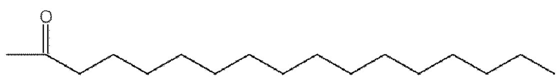

,

stearyl

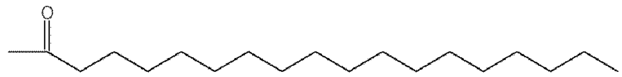

,

oleoyl

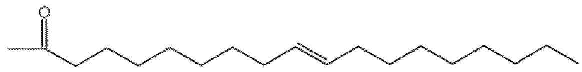

,

linoleoyl

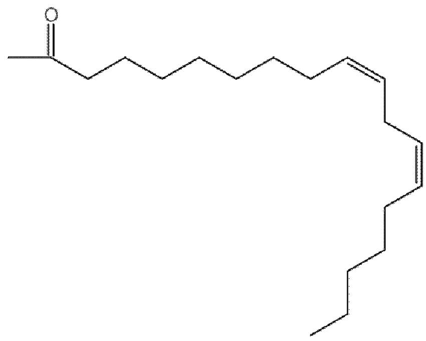

,

erucoyl

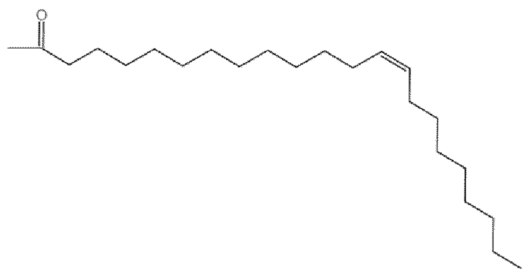

,

arachidoyl

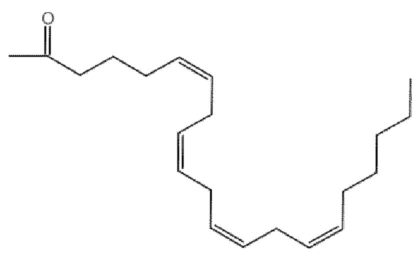

or
phytanoyl

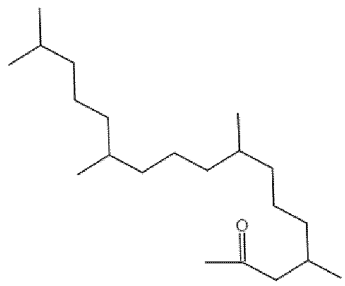

.

4. The metal-chelated phospholipid complex of claim 3, wherein the phospholipid molecular moiety is selected from the group consisting of lecithin (PC, Formula 1), phosphatidyl ethanolamine (PE, Formula 2), phosphatidic acid (PA, Formula 4), phosphatidyl glycerol (PG, Formula 5), derivatives thereof, and combinations thereof.

5. The metal-chelated phospholipid complex of claim 4, wherein the phospholipid molecular moiety is selected from the group consisting of DSPC, DSPE, DSPA, DSPG, derivatives thereof, and combinations thereof.

6. The metal-chelated phospholipid complex of claim 5, wherein the phospholipid molecular moiety is selected from the group consisting of

DSPC (Formula 46)

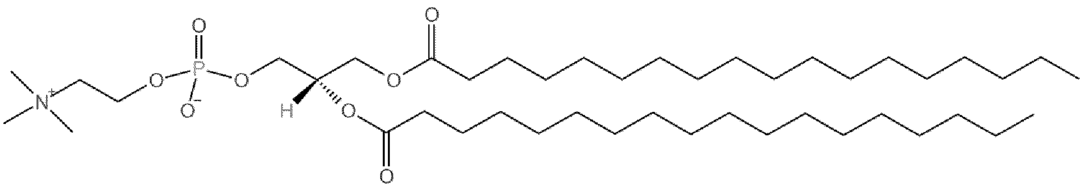

DSPE (Formula 47)

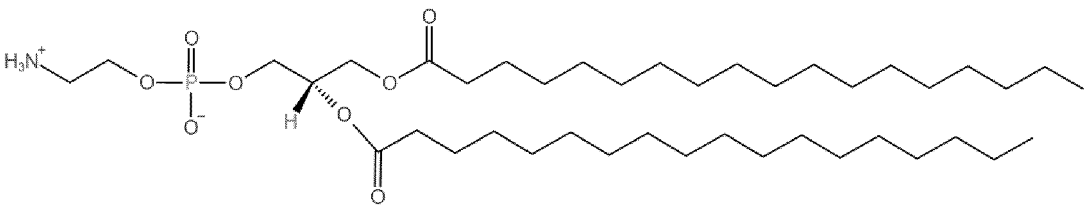

,

DSPA (Formula 48)

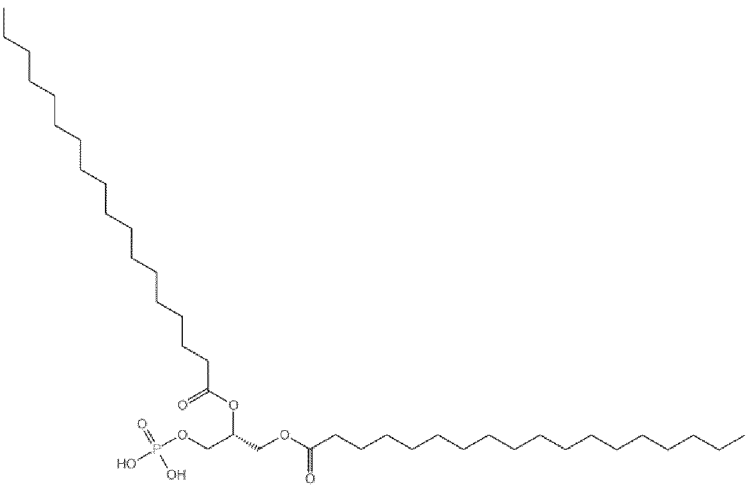

,

DSPG (Formula 49)

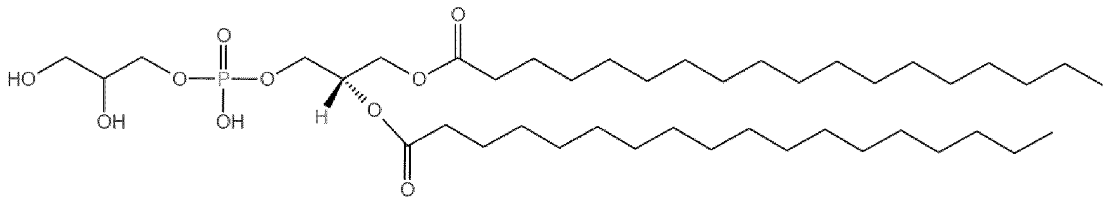

,

derivatives thereof, and combinations thereof.

7. The metal-chelated phospholipid complex of claim 6, wherein the phospholipid molecular moiety is selected from DSPC (Formula 46), DSPE (Formula 47) and DSPA (Formula 48).

8. The metal-chelated phospholipid complex of any one of claims 1 to 7, wherein the linker molecular moiety is selected from the group consisting of curcumin, chlorogenic acid, anthocyanins, quercetin, dihydromyricetin, hesperetin, naringenin, apigenin, catechin, tea polyphenol, epigallocatechin gallate, ellagic acid, morin, epicatechin gallate, catechin gallate, gallocatechin gallate, Pingpeimine C, derivatives thereof, and combinations thereof.

9. The metal-chelated phospholipid complex of claim 8, wherein the linker molecular moiety is selected from the group consisting of

curcumin

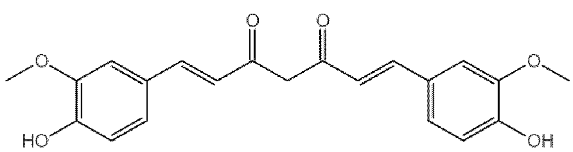

Formula 19,

chlorogenic acid

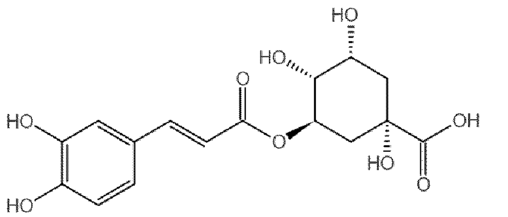

Formula 20,

anthocyanidin

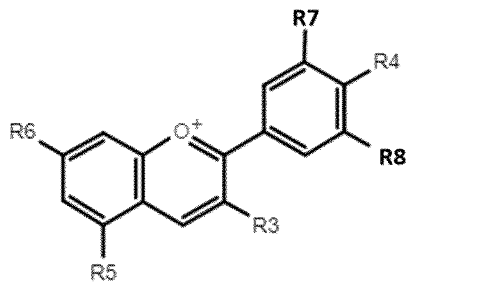

Formula 21,

wherein R7 and R8 are H, OH or $OH_3$, R3 is H or a glycosyl, R4, R5 and R6 are OH or a glycosyl, quercetin

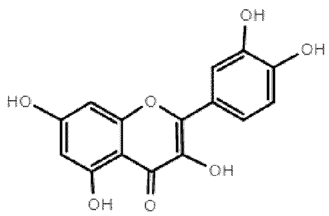

Formula 22,

dihydromyricetin

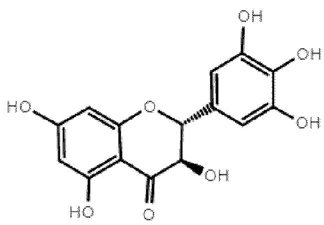

Formula 23,

hesperetin

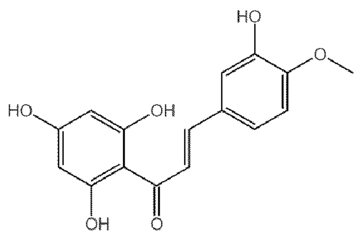

Formula 24,

naringenin

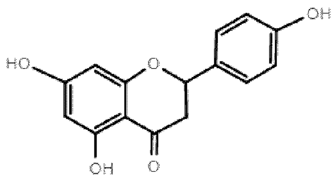

Formula 25,

apigenin

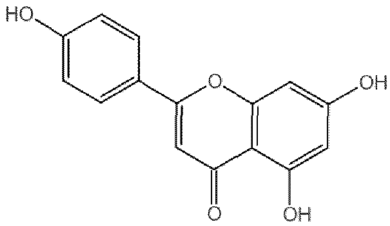

Formula 26,

catechin

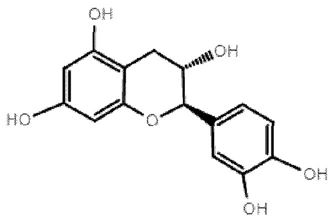

Formula 27,

tea polyphenol

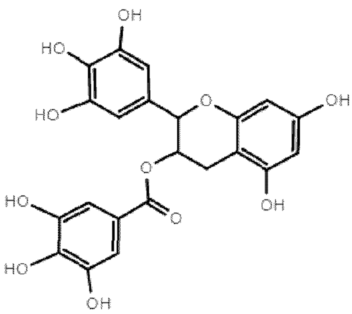

Formula 28,

epigallocatechin gallate

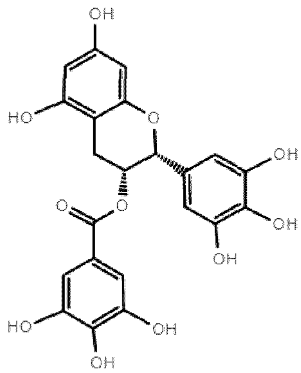

Formula 29,

ellagic acid

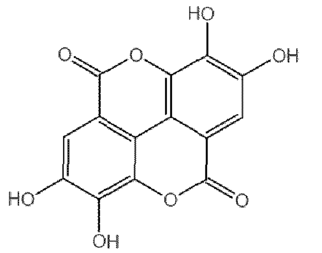

Formula 30,

morin

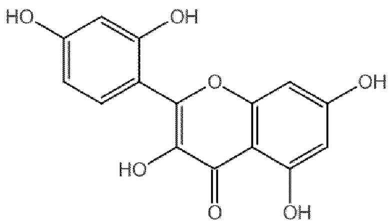

Formula 31,

epicatechin gallate

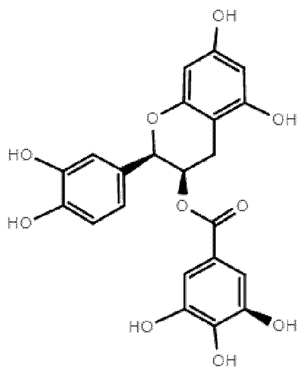

Formula 32,

catechin gallate

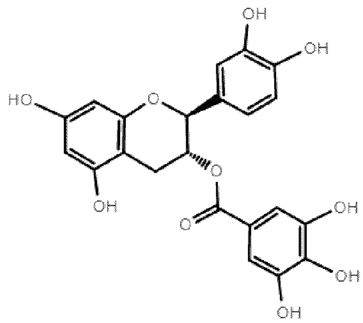

Formula 33,

gallocatechin gallate

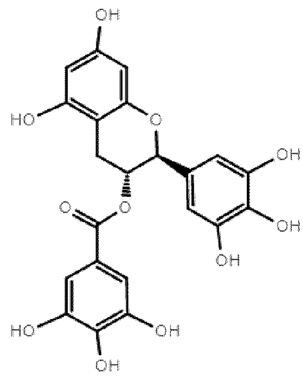

Formula 34,

Pingpeimine C

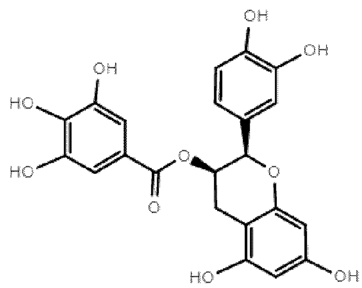

Formula 35,

derivatives thereof, and combinations thereof.

**10.** The metal-chelated phospholipid complex of claim 9, wherein the linker molecular moiety is selected from the group consisting of curcumin (Formula 19),

dihydrocurcumin

(Formula 36) 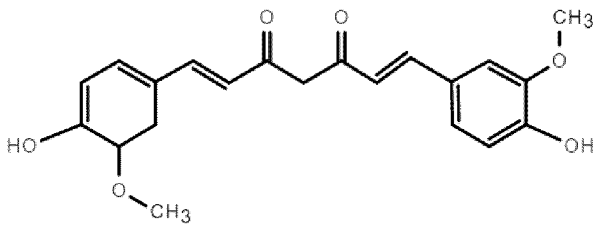 ,

hexahydrocurcumin

(Formula 37) 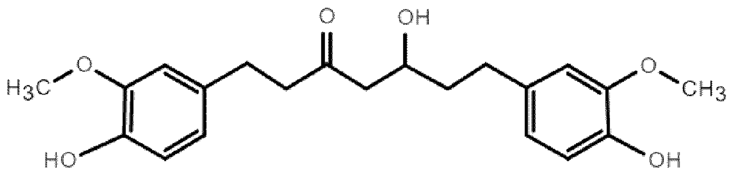 ,

curcumin sulfate

(Formula 38) 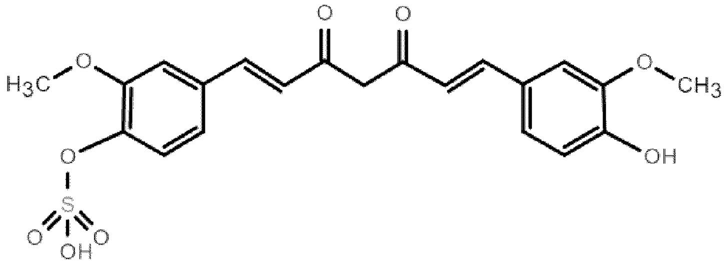 ,

bisdemethoxycurcumin

(Formula 39) 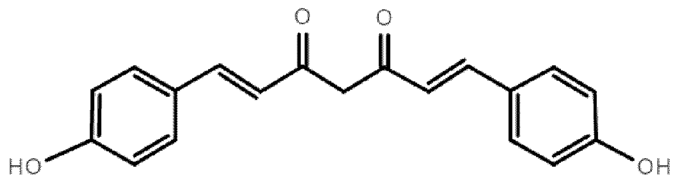

and combinations thereof.

**11.** The metal-chelated phospholipid complex of claim 9, wherein the linker molecular moiety is selected from the group consisting of curcumin (Formula 19), hesperetin (Formula 24), tea polyphenol (Formula 28), derivatives thereof, and combinations thereof.

**12.** The metal-chelated phospholipid complex of claim 11, wherein the linker molecular moiety is selected from curcumin (Formula 19), hesperetin (Formula 24) and tea polyphenol (Formula 28).

**13.** The metal-chelated phospholipid complex of any one of claims 1 to 12, wherein the metal ion moiety is selected from the group consisting of $Fe^{3+}$, $Ag^{+}$, $Ba^{2+}$, $Ca^{2+}$, $Cd^{2+}$, $Cu^{2+}$, $Fe^{2+}$, $Mn^{2+}$, $Mg^{2+}$, $Mo^{2+}$, $Zn^{2+}$, $Pt^{2+}$, $Au^{2+}$, $Al^{3+}$, $Ce^{3+}$, $Co^{3+}$, $Cr^{3+}$, $Eu^{3+}$, $Gd^{3+}$, $Ni^{3+}$, $W^{3+}$, $V^{3+}$, $Zr^{3+}$, and combinations thereof.

**14.** The metal-chelated phospholipid complex of claim 13, wherein the metal ion moiety is selected from the group consisting of $Fe^{3+}$, $Ca^{2+}$, $Al^{3+}$, and combinations thereof.

**15.** The metal-chelated phospholipid complex of claim 14, wherein the metal ion moiety is selected from $Fe^{3+}$, $Ca^{2+}$, and $Al^{3+}$.

**16.** The metal-chelated phospholipid complex of any one of claims 1 to 15, wherein the metal-chelated phospholipid complex is made from a phospholipid molecular moiety selected from DSPC, DSPE and DSPA, a linker molecular moiety selected from curcumin, hesperetin and tea polyphenol, and a metal ion moiety selected from $Fe^{3+}$, $Ca^{2+}$ and $Al^{3+}$.

**17.** The metal-chelated phospholipid complex of claim 16, wherein the metal-chelated phospholipid complex is made from a phospholipid molecular moiety selected from DSPC (Formula 46), DSPE (Formula 47) and DSPA (Formula 48), a linker molecular moiety selected from curcumin (Formula 19), hesperetin (Formula 24) and tea polyphenol (Formula 28), and a metal ion moiety selected from $Fe^{3+}$, $Ca^{2+}$an $Al^{3+}$.

**18.** The metal-chelated phospholipid complex of claim 16 or 17, wherein the molar ratio among the phospholipid molecular moiety, the linker molecular moiety and the metal ion moiety is 1:1: (0.5-2).

**19.** The metal-chelated phospholipid complex of claim 18, wherein the molar ratio among the phospholipid molecular moiety, the linker molecular moiety, and the metal ion moiety is 1:1: 1.

**20.** The preparation method for a metal-chelated phospholipid complex of any one of claims 1 to 19, wherein the method comprises the steps of:

(1) forming a phospholipid complex by reacting and connecting a phospholipid molecule and a linker molecule; and
(2) forming a metal-chelated phospholipid complex by reacting the prepared phospholipid complex and a metal ion through a coordination bond.

**21.** The preparation method of claim 20, wherein in the step of forming the phospholipid complex, the phospholipid molecule and the linker molecule are dissolved in ethanol to react, n-hexane is added and the resultant is precipitated, so as to obtain the phospholipid complex.

**22.** The preparation method of claim 21, wherein the molar ratio of the phospholipid molecule to the linker molecule is 1: 1.

**23.** The preparation method of claim 21 or 22, wherein the reaction conditions include a reaction temperature of 65 °C and a duration of 2 hours.

**24.** The preparation method of any one of claims 20 to 23, wherein in the step of forming the metal-chelated phospholipid complex, the phospholipid complex and the metal ion are dissolved in ethanol to react and obtain the metal-chelated phospholipid complex.

**25.** The preparation method of claim 24, wherein the molar ratio of the phospholipid complex to the metal ion is 1: (1-2).

**26.** The preparation method of claim 24 or 25, wherein the reaction conditions include a reaction temperature of 60 °C and a duration of 2 hours.

**27.** A metal-chelated phospholipid complex nanoparticle, wherein the metal-chelated phospholipid complex nanoparticle comprises:

(i) a metal-chelated phospholipid complex, wherein the metal-chelated phospholipid complex is a metal-chelated phospholipid complex of any one of claims 1 to 19;
(ii) a particle aggregation-inhibiting conjugated lipid, wherein the particle aggregation-inhibiting conjugated lipid is not a cationic lipid or an ionizable lipid; and
(iii) a non-cationic lipid or a non-ionizable lipid other than the metal-chelated phospholipid complex and the particle aggregation-inhibiting conjugated lipid.

**28.** The metal-chelated phospholipid complex nanoparticle of claim 27, wherein the particle aggregation-inhibiting conjugated lipid comprises PEG-lipid conjugate and/or PEG-DAA.

**29.** The metal-chelated phospholipid complex nanoparticle of claim 28, wherein the PEG-lipid conjugate is selected from the group consisting of

phosphatidyl ethanolamine-polyethylene glycol 2000 (Formula 42)

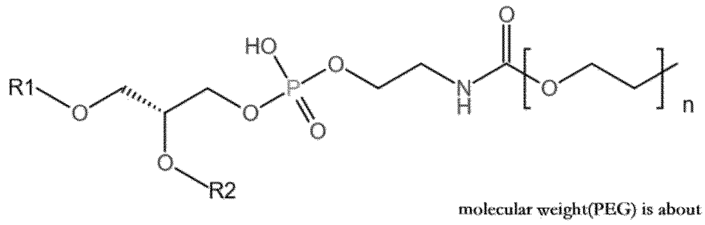

molecular weight(PEG) is about 2000

,

phosphatidyl ethanolamine-polyethylene glycol 700 (Formula 43)

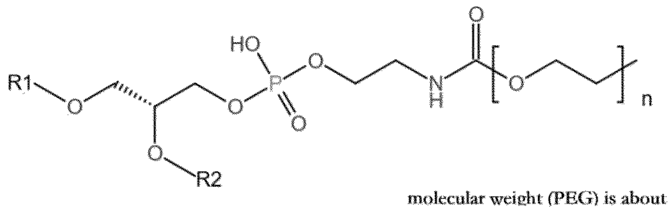

molecular weight (PEG) is about 700

,

phosphatidyl ethanolamine-polyethylene glycol 1000 (Formula 44)

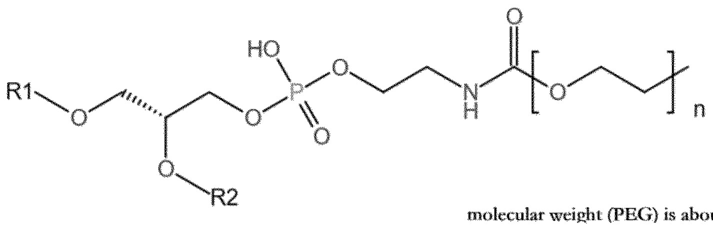

molecular weight (PEG) is about 1000

,

phosphatidyl ethanolamine-polyethylene glycol 5000 (Formula 45)

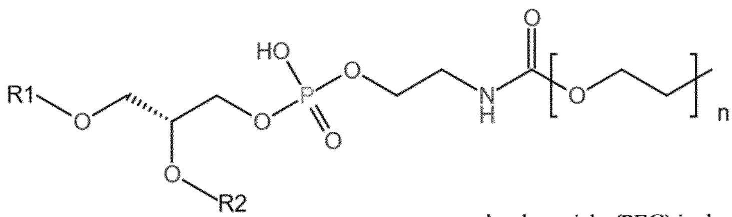

molecular weight (PEG) is about 5000

,

derivatives thereof, and combinations thereof;

R1, R2 are each independently decanoyl, lauroyl, myristoyl, palmitoyl, stearoyl, oleoyl, linoleoyl, erucyl, arachidoyl or phytanoyl.

**30.** The metal-chelated phospholipid complex nanoparticle of claim 29, wherein the PEG-lipid conjugate is selected from the group consisting of DSPE-PEG2000, DSPE-PEG700, DSPE-PEG1000, and DSPE-PEG 5000, and combination thereof.

**31.** The metal-chelated phospholipid complex nanoparticle of claim 30, wherein the PEG-lipid conjugate is selected from the group consisting of

DSPE-PEG2000 (Formula 53)

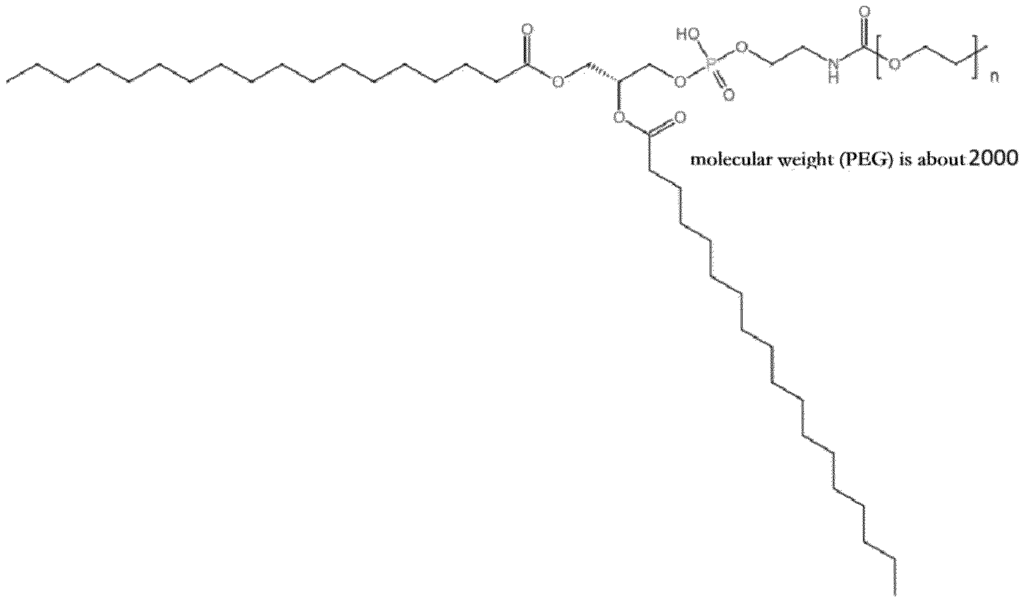


,

DSPE-PEG700 (Formula 50)

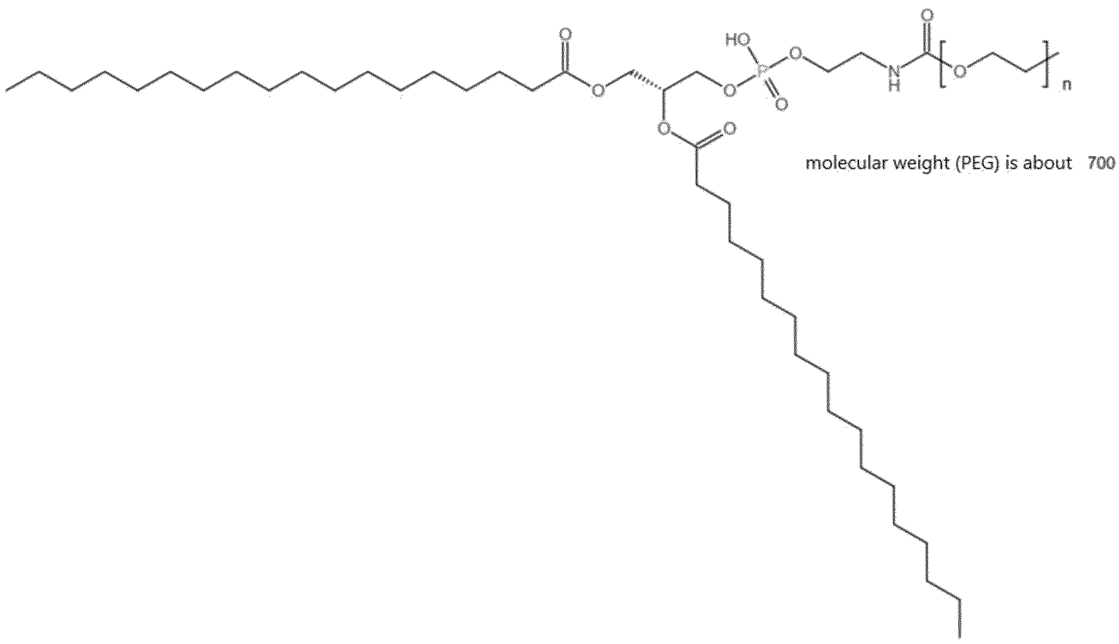


,

DSPE-PEG1000 (Formula 51)

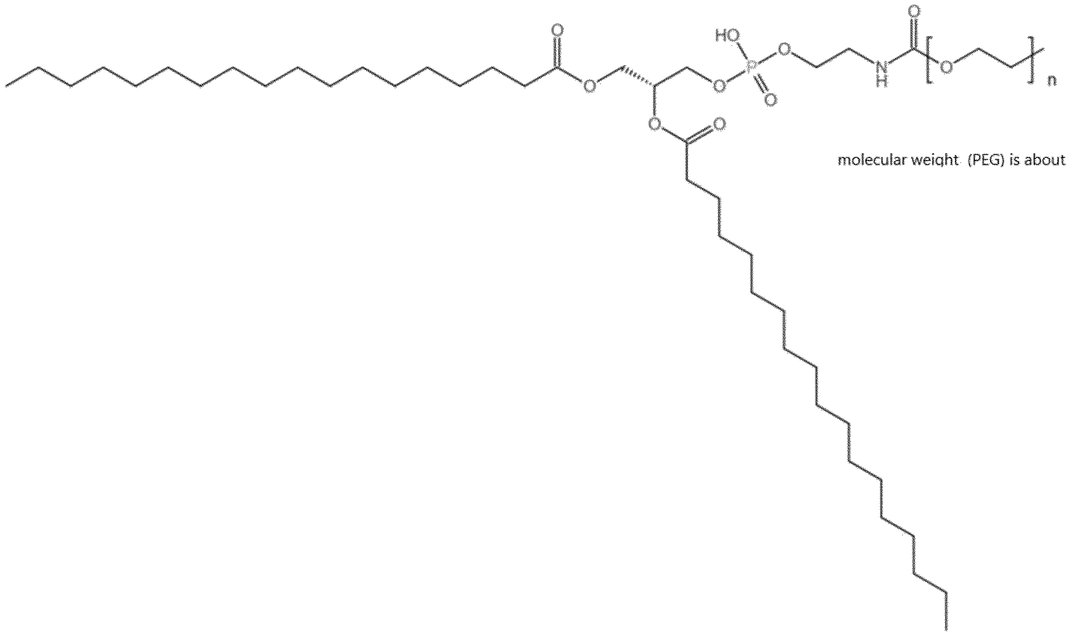

molecular weight (PEG) is about 1000

,

and

DSPE-PEG5000 (Formula 52)

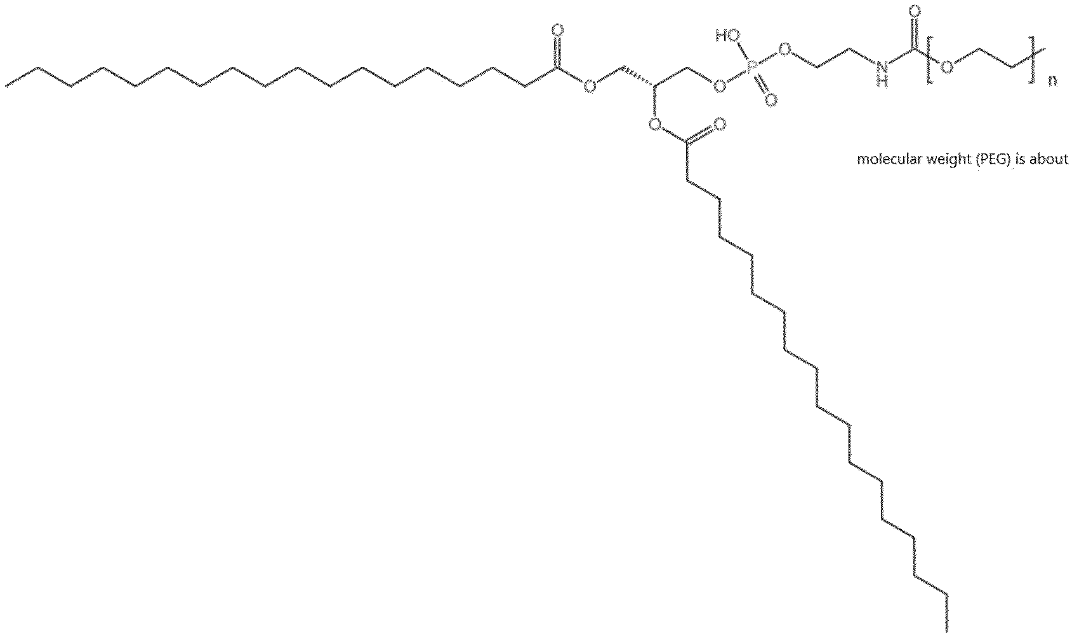

molecular weight (PEG) is about 5000

.

**32.** The metal-chelated phospholipid complex nanoparticle of any one of claims 27 to 31, wherein the non-cationic lipid or the non-ionizable lipid of (iii) is selected from cholesterol, its derivatives, and combinations thereof.

**33.** The metal-chelated phospholipid complex nanoparticle of claim 32, wherein the non-cationic lipid or the non-ionizable lipid in (iii) is cholesterol (Formula 40)

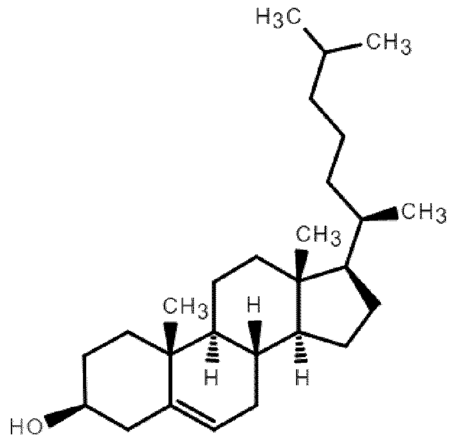

.

**34.** The metal-chelated phospholipid complex nanoparticle of claim 32, wherein the non-cationic lipid or the non-ionizable lipid of (iii) further comprises one or more selected from the group consisting of lecithin PC, phosphatidyl ethanolamine PE, phosphatidyl serine PS, phosphatidic acid PA, phosphatidyl glycerol PG, ceramide-1-phosphate CP, phosphatidyl inositol PI, phosphatidyl threonine PT, sphingomyelin SM, lysolecithin LPC, lysophosphatidyl ethanolamine LPE, lysophosphatidylserine LPS, lysophosphatidic acid LPA, lysophosphatidyl glycerol LPG, lysophosphatidylinositol LPI, lysophosphatidylthreonine LPT, lysosphingomyelin LSM, sphingosine 1-phosphate S1P, cholesterol sulfate, and derivatives thereof.

**35.** The metal-chelated phospholipid complex nanoparticle of claim 34, wherein the non-cationic lipid or the non-ionizable lipid of (iii) further comprises one or more selected from the group consisting of lecithin (PC, Formula 1), phosphatidyl ethanolamine (PE, Formula 2), Phosphatidyl serine (PS, Formula 3), phosphatidic acid (PA, Formula 4), phosphatidyl glycerol (PG, Formula 5), ceramide-1-phosphate (CP, Formula 6), phosphatidyl inositol (PI, Formula 7), phosphatidyl threonine (PT, Formula 8), sphingomyelin (SM, Formula 9), lysolecithin (LPC, Formula 10), lysophosphatidyl ethanolamine (LPE, Formula 11), lysophosphatidylserine (LPS, Formula 12), lysophosphatidic acid (LPA, Formula 13), lysophosphatidyl glycerol (LPG, Formula 14), lysophosphatidylinositol (LPI, Formula 15), lysophosphatidylthreonine (LPT, Formula 16), lysosphingomyelin (LSM, Formula 17), sphingosine 1-phosphate (S1P, Formula 18), cholesterol sulfate

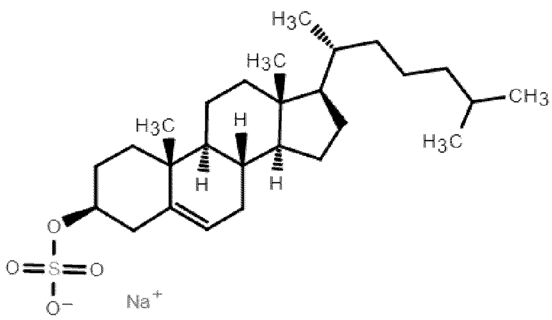

(Formula 41),

and derivatives thereof.

**36.** The metal-chelated phospholipid complex nanoparticle of claim 34 or 35, wherein the non-cationic lipid or the non-ionizable lipid of (iii) comprises cholesterol, and one or more selected from the group consisting of DSPC, DSPE, DSPA and DSPG.

**37.** The metal-chelated phospholipid complex nanoparticle of claim 36, wherein the non-cationic lipid or the non-ionizable lipid in (iii) comprises cholesterol (Formula 40) and DSPC (Formula 46).

**38.** The metal-chelated phospholipid complex nanoparticle of any one of claims 34 to 37, wherein the metal-chelated phospholipid complex nanoparticle is made from (i) a metal-chelated phospholipid complex, (ii) a particle aggregation-inhibiting conjugated lipid, and (iii) a non-cationic lipid or a non-ionizable lipid, wherein the molar proportion of

metal-chelated phospholipid complex in starting material is 10% to 40%, the molar proportion of the particle aggregation-inhibiting conjugated lipid in starting material is 2% to 10%, the molar proportion of the cholesterol in starting material is 35% to 75%, and the molar proportion of the non-cationic lipid or the non-ionizable lipid other than cholesterol in the starting material is 0% to 40%.

**39.** The metal-chelated phospholipid complex nanoparticle of any one of claims 34 or 37, wherein the metal-chelated phospholipid complex nanoparticle is made from (i) a metal-chelated phospholipid complex, (ii) a particle aggregation-inhibiting conjugated lipid, and (iii) a non-cationic lipid or a non-ionizable lipid, wherein the molar proportion of metal-chelated phospholipid complex in starting material is 5% to less than 10%, the molar proportion of the particle aggregation-inhibiting conjugated lipid in starting material is 2% to 10%, the molar proportion of the cholesterol in starting material is 15% to less than 35%, 35% to 75% or more than 75% to 80%, and the molar proportion of the non-cationic lipid or the non-ionizable lipid other than cholesterol in the starting material is 0% to 40% or more than 40% to 51%; or

the molar proportion of metal-chelated phospholipid complex in starting material is more than 40% to 50%, the molar proportion of the particle aggregation-inhibiting conjugated lipid in starting material is 2% to 10%, the molar proportion of the cholesterol in starting material is 15% to less than 35%, 35% to 75%, or more than 75% to 80%, and the molar proportion of the non-cationic lipid or the non-ionizable lipid other than cholesterol in the starting material is 0% to 40% or more than 40% to 51%; or
the molar proportion of metal-chelated phospholipid complex in starting material is 10% to 40%, the molar proportion of the particle aggregation-inhibiting conjugated lipid in starting material is 2% to 10%, the molar proportion of the cholesterol in starting material is 15% to less than 35%, or more than 75% to 80%, and the molar proportion of the non-cationic lipid or the non-ionizable lipid other than cholesterol in the starting material is 0% to 40% or more than 40% to 51%.

**40.** The metal-chelated phospholipid complex nanoparticle of claim 38 or 39, wherein the molar proportion of the metal-chelated phospholipid complex in starting material is 7% to less than 10%, 10% to 30%, or 20% to 30%, preferably 25%.

**41.** The metal-chelated phospholipid complex nanoparticle of claims 38 to 40, wherein the molar proportion of the particle aggregation-inhibiting conjugated lipid in starting material is 3% to 10% or 5% to 10%, preferably 10%.

**42.** The metal-chelated phospholipid complex nanoparticles of any one of claims 38 to 41, wherein the molar proportion of the cholesterol in starting material is 15% to less than 35%, 35% to 56%, or 35% to 55%, preferably 40%.

**43.** The metal-chelated phospholipid complex nanoparticle of any one of claims 38 to 42, wherein the molar proportion of the non-cationic lipid or the non-ionizable lipid other than cholesterol in starting material is 5% to 30%, 25% to 40%, greater than 40% to 45%, or 20% to 25%.

**44.** The metal-chelated phospholipid complex nanoparticles of claim 38, wherein the molar proportion of metal-chelated phospholipid complex in starting material is 15% to 25%, the molar proportion of the particle aggregation-inhibiting conjugated lipid in starting material is 4% to 10%, the molar proportion of the cholesterol in starting material is 40% to 46%, and the molar proportion of DSPC in the starting material is 25% to 35%, and the metal ion moiety in the metal-chelated phospholipid complex is $Fe^{3+}$.

**45.** The metal-chelated phospholipid complex nanoparticle of claim 44, wherein the molar proportion of metal-chelated phospholipid complex in starting material is 15%, the molar proportion of the particle aggregation-inhibiting conjugated lipid in starting material is 4%, the molar proportion of the cholesterol in starting material is 46%, and the molar proportion of DSPC in the starting material is 35%; or wherein the molar proportion of metal-chelated phospholipid complex in starting material is 25%, the molar proportion of the particle aggregation-inhibiting conjugated lipid in starting material is 10%, the molar proportion of the cholesterol in starting material is 40%, and the molar proportion of DSPC in the starting material is 25%; the metal ion part in the metal-chelated phospholipid complex is $Fe^{3+}$.

**46.** The metal-chelated phospholipid complex nanoparticle of claim 38, wherein the molar proportion of metal-chelated phospholipid complex in starting material is 10% to 30%, the molar proportion of the particle aggregation-inhibiting conjugated lipid in starting material is 3% to 10%, the molar proportion of the cholesterol in starting material is 35% to 56%, and the molar proportion of DSPC in the starting material is 34% to 40%, and the metal ion moiety in the metal-chelated phospholipid complex is $Al^{3+}$.

**47.** The metal-chelated phospholipid complex nanoparticle of claim 39, wherein the molar proportion of metal-chelated phospholipid complex in starting material is 10% to 30%, the molar proportion of the particle aggregation-inhibiting conjugated lipid in starting material is 3% to 10%, the molar proportion of the cholesterol in starting material is 35% to 56%, and the molar proportion of DSPC in the starting material is 40% to 45%; or

the molar proportion of metal-chelated phospholipid complex in starting material is 10% to 30%, the molar proportion of the particle aggregation-inhibiting conjugated lipid in starting material is 3% to 10%, the molar proportion of the cholesterol in starting material is 15% to 35%, and the molar proportion of DSPC in the starting material is 34% to 40% or more than 40% to 45%; or
the molar proportion of metal-chelated phospholipid complex in starting material is 7% to less than 10%, the molar proportion of the particle aggregation-inhibiting conjugated lipid in starting material is 3% to 10%, the molar proportion of the cholesterol in starting material is 15% to less than 35% or 35% to 56%, and the molar proportion of DSPC in the starting material is 34% to 40% or more than 40% to 45%;
the metal ion part in the metal-chelated phospholipid complex is $Al^{3+}$.

**48.** The metal-chelated phospholipid complex nanoparticle of claim 47, wherein the molar proportion of metal-chelated phospholipid complex in starting material is 7%, the molar proportion of the particle aggregation-inhibiting conjugated lipid in starting material is 3%, the molar proportion of the cholesterol in starting material is 56%, the molar proportion of DSPC in the starting material is 34%, and the metal ion moiety in the metal-chelated phospholipid complex is $Al^{3+}$.

**49.** A preparation method for a metal-chelated phospholipid complex nanoparticle of any one of claims 27 to 48, wherein the preparation method comprises mixing (i) a metal-chelated phospholipid complex, (ii) a particle aggregation-inhibiting conjugated lipid, and (iii) a non-cationic lipid or a non-ionizable lipid other than the metal-chelated phospholipid complex and the particle aggregation-inhibiting conjugated lipid to obtain the metal-chelated phospholipid complex nanoparticle.

**50.** A drug-lipid particle, wherein the drug-lipid particle comprises:

(a) a drug being a negatively charged molecule; and
(b) the metal-chelated phospholipid complex nanoparticle of any one of claims 27 to 48.

**51.** The drug-lipid particle of claim 50, wherein the drug is encapsulated in the metal-chelated phospholipid complex nanoparticles.

**52.** The drug-lipid particle of claim 50 or 51, wherein the drug is selected from the group consisting of nucleic acid, protein, polypeptide, small molecule, nucleic acid analog, protein analog, polypeptide analog, and combinations thereof.

**53.** The drug-phospholipid particle of claim 52, wherein the nucleic acid is selected from the group consisting of mRNA, siRNA, sgRNA, ASO, circRNA, microRNA, DNA, ecDNA, artificial nucleic acid, and combinations thereof.

**54.** The drug-phospholipid particle of claim 52 or 53, wherein the nucleic acid is an mRNA sequence shown in SEQ ID No.1 for encoding eGFP, an mRNA sequence shown in SEQ ID No.2 for encoding a receptor binding domain RBD of a novel coronavirus S1 subunit, an mRNA sequence shown in SEQ ID No.3 for encoding NY-ESO-1, an siRNA sequence of a Bcl-2 gene having an antisense strand shown in SEQ ID No.4 and a sense strand shown in SEQ ID No.21, an siRNA sequence of a PLK1 gene having an antisense strand shown in SEQ ID No.6 and a sense strand showed in SEQ ID No.23, an siRNA sequence of A Gal-1 gene shown in SEQ ID No.8, an ASO sequence of a STAT-3 gene shown in SEQ ID No.10, an ASO sequence of an alpha-syn gene shown in SEQ ID No.12, an ASO sequence of a Bcl-2 gene shown in SEQ ID No.14, an mRNA sequence shown in SEQ ID No.16 for encoding a wild-type novel coronavirus S protein, a double-stranded DNA sequence having an antisense strand shown in SEQ ID No.17 and a sense strand shown in SEQ ID No.25, a single-stranded DNA shown in SEQ ID No.18, or a siRNA sequence of a B7-H4 gene having a sense strand shown in SEQ ID No.19 and an antisense strand shown in SEQ ID No. 26.

**55.** A preparation method for a drug-lipid particle of any one of claims 50 to 54, wherein a drug is encapsulated in the metal-chelated phospholipid complex nanoparticle to obtain the drug-lipid particle.

**56.** The preparation method of claim 55, wherein the metal-chelated phospholipid complex, the particle aggregation-inhibiting conjugated lipid, and the non-cationic lipid or the non-ionizable lipid other than the metal-chelated phospholipid complex and the particle aggregation-inhibiting conjugated lipid are dissolved in an organic compound

to form an organic phase, the drug is dissolved in a buffer to form an aqueous phase, and the organic phase is mixed with the aqueous phase to obtain the drug-lipid particle.

**57.** The preparation method of claim 56, wherein the organic compound is ethanol.

**58.** The preparation method of claim 56 or 57, wherein the buffer is an enzyme-free PBS buffer.

**59.** The preparation method of any one of claims 56 to 58, wherein mixing means of the organic phase and the aqueous phase includes micro-fluidic chip or ultrasound.

**60.** Use of a metal-chelated phospholipid complex of any one of claims 1 to 19 in a nucleic acid delivery system.

**61.** The use of claim 60, wherein the nucleic acid delivery system is used for introducing a nucleic acid into a cell.

**62.** The use of claim 61, wherein the nucleic acid is used for silencing expression of a target sequence in a mammalian subject, for delivering a drug in a mammal body, for delivering a drug into mammalian cells from the body, or for treating a disease or disorder in a mammal.

**63.** Use of the metal-chelated phospholipid complex nanoparticle of any one of claims 27 to 48 or the drug-lipid particle of any one of claims 47 to 51 in a composition for the delivery of a drug.

**64.** The use of claim 63, wherein the composition is used for introducing a drug into a cell.

**65.** The use of claim 63 or 64, wherein the composition is a medicament.

**66.** The use of claim 65, wherein the medicament is used for silencing expression of a target sequence in a mammalian subject, for delivering a drug in a mammal body, for delivering a drug into mammalian cells from the body, or for treating a disease or disorder in a mammal.

**67.** The use of claim 62 or 66, wherein the mammal is a human.

**68.** The use of claim 62 or 66, wherein the disease or disorder is associated with expression of a gene comprising a target sequence for a drug.

**69.** The use of claim 62 or 66, wherein the disease or disorder comprises cancer, a viral infection, an autoimmune disease, diabetes, and Alzheimer's disease.

**70.** The use of claim 69, wherein the viral infection comprises hepatitis A, hepatitis B, hepatitis C, SARS-Cov-2, HIV, HPV, influenza, smallpox, and syphilis.

**71.** The use of claim 69, wherein the cancer comprises liver cancer, glioma, melanoma, lung cancer, pancreatic cancer, and breast cancer.

**72.** The use of claim 65, wherein the medicament is a vaccine.

**73.** The use of claim 65 or 72, wherein the route of administration of the medicament comprises intrathecal injection, intramuscular injection, intracranial injection, intravenous injection, and intratumoral injection.

**74.** A medicament containing a metal-chelated phospholipid complex of any one of claims 1 to 19 or a metal-chelated phospholipid complex nanoparticle of any one of claims 27 to 48 or a drug-lipid particle of any one of claims 50 to 54.

**75.** The medicament of claim 74, wherein the medicament is a vaccine.

**76.** The medicament of claim 75, wherein the vaccine is a novel coronavirus vaccine.

**77.** Use of the metal-chelated phospholipid complex of any one of claims 1 to 19 or the metal-chelated phospholipid complex nanoparticle of any one of claims 27 to 48 or the drug-lipid particle of any one of claims 50 to 54 or the medicament of any one of claims 74 to 76 in prevention/treatment of a disease or disorder in a mammal.

**78.** The use of claim 77, wherein said mammal is a human.

**79.** The use of claim 77, wherein the disease or disorder is associated with expression of a gene comprising a target sequence for a drug.

**80.** The use of claim 77, wherein the disease or disorder comprises cancer, a viral infection, an autoimmune disease, diabetes, and Alzheimer's disease.

**81.** The use of claim 80, wherein the viral infection comprises hepatitis A, hepatitis B, hepatitis C, SARS-Cov-2, HIV, HPV, influenza, smallpox, and syphilis.

**82.** The use of claim 80, wherein the cancer comprises liver cancer, glioma, melanoma, lung cancer, pancreatic cancer, and breast cancer.

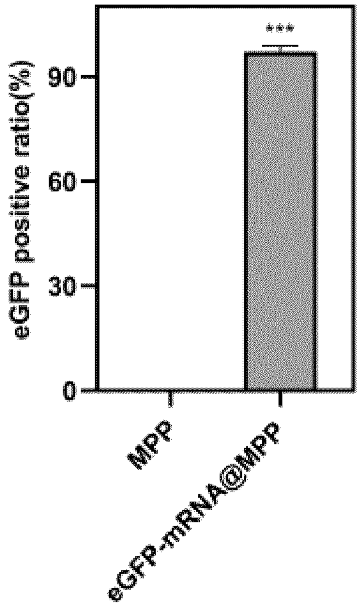
eGFP positive ratio(%)
90
60
30
0
***
MPP
eGFP-mRNA@MPP

Fig. 1-1

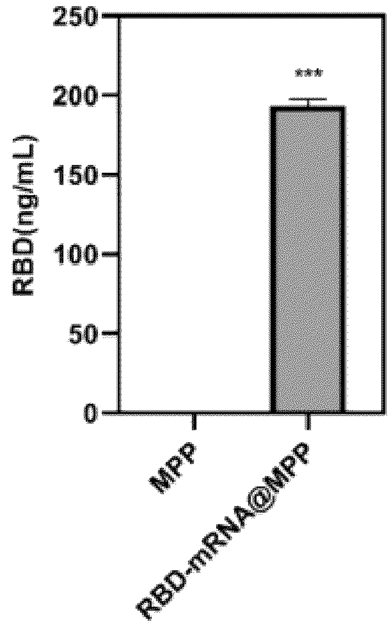
RBD(ng/mL)
250
200
150
100
50
0
***
MPP
RBD-mRNA@MPP

Fig. 1-2

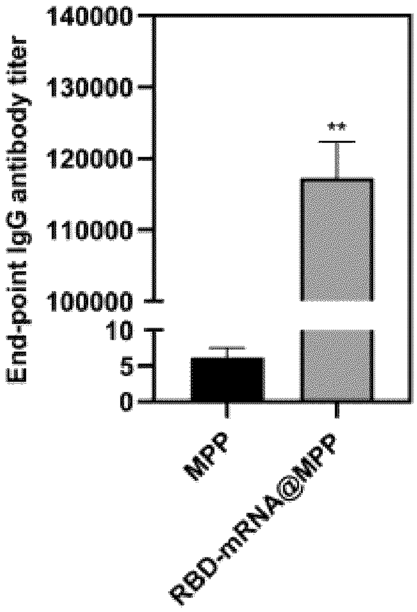
End-point IgG antibody titer
140000
130000
120000
110000
100000
10
5
0
**
MPP
RBD-mRNA@MPP

Fig. 1-3

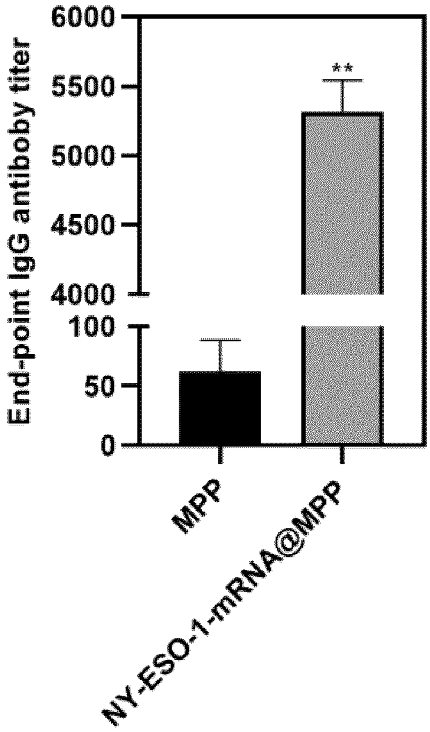
End-point IgG antibody titer
6000
5500
5000
4500
4000
100
50
0
**
MPP
NY-ESO-1-mRNA@MPP

Fig. 1-4

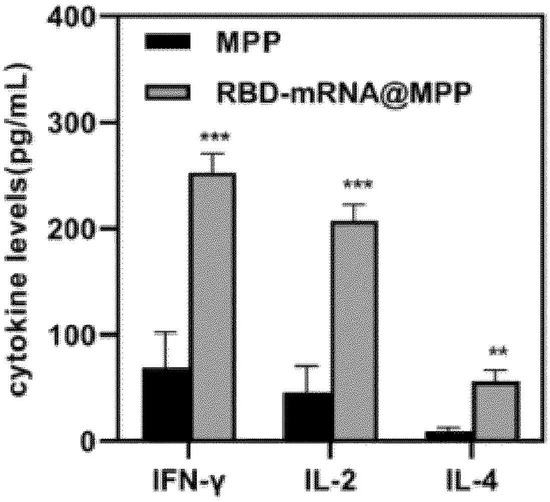
400
300
200
100
0
cytokine levels(pg/mL)
MPP
RBD-mRNA@MPP
***
***
**
IFN-γ
IL-2
IL-4

Fig. 1-5

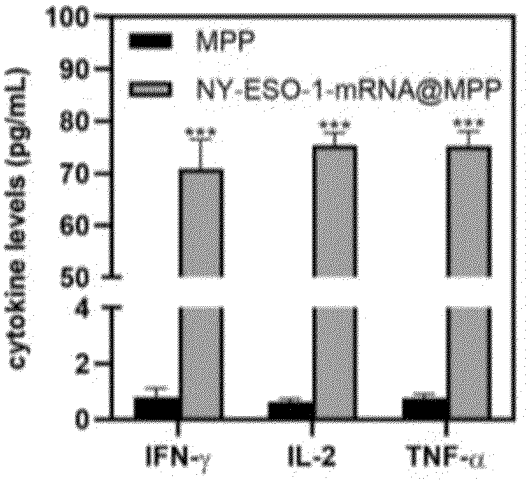
100
90
80
70
60
50
4
2
0
cytokine levels (pg/mL)
MPP
NY-ESO-1-mRNA@MPP
***
***
***
IFN-γ
IL-2
TNF-α

Fig. 1-6

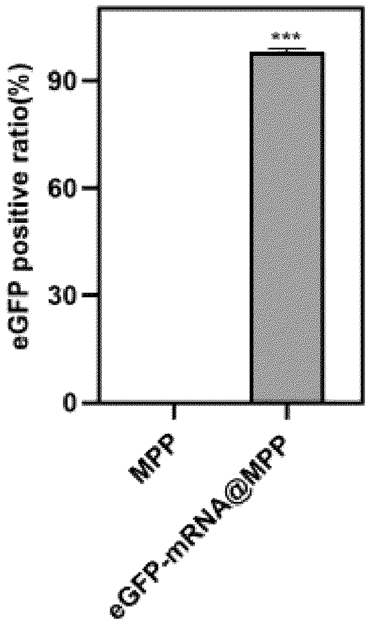
***
eGFP positive ratio(%)
0
30
60
90
MPP
eGFP-mRNA@MPP

Fig. 1-7

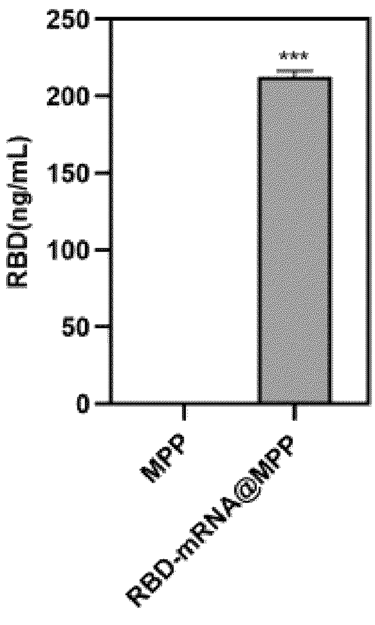
***
RBD(ng/mL)
0
50
100
150
200
250
MPP
RBD-mRNA@MPP

Fig. 1-8

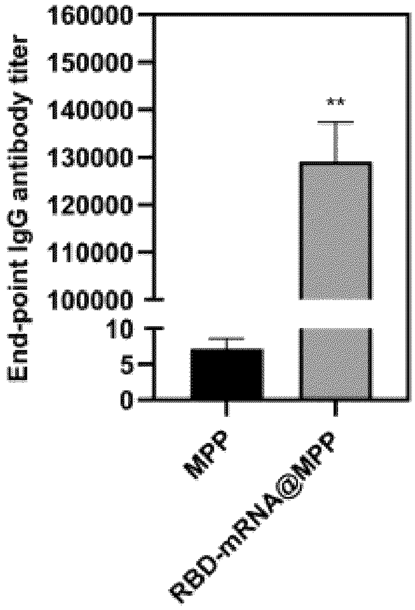


Fig. 1-9

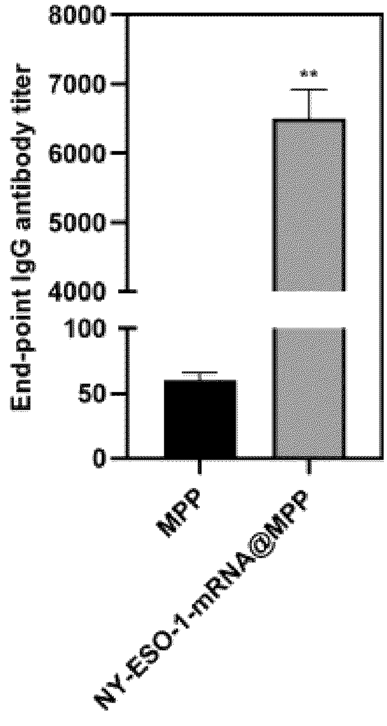


Fig. 1-10

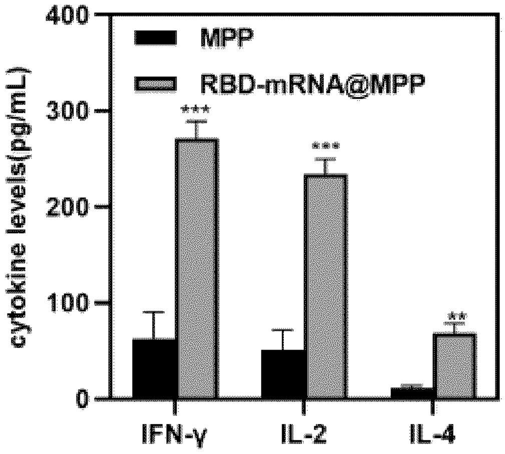


Fig. 1-11

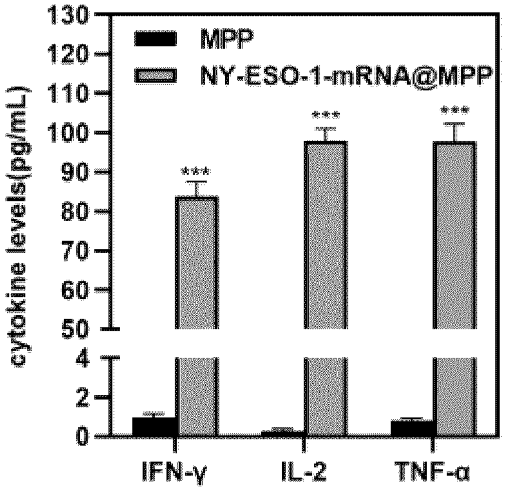


Fig. 1-12

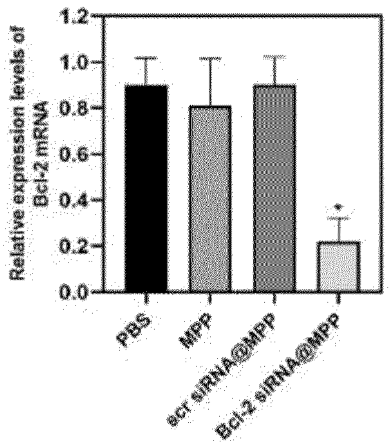


Fig. 1-13

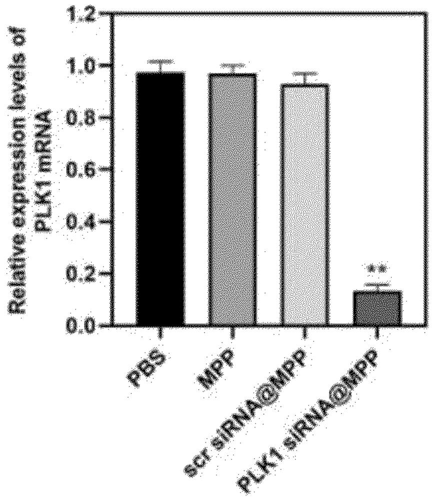


Fig. 1-14

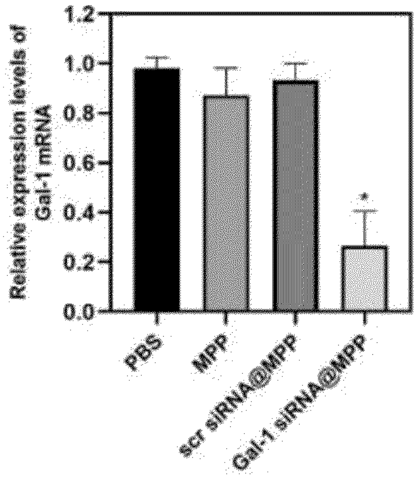


Fig. 1-15

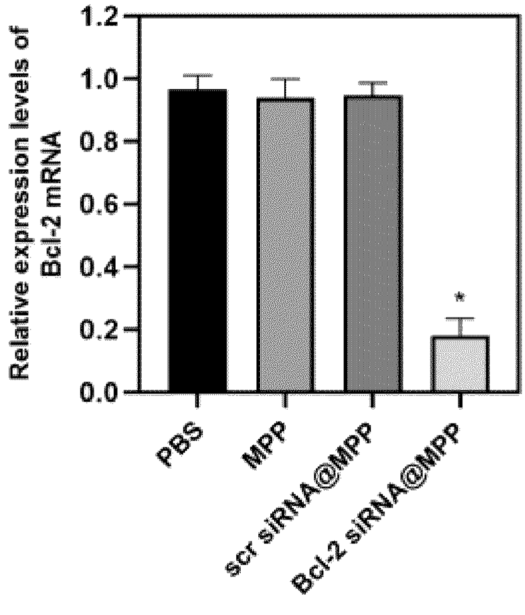


Fig. 1-16

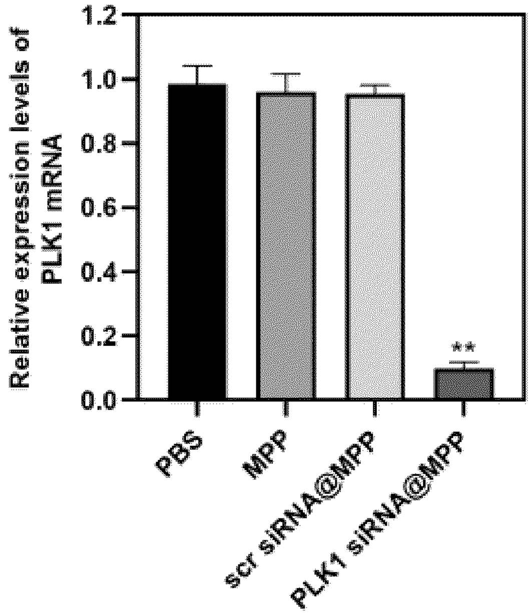


Fig. 1-17

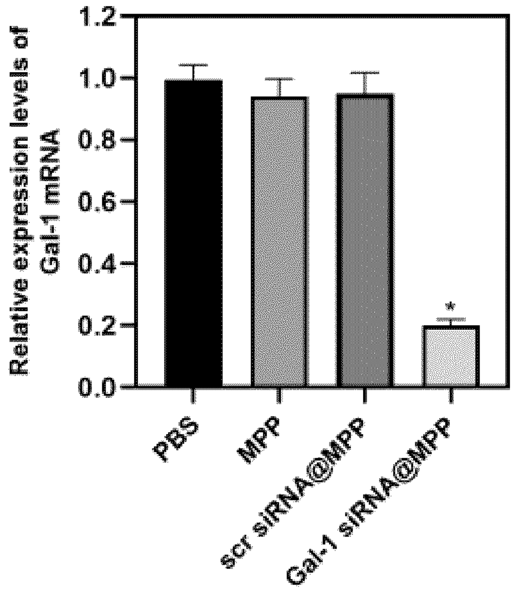


Fig. 1-18

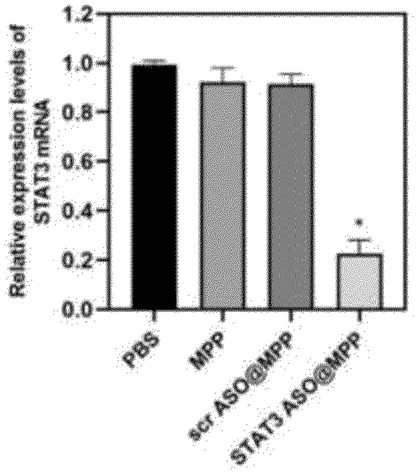


Fig. 1-19

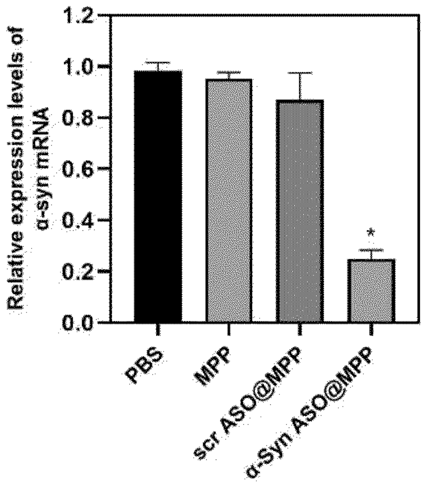


Fig. 1-20

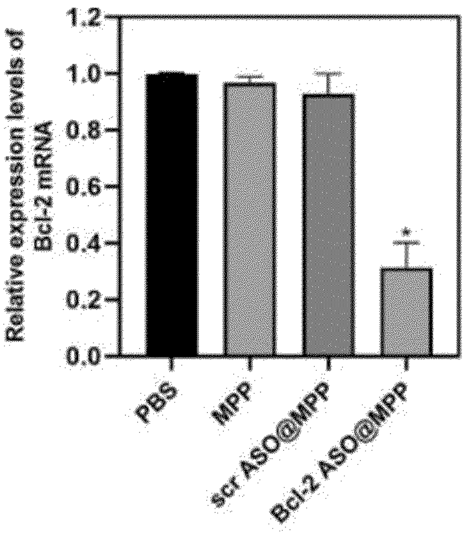


Fig. 1-21

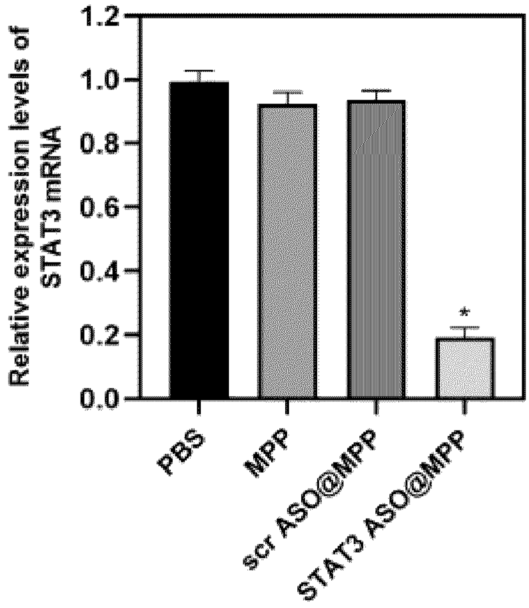


Fig. 1-22

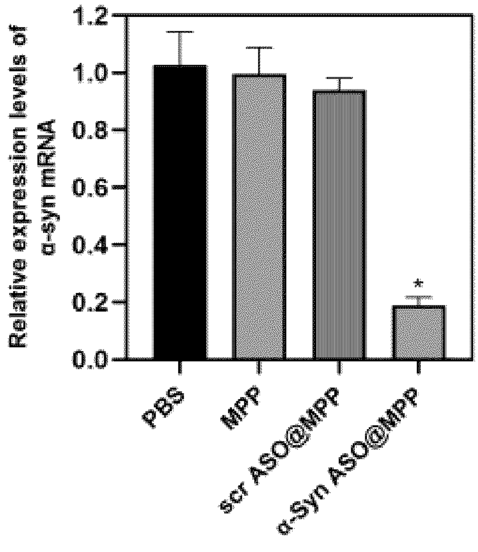


Fig. 1-23

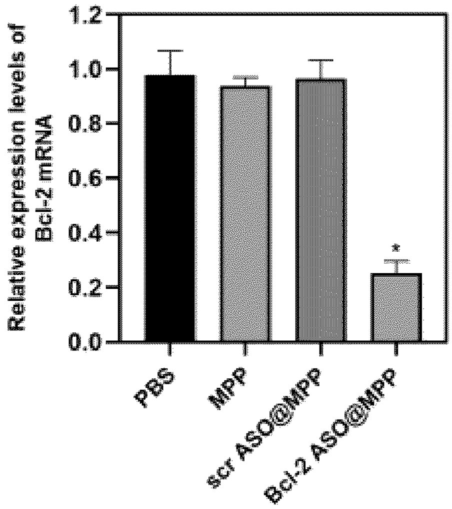


Fig. 1-24

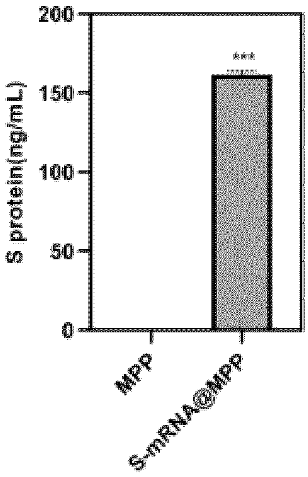


Fig. 1-25

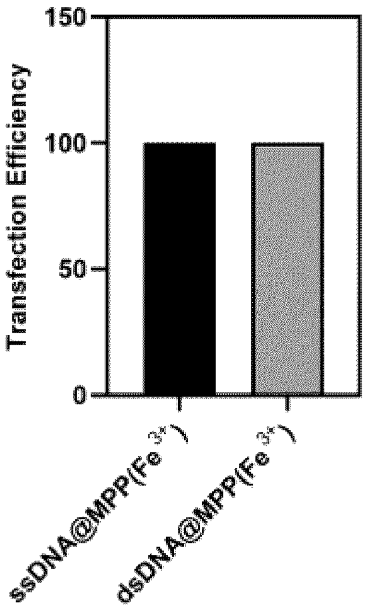


Fig. 1-26

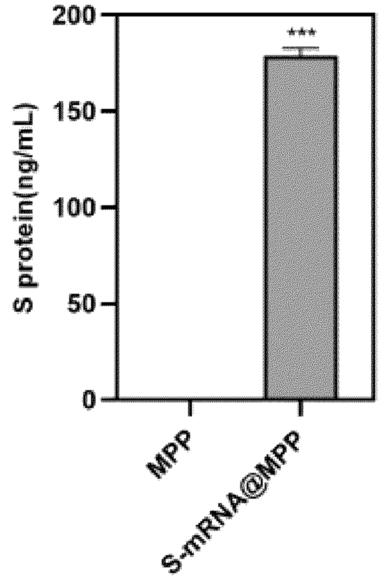


Fig. 1-27

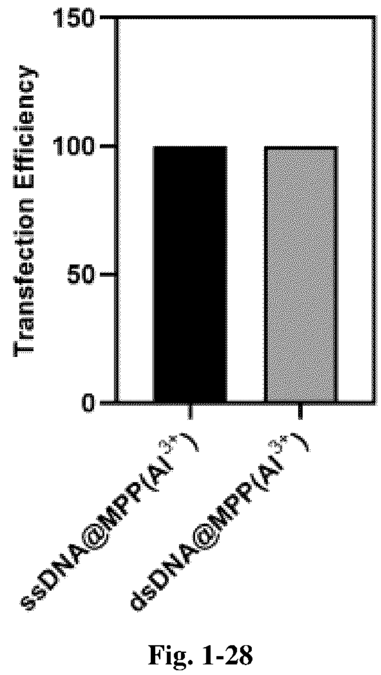


Fig. 1-28

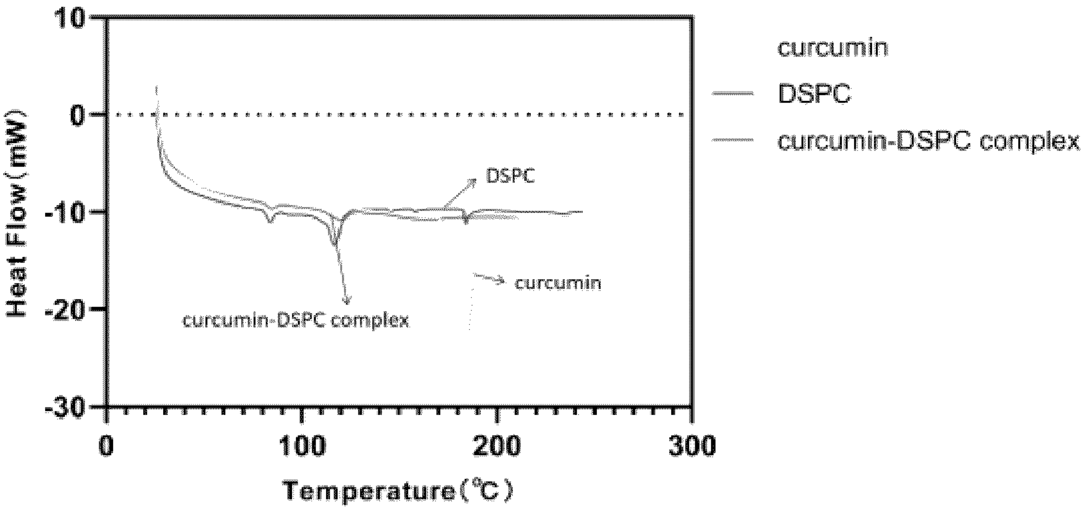
10
0
-10
-20
-30
Heat Flow（mW）
0
100
200
300
Temperature（℃）
curcumin
DSPC
curcumin-DSPC complex
DSPC
curcumin
curcumin-DSPC complex

Fig. 2-1

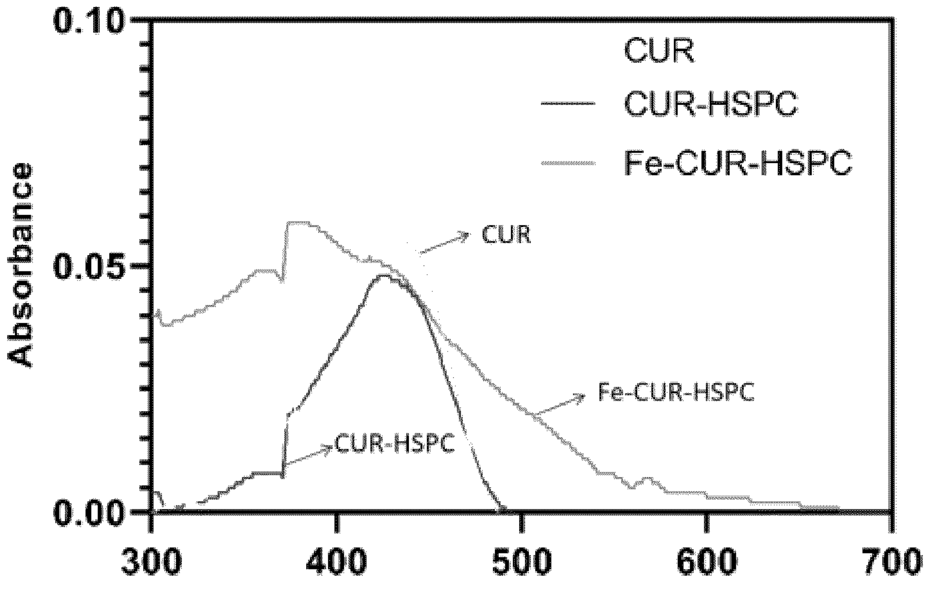
0.10
0.05
0.00
Absorbance
300
400
500
600
700
Wavelength（nm）
CUR
CUR-HSPC
Fe-CUR-HSPC
CUR
Fe-CUR-HSPC
CUR-HSPC

Fig. 2-2

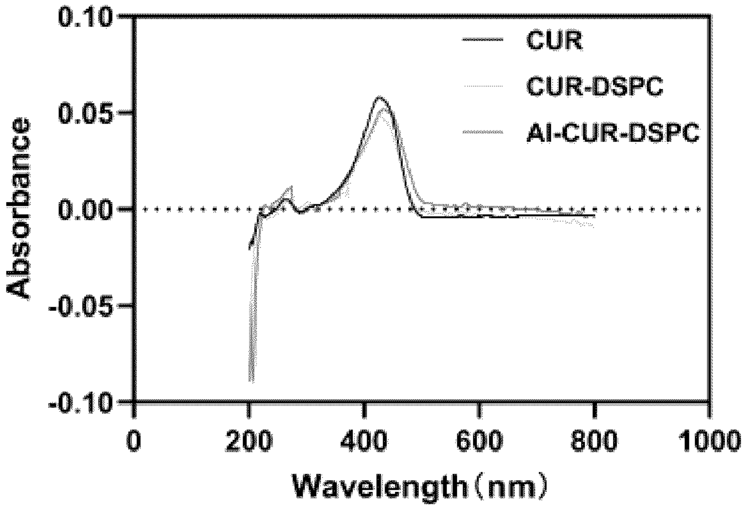


Fig. 2-3

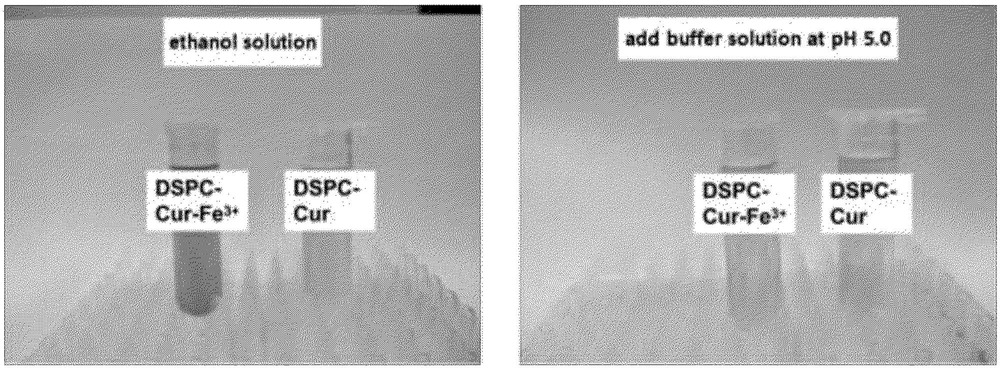


图2-4

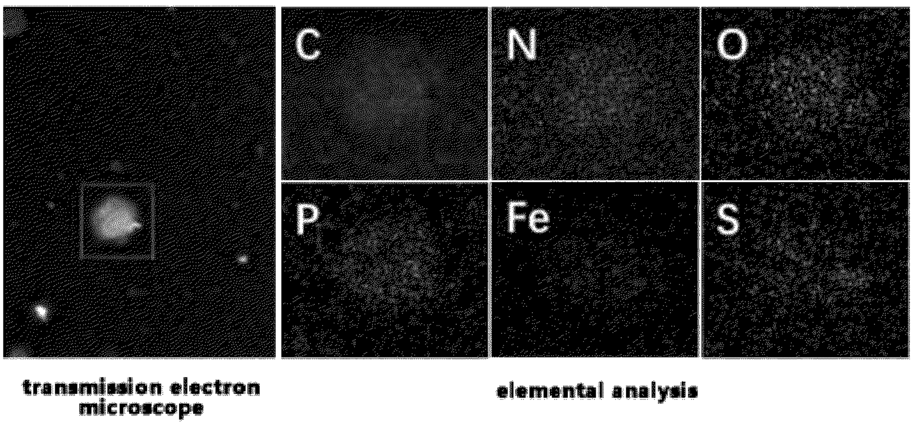


Fig. 2-5

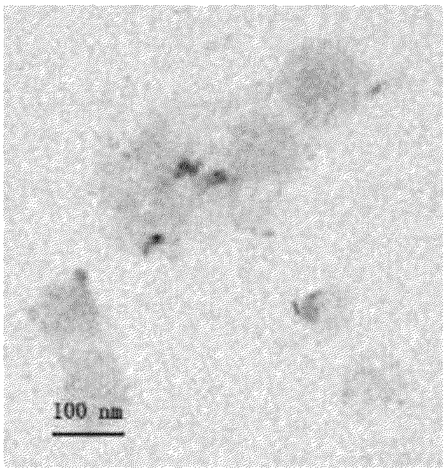
100 nm

Fig. 2-6

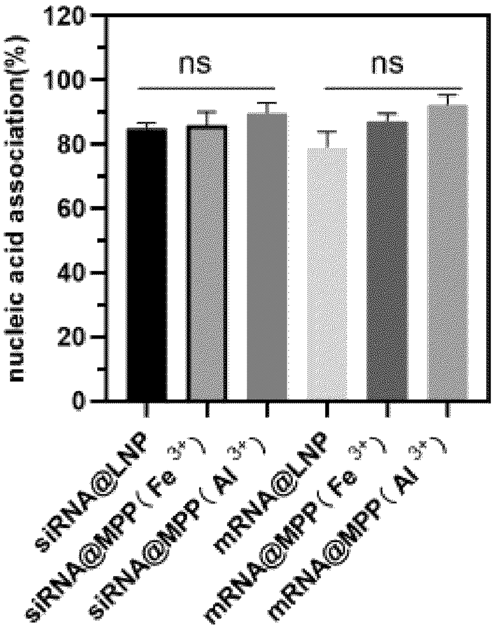
nucleic acid association(%)
120
100
80
60
40
20
0
ns
ns
siRNA@LNP
siRNA@MPP（Fe 3+）
siRNA@MPP（Al 3+）
mRNA@LNP
mRNA@MPP（Fe 3+）
mRNA@MPP（Al 3+）

Fig. 2-7

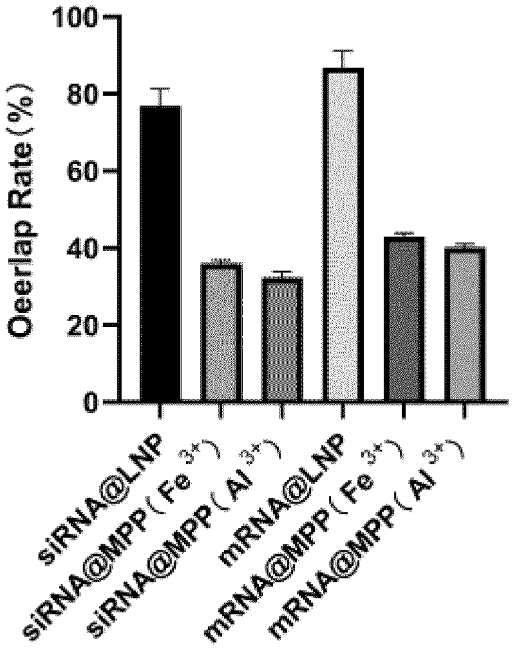
Oeerlap Rate (%)
100
80
60
40
20
0
siRNA@LNP
siRNA@MPP ( Fe 3+ )
siRNA@MPP ( Al 3+ )
mRNA@LNP
mRNA@MPP ( Fe 3+ )
mRNA@MPP ( Al 3+ )

Fig. 2-8

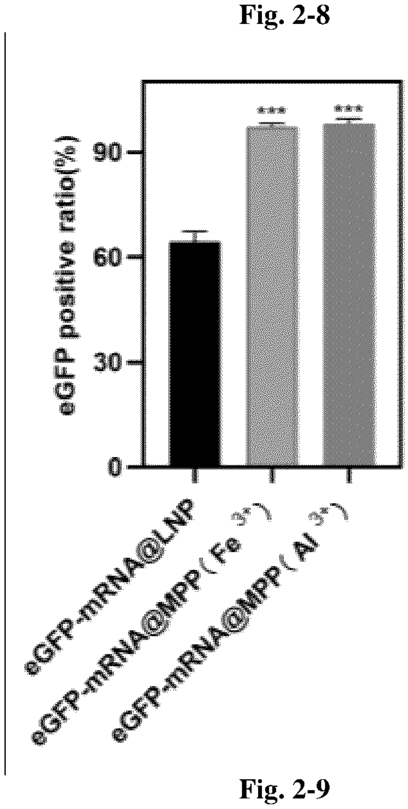
eGFP positive ratio(%)
90
60
30
0
***
***
eGFP-mRNA@LNP
eGFP-mRNA@MPP ( Fe 3+ )
eGFP-mRNA@MPP ( Al 3+ )

Fig. 2-9

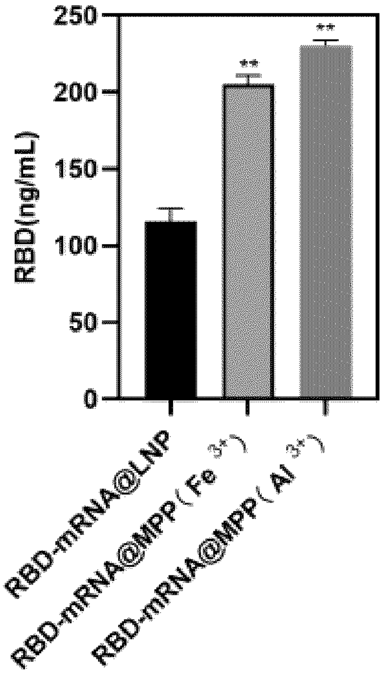


Fig. 2-10

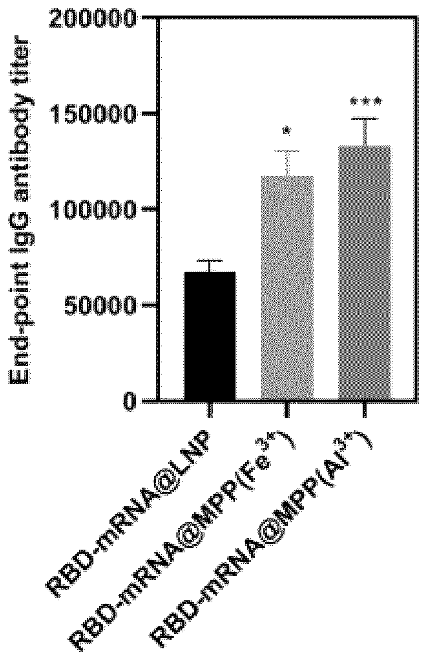


Fig. 2-11

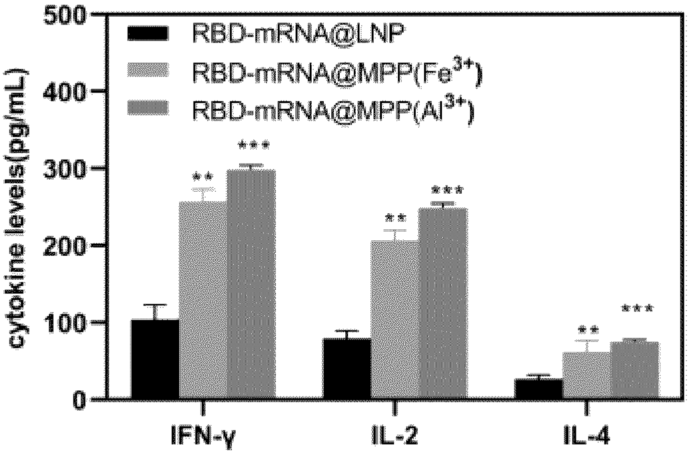


Fig. 2-12

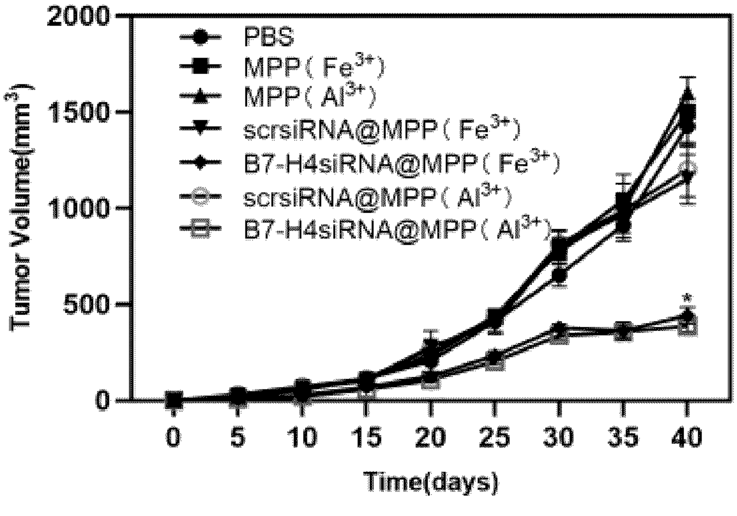


Fig. 3-1

## INTERNATIONAL SEARCH REPORT

International application No.
**PCT/CN2023/111279**

### A. CLASSIFICATION OF SUBJECT MATTER

A61K 47/54(2017.01)i； A61K9/127(2006.01)i； A61K31/12(2006.01)i； A61P35/00(2006.01)i； A61P3/10(2006.01)i； A61P31/12(2006.01)i； A61P37/00(2006.01)i； A61P25/28(2006.01)i

According to International Patent Classification (IPC) or to both national classification and IPC

### B. FIELDS SEARCHED

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT, CNABS, DWPI, WPABS, WOTXT, ENTXT, VEN, 百度, Baidu, CNKI, STNext, PUBMED, ISI WEB OF SCIENCE: 复合物, 磷脂, 磷脂复合物, 姜黄素, 橙皮素, 茶多酚, 二硬脂酰基磷脂酰胆碱, 湖南健瑞医药科技有限公司, 王珊, drug delivery carriers, Curcumin, Hesperetin, Tea polyphenol, DSPC, Phospholipid, complex phytosome

### C. DOCUMENTS CONSIDERED TO BE RELEVANT

| Category* | Citation of document, with indication, where appropriate, of the relevant passages | Relevant to claim No. |
|---|---|---|
| A | CN 112451487 A (SHENYANG PHARMACEUTICAL UNIVERSITY) 09 March 2021 (2021-03-09)<br>claims 1, 5 and 7-10, and description, paragraph 4 | 1-82 |
| A | WO 2005063213 A1 (BIODELIVERY SCIENCES INTERNATIONAL, INC.) 14 July 2005 (2005-07-14)<br>claims 1-40, and description, page 7, paragraph 4 to page 9, paragraph 2 | 1-82 |
| A | CN 101400360 A (INDENA S.P.A) 01 April 2009 (2009-04-01)<br>claims 1-6 | 1-82 |
| A | WO 2018181538 A1 (KANEKA CORP.) 04 October 2018 (2018-10-04)<br>claims 1-13, and description, paragraphs 35-36, and embodiment 7 | 1-82 |
| A | US 2022054580 A1 (STABICON LIFE SCIENCES PVT. LTD. et al.) 24 February 2022 (2022-02-24)<br>claims 1-20 | 1-82 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 November 2023** | **15 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/111279**

**C. DOCUMENTS CONSIDERED TO BE RELEVANT**

| Category* | Citation of document, with indication, where appropriate, of the relevant passages | Relevant to claim No. |
|---|---|---|
| A | MAITI, Kuntal et al. "Curcumin-phospholipid Complex: Preparation, Therapeutic Evaluation and Pharmacokinetic Study in Rats" *International Journal of Pharmaceutics,* Vol. vol. 330, 23 September 2006 (2006-09-23), pp. 155-163<br>pp. 155-163 | 1-82 |
| A | ZHANG, Bo et al. "Effect of Phosphatidylcholine on the Stability and Lipolysis of Nanoemulsion Drug Delivery Systems" *International Journal of Pharmaceutics,* Vol. vol. 583, 26 April 2020 (2020-04-26), 119354 (pages 1-9)<br>119354 (pages 1-9) | 1-82 |
| A | KUMAR Varun et al. "Preparation and Characterization of Nanocurcumin Based Hybrid Virosomes as a Drug Delivery Vehicle with Enhanced Anticancerous Activity and Reduced Toxicity" *Scientific Reports,* Vol. vol. 11, 31 December 2021 (2021-12-31), 368 (pages 1-14)<br>368 (pages 1-14) | 1-82 |
| A | MOUSSA Zeinab et al. "Interaction of Curcumin with 1,2-dioctadecanoyl-sn-glycero-3-phosphocholine Liposomes: Intercalation of Rhamnolipids Enhances Membrane Fluidity, Permeability and Stability of Drug Molecule" *Colloids and Surfaces B: Biointerfaces,* Vol. vol. 149, 03 October 2016 (2016-10-03), pp. 30-37<br>pp. 30-37 | 1-82 |
| A | KALITA Bhupen et al. "Formulation and In Vitro Evaluation of Hesperidin-Phospholipid Complex and its Antioxidant Potential" *Current Drug Therapy,* Vol. 15, No. (1), 31 December 2020 (2020-12-31), pp. 28-36<br>pp. 28-36 | 1-82 |
| A | 任金妹等 (REN, Jinmei et al.). "提高姜黄素口服生物利用度方法的研究进展 (Non-official translation: Research Progress on Method for Improving Oral Bioavailability of Curcumin)" *中国药房 (C鯿na Pharmacy),* Vol. 29, No. (23), 31 December 2018 (2018-12-31), pp. 3303-3308<br>pp. 3303-3308 | 1-82 |

Form PCT/ISA/210 (second sheet) (July 2022)

**INTERNATIONAL SEARCH REPORT**

International application No.
**PCT/CN2023/111279**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **60-73, 77-82**
because they relate to subject matter not required to be searched by this Authority, namely:

Claims 60-73 relate to the use of a metal-phospholipid complex in a nucleic acid delivery system, and claims 77-82 relate to the use of a metal-phospholipid complex, a metal-phospholipid complex particle, a drug-lipid particle or a medicament in the prevention/treatment of a disease or condition in a mammal. Said claims do not comply with PCT Rule 39.1(iv). The basis of this report is to amend the designation of the subject matter thereof to: the use of a metal-phospholipid complex in the preparation of a drug for a nucleic acid delivery system, or the use of a metal-phospholipid complex, a metal-phospholipid complex particle, a drug-lipid particle or a medicament in the preparation of a drug for preventing/treating a disease or condition in a mammal.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/111279**

| Patent document cited in search report | | | Publication date (day/month/year) | Patent family member(s) | | | Publication date (day/month/year) |
|---|---|---|---|---|---|---|---|
| CN | 112451487 | A | 09 March 2021 | None | | | |
| WO | 2005063213 | A1 | 14 July 2005 | None | | | |
| CN | 101400360 | A | 01 April 2009 | AU | 2007222667 | A1 | 13 September 2007 |
| | | | | AU | 2007222667 | B2 | 08 November 2012 |
| | | | | SI | 1991244 | T1 | 29 November 2019 |
| | | | | WO | 2007101551 | A2 | 13 September 2007 |
| | | | | WO | 2007101551 | A3 | 25 October 2007 |
| | | | | CA | 2644017 | A1 | 13 September 2007 |
| | | | | CA | 2644017 | C | 30 July 2019 |
| | | | | EP | 1991244 | A2 | 19 November 2008 |
| | | | | EP | 1991244 | B1 | 02 October 2019 |
| | | | | ES | 2757580 | T3 | 29 April 2020 |
| | | | | EP | 1837030 | A1 | 26 September 2007 |
| | | | | IL | 193966 | A0 | 01 August 2011 |
| | | | | IL | 193966 | A | 29 January 2015 |
| | | | | HUE | 046597 | T2 | 30 March 2020 |
| | | | | RU | 2008136189 | A | 20 March 2010 |
| | | | | RU | 2450818 | C2 | 20 May 2012 |
| | | | | US | 2009131373 | A1 | 21 May 2009 |
| | | | | US | 10603329 | B2 | 31 March 2020 |
| | | | | PT | 1991244 | T | 29 November 2019 |
| | | | | KR | 20080112224 | A | 24 December 2008 |
| | | | | KR | 101423269 | B1 | 25 July 2014 |
| | | | | NO | 20083850 | L | 03 December 2008 |
| | | | | NO | 344242 | B1 | 14 October 2019 |
| | | | | US | 2019175628 | A1 | 13 June 2019 |
| | | | | JP | 2009529506 | A | 20 August 2009 |
| | | | | JP | 5448462 | B2 | 19 March 2014 |
| | | | | DK | 1991244 | T3 | 18 November 2019 |
| | | | | PL | 1991244 | T3 | 31 March 2020 |
| WO | 2018181538 | A1 | 04 October 2018 | None | | | |
| US | 2022054580 | A1 | 24 February 2022 | WO | 2020084634 | A1 | 30 April 2020 |
| | | | | JP | 2022509463 | A | 20 January 2022 |
| | | | | EP | 3870201 | A1 | 01 September 2021 |
| | | | | EP | 3870201 | A4 | 03 August 2022 |
| | | | | SG | 11202103782 | SA | 28 May 2021 |
| | | | | AU | 2019365596 | A1 | 15 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210951941
- CN 202210950392
- CN 202210950391
- US 5885613 A
- US 136707
- US 20030077829 A
- CA 0200669 W
- US 5820873 A
- US 6320017 B
- US 6586559 B
- US 6573099 B
- US 5962428 A
- US 5910488 A
- US 4683195 A
- US 4683202 A
- US 5543158 A
- US 5641515 A
- US 5399363 A
- US 5426039 A, Wallace


### Non-patent literature cited in the description


- **BATZER et al.** *Nucleic Acid Res.*, 1991, vol. 19, 5081
- **OHTSUKA et al.** *J. Biol.Chem.*, 1985, vol. 260, 2605-2608
- **ROSSOLINI et al.** *Mol.Cell.Probes*, 1994, vol. 8, 91-98
- **BRUMMELKAMP et al.** *Science*, 2002, vol. 296, 550
- **DONZÉ**. *Nucleic AcidsRes.*, 2002, vol. 30, e46
- **PADDISON et al.** *Genes Dev.*, 2002, vol. 16, 948
- **YU et al.** *Proc.Natl.Acad.Sci.*, 2002, vol. 99, 6047
- **LEE et al.** *Nat.Biotech.*, 2002, vol. 20, 500
- **MIYAGISHI et al.** *Nat.Biotech.*, 2002, vol. 20, 497
- **PAUL et al.** *Nat.Biotech.*, 2002, vol. 20, 505
- **SUI et al.** *Proc.Natl.Acad.Sci.*, 2002, vol. 99, 5515
- **ELBASHIR et al.** *Genes Dev.*, 2001, vol. 15, 188
- **NYKÄNEN et al.** *Cell*, 2001, vol. 107, 309
- **GUBLER** ; **HOFFMAN**. *Gene*, 1983, vol. 25, 263-269
- PCR Protocols: A Guide to methods and Applications. 1990
- **SAMBROOK et al.** Molecular Cloning, A Laboratory Manual. 1989
- **KRIEGLER**. Gene Transfer and Expression: A Laboratory Manual. 1990
- Current Protocols in Molecular Biology. 1994
- **HAMASAKI et al.** *FEBS Lett.*, 2003, vol. 543, 51
- **YOKOTA et al.** *EMBO Rep.*, 2003, vol. 4, 602
- **SCHLOMAI et al.** *Hepatology*, 2003, vol. 37, 764
- **WILSON et al.** *Proc.Natl.Acad.Sci.*, 2003, vol. 100, 2783
- **KAPADIA et al.** *Proc.Natl.Acad.Sci.*, 2003, vol. 100, 2014
- FIELDSVIROLOGY. 2001
- **BANERJEA et al.** *Mol Ther.*, 2003, vol. 8, 62
- **SONG et al.** *J.Virol.*, 2003, vol. 77, 7174
- **STEPHENSON**. *JAMA*, 2003, vol. 289, 1494
- **QIN et al.** *Proc.Natl.Acad.Sci.*, 2003, vol. 100, 183
- **JIA et al.** *J.Virol.*, 2003, vol. 77, 3301
- **HALL et al.** *J.Virol.*, 2003, vol. 77, 6066
- **JIANG et al.** *Oncogene*, 2002, vol. 21, 6041
- *FIELDS VIROLOGY*, 2001
- **FORMAN et al.** *Cell*, 1995, vol. 81, 687
- **SEOL et al.** *Mol.Endocrinol.*, 1995, vol. 9, 72
- **ZAVACKI et al.** *PNAS USA*, 1997, vol. 94, 7909
- **SAKAI et al.** *Cell*, 1996, vol. 85, 1037-1046
- **DUNCAN et al.** *J.Biol.Chem.*, 1997, vol. 272, 12778-12785
- **WILLY et al.** *Genes Dev.*, 1995, vol. 9 (9), 1033-45
- **LEHMANN et al.** *J.Biol.Chem.*, 1997, vol. 272 (6), 3137-3140
- **JANOWSKI et al.** *Nature*, vol. 383, 728-731
- **PEET et al.** *Cell*, 1998, vol. 93, 693-704
- **WILDA et al.** *Oncogene*, 2002, vol. 21, 5716
- **SCHERR et al.** *Blood*, vol. 101, 1566
- **HEIDENREICH et al.** *Blood*, 2003, vol. 101, 3157
- **NIETH et al.** *FEBS Lett.*, 2003, vol. 545, 144
- **WU et al.** *Cancer Res.*, 2003, vol. 63, 1515

- **LI et al.** *Cancer Res.*, 2003, vol. 63, 3593
- **ZOU et al.** *Genes Dev.*, 2002, vol. 16, 2923
- **VERMA et al.** *Clin Cancer Res.*, 2003, vol. 9, 1291
- **KOSCIOLEK et al.** *Mol Cancer Ther.*, 2003, vol. 2, 209
- **NAGY**. *Exp. Cell Res.*, 2003, vol. 285, 39
- **TUSCHL** ; **BORKHARDT**. *Mol. Interventions*, 2002, vol. 2, 158
- **COLLIS et al.** *Cancer Res.*, 2003, vol. 63, 1550
- **REICH et al.** *Mol. Vis.*, 2003, vol. 9, 210
- **HILL et al.** *J.Immunol.*, 2003, vol. 171, 691
- **SONG et al.** *Nat. Med.*, 2003, vol. 9, 347
- **HEINONEN et al.** *FEBS lett.*, 2002, vol. 527, 274
- **CAPLEN et al.** *Hum. Mol. Gene*, 2002, vol. 11, 175
- REMINGTON'S PHARMACEUTICAL SCIENCES. Mack Publishing Company, 1985
- *FIELDS VIROGY*, 2001
- **MEDINA et al.** *Antiviral Res.*, 2003, vol. 60 (2), 135-143
- **HUGLE** ; **CERNY**. *Rev. Med. Virol.*, 2003, vol. 13 (6), 361-71
- **KOCK et al.** *Hepatoloy*, 2003, vol. 38 (6), 1410-8
- **BORDIER et al.** *J.Clin.invest.*, 2003, vol. 112 (3), 407-414
- **YURDAYDIN et al.** *J Hepatol.*, 2002, vol. 37 (2), 266-71
- **CHIANG et al.** *Planta Med.*, 2003, vol. 69 (8), 705-9
- **BAYES** ; **STEIN**. *Expert Opin. Pharmacother.*, 2003, vol. 4 (11), 1901-38
- **SAMBROOK et al.** Molecular Cloning: A Laboratory Manual. Cold Spring Harbor Laboratory Press, 2000
- **AUSUBEL et al.** SHORT PROTOCOLS IN MOLECULAR BIOLOGY. Greene Publishing Associates, Inc. and John Wiley & Sons, Inc., 2002
- PCR Protocols A Guide to Methods and Applications. Academic Press Inc., 1990
- *The Journal Of NIH Research*, 1991, vol. 3, 81
- **KWOH et al.** *Proc. Natl. Acad. Sci. USA*, 1989, vol. 86, 1173
- **GUATELLI et al.** *Proc. Natl. Acad. Sci. USA*, 1990, vol. 87, 1874
- **LOMELL et al.** *J. Clin. Chem.*, 1989, vol. 35, 1826
- **LANDEGREN et al.** *Science*, 1988, vol. 241, 1077
- **VAN BRUNT**. *Biotechnology*, 1990, vol. 8, 291
- **WU** ; **WALLACE**. *Gene*, 1989, vol. 4, 560
- **BARRINGER et al.** *Gene*, 1990, vol. 89, 117
- **SOOKNANAN** ; **MALEK**. *Biotechnology*, 1995, vol. 13, 563
- **BEAUCAGE** ; **CARUTHERS**. *Tetrahedron letts*, 1981, vol. 22 (20), 1859-1862
- **NEEDHAM VANDEVANTER et al.** *Nucleic Acids Res.*, 1984, vol. 12, 6159
- **PEARSON** ; **REGNIER**. *J. Chrom.*, 1983, vol. 255, 137-149
- **MAXAM** ; **GILBERT**. Methods in Enzymology. Academic Press, 1980, vol. 65, 499